# EUROPEAN PATENT APPLICATION

(11) **EP 3 050 888 A1**
(43) Date of publication of application: **03.08.2016**
(21) Application number: 15000223.6
(22) Date of filing: 27.01.2015
(51) Int. Cl.: C07D 498/04, A61K 31/5383, A61P 31/04

(54) **Compounds for the treatment of bacterial infections**

(71) Applicant: Merck Sharp & Dohme Corp., Rahway, NJ 07065-0907 (US); Intervet International B.V., 5831 AN Boxmeer (NL); Kyorin Pharmaceutical Co., Ltd., Tokyo 101-8311 (JP)
(72) Inventor: Fukuda, Yasumichi, Okinawa 904-2234 (JP); Kawashima, Mikako, Tochigi 329-0114 (JP); Takano, Hisashi, Tokyo 101-8311 (JP); Singh, Sheo B., Edison, NJ 08820 (US); Warrass, Ralf, 55270 Schwabenheim (DE); Ullrich, Joachim, 55270 Schwabenheim (DE)
(74) Representative: Hutter, Anton

(57) **Abstract**

Novel bridged compounds of formula (I) are disclosed herein, along with their pharmaceutically acceptable salts and hydrates. Also disclosed are compositions comprising such compounds, methods of preparing such compounds and methods of using such compounds as antibacterial agents. The disclosed compounds, their pharmaceutically acceptable salts, hydrates and prodrugs, as well as compositions comprising such compounds, salts, hydrates and prodrugs, are useful for treating bacterial infections and associated diseases and conditions.

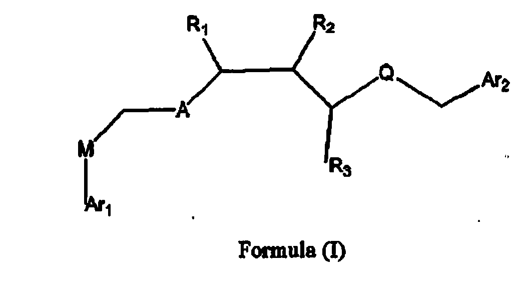

## Description

### FIELD OF THE INVENTION

The present invention relates to novel bridged compounds (including pharmaceutically acceptable salts, hydrates and prodrugs thereof), compositions containing such compounds, synthesis of such compounds, and use of such compounds as antibacterial agents. The novel compounds of this disclosure and compositions comprising such compounds are useful for treating bacterial infections and associated diseases and conditions.

### BACKGROUND OF THE INVENTION

Bacterial infection is a major healthcare problem, and the incidence of hospital-acquired bacterial diseases continues to rise, particularly with drug-resistant strains. See Chu et al., 1996, J. Med. Chem. 39:3853-3874 and Boucher et. al., 2009, Clin. Infect. Dis. 48:1-12. As a result of drug resistance, many bacterial infections are either difficult to treat with today's antibiotics or even untreatable. See Klevens et. al. 2007 J. Am. Med. Assoc. 298, 1763-1771 and Boucher et. al., 2009, Clin. Infect. Dis. 48:1-12. The problem of resistant bacteria is not confined to human health, but also has an increasing impact on the welfare of animals and the management of food-producing animals (Schwarz, S. & Chaslus-Dancla, 2001. Vet. Res. 32:201-225; Catry et al., 2003. J. vet Pharmacol. Therap. 26:81-93)..

Antibiotics of nearly all chemical classes are used to treat or prevent infections in livestock, poultry, aquatic and companion animals (De Briyne et al., 2014. Veterinary Record 1-8.; Mathew et al., 2007. Foodborne Pathogens and Disease 4:115-133). The rise of drug-resistance in veterinary pathogens poses a threat to animal welfare, food production and, particularly in the case of zoonotic, food-borne pathogens like *Salmonella spp.* and *Campylobacter spp.,* is considered a serious public health concern (Schwarz et al., 2001 Int. J. Antimicrob. Agents 17:431-437; Swartz, M.N., 2002. Clinical Infectious Diseases 34:S111-22; Catry et al., 2003. J. vet Pharmacol. Therap. 26:81-93; Mathew et al., 2007. Foodborne Pathogens and Disease 4:115-133). The increase of resistance in zoonotic pathogens has led to the ban of antibiotic growth promotors in Europe (Casewell et al., 2003. Journal of Antimicrobial Chemotherapy 52:159-161) and to resistance surveillance programs in Europe and in the US (Gilbert et al., 2007. Future Microbiology 2:493-500; de Jong et al., 2013. Int J Antimicrob Agents 41:403-409). It is therefore imperative to human food safety and animal health to develop new antibiotics with resistance-breaking properties (Stanton T.B., 2013. Trends in Microbiology 21:111-113).

International Publication No. WO 2013/003383 discloses novel bridged bicyclo compounds of the following formula (including pharmaceutically acceptable salts, hydrates and prodrugs thereof) useful for treating bacterial infections and associated diseases and conditions.

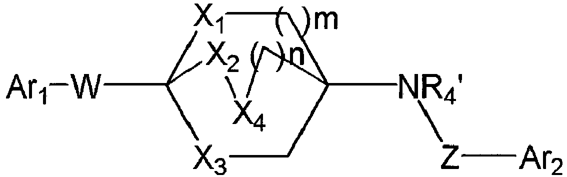

### SUMMARY OF THE INVENTION

The present invention relates to bridged compounds. These compounds, or pharmaceutically acceptable salts thereof, are useful in the treatment of bacterial infections caused by one or more of various pathogens including, but not limited to, *Staphylococcus aureus..* In particular, the present invention includes a compound of Formula **I:** 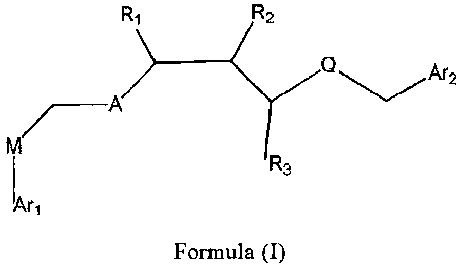 wherein
A is CH₂, O or Q;
M is (CH₂)ₙ;
n = 0-3
R₁ and R₃ are independently H, N(R₄R₅), or C₁-C₆ alkyl, wherein the C₁-C₆ alkyl is optionally substituted with one or more substituents selected from H, halogen, C₁-C₆ alkyl, N(R₄R₅) or OR₅;
R₂ is **H,** halogen, OR₅, N(R₄R₅), or C₁-C₆ alkyl, wherein the C₁-C₆ alkyl is optionally substituted with one or more substituents selected from H, halogen, C₁-C₆ alkyl, N(R₄R₅)or OR₅; or
R₁ and R₂ are taken together to form a 3-7 member ring which includes one or more C, O, or N(R₄)atom;
Q is N(R₄)
R₄ are each individually H or C₁-C₆ alkyl;
R₅ are each individually H or C₁-C₆ alkyl;
Ar₁ is selected from a group having one of the following structures 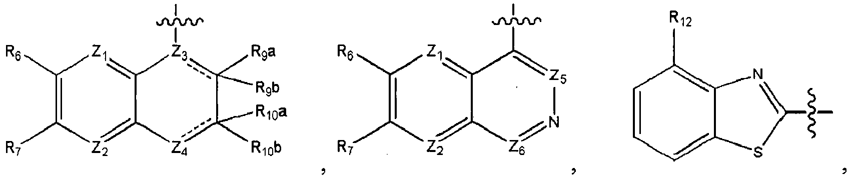 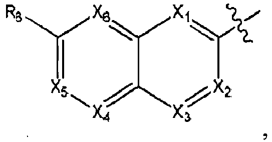 or 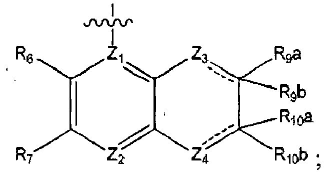
   Z₁ is CR₁ₐ or N;
   Z₂, Z₅ and Z₆ are independently selected from CR_{1b}, or N;
   Z₃ is C or N if the ------ bond to which it is attached is a single bond; or
   Z₃ is C if the ------ bond to which it is attached is a double bond;
   Z₄ is CR₁₁ₐR_{11b}, NR₄, or O if the ------ bond to which it is attached is a single bond; or
   Z₄ is CR₁₁ₐ or N if the ------ bond to which it is attached is a double bond;
   X₁, X₃, X₄ and X₆ are independently selected from N or CR₁ₐ;
   wherein at least one of X₁, X₃, X₄ and X₆ is N;
   X₂ is CH, C-(C₁₋₆)alkyl, C-(C₁₋₆)alkoxy, C-halo, or C-COOH;
   X₅ is CH, C-(C₁₋₆)alkyl or C-halo;
   R₆ is H; OH; NR₁₃R₁₄; (C₁₋₆)alkyl; C(O)OR₁₃; halo; CF₃; cyano; allyloxy;
-R₁₅COOR₁₄; -OR₁₅COOR₁₄; (C₁₋₆)alkoxy, (C₃₋₆)cycloalkoxy, (C₃₋₆)heterocycleoxy, (C₃₋₆)cycloalkylalkoxy, or (C₃₋₆)heterocycloalkoxy which are optionally substituted with NR₁₃R₁₄, OH, CF₃, COOR₁₄, cyano, oxo, (C₁₋₆)alkyl or (C₁₋₆)alkoxy; S(O)₂R₁₃ optionally substituted with a (C₁₋₆)alkyl; or 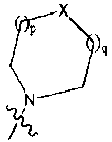 wherein X is CR_{1c}, O or S;
   each p and q is 0, 1, or 2, with the proviso that if X is O or S, both p and q cannot be 0;
   each R₇ is independently H, halo, OH, (C₁₋₆)alkoxy, NR₁₃R₁₄, CF₃, or cyano;
   R₉ₐ is H, halo, OH, (C₁₋₆)alkoxy, NH₂, or cyano; R_{9b} is absent; and the ------ bond attached to Z₃ is a double bond; or
   R₉ₐ and R_{9b} together form oxo; and the ------ bond attached to Z₃ is a single bond;
   R₁₀ₐ is H or (C₁₋₆)alkyl; R_{10b} is absent; and the ------ bond attached to Z₄ is a double bond; or
   R₁₀ₐ and R_{10b} together form oxo; and the ------ bond attached to Z₄ is a single bond;
   R₁₁ₐ is H or (C₁₋₆)alkyl; and R_{11b} is absent; and the ------ bond attached to Z₄ is a double bond; or
   R₁₁ₐ and R_{11b} together form oxo; and the ------ bond attached to Z₄ is a single bond;
   or R₁₀ₐ and R₁₁ₐ together with the atoms to which they are attached form a 5-membered saturated, unsaturated or aromatic ring having 0 to 3 N and optionally substituted with a (C₁₋₆)alkyl, wherein R_{10b} and R_{11b} are H when the ------ bond attached to Z₄ is a single bond and absent when the ------ bond attached to Z₄ is a double bond;
   each R₁₂, R₁₃ and R₁₄ is independently H or (C₁₋₆)alkyl;
   each R₁₅ is independently (C₁-C₆)alkyl or (C₂-C₆)alkenyl with the proviso that when R₆ is -OR₁₅COOR₁₄, R₁₅ is not C₂alkenyl;
   R₁ₐ is H, OH, (C₁₋₆)alkoxy, cyano, or halo;
   R_{1b} is H, (C₁₋₆)alkyl, (C₁₋₆)alkoxy, halo, cyano, or C(O)OR₁₃;
   R_{1c} is H, halo or (C₁₋₆)alkyl;
   and
Ar₂ is selected from
   (i) C₃-C₆-cycloalkyl, optionally substituted with -OH, halo, cyano, NR₁₃R₁₄ or (C₁₋₆)alkyl;
   (ii) aryl, wherein aryl is phenyl or naphthyl optionally substituted with 1 to 3 substituents selected from OH, halo, (C₁₋₆)alkoxy and (C₁₋₆)alkyl;
   (iii) a heterocyclyl, wherein the heterocyclyl is a 5- to 6-membered non-aromatic or aromatic ring having 1 or 2 heteroatoms selected from N, O or S optionally substituted with 1 to 3 substituents selected from OH, halo, cyano, (C₁₋₆)alkoxy, (C₁₋₆)alkyl, NR₁₃R₁₄ and a 5- to 6-membered aromatic or non-aromatic ring having 1 or 2 heteroatoms selected from N, O or S;
   wherein (C₁₋₆)alkoxy or (C₁₋₆)alkyl optionally substituted with 1 or 2 halo; or
   (iv) a group having one of the following structures: 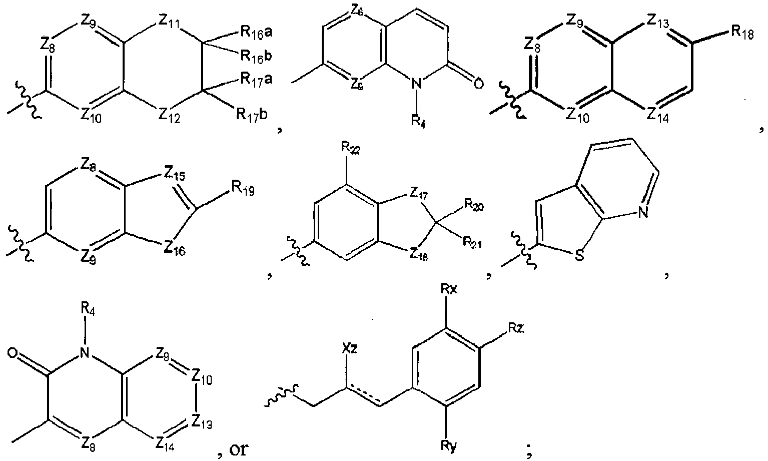 each Z₈, Z₉ and Z₁₀ is independently CR₁ₐ or N;
      Z₁₁ and Z₁₂ are each independently selected from CR₁ₐR_{1b}, NR₄, O, and S;
      Z₁₃ and Z₁₄ are each independently selected from CR₁ₐ and N;
      Z₁₅ is CR₁ₐ or N;
      Z₁₆ is CR₁ₐR_{1b} or NH;
      each Z₁₇ and Z₁₈ is independently NR₄ or O;
      each R₁₆ₐ and R_{16b} is independently H or CH₃;
      or R₁₆ₐ and R_{16b} together form oxo;
      R₁₇ₐ and R_{17b} are H;
      or R₁₇ₐ and R_{17b} together form oxo or =NOR₃;
      R₁₈ is H or (C₁₋₆)alkoxy;
      R₁₉ is H or halo;
each R₂₀, R₂₁ and R₂₂ is independently H or halo
each Rₓ, R_{y} and R_{z} is independently H, halo or (C1-6)alkyl
X_{z} is H, halo or (C1-6)alkyl
or a pharmaceutically acceptable salt thereof.

These compounds are potent antibacterial agents useful against pathogens associated with bacterial infections.

Additional aspects of the invention relate to compositions comprising the compounds of the invention, optionally in the presence of a second therapeutic agent. In addition, aspects of the invention relate to methods of preparing a compound of the invention, to methods of preparing compositions of the invention, to methods of treating bacterial infection in patients and animals using a compound of the invention, and to methods of controlling bacterial infection in patients and animals using a compound of the invention.

Other embodiments, aspects and features of the present invention are either further described in or will be apparent from the ensuing description, examples and appended claims.

### DETAILED DESCRIPTION OF THE INVENTION

The present invention relates to compounds of Formula (I) and pharmaceutically acceptable salts thereof, as defined above and a first embodiment of the invention. Different embodiments further describing Formula (I) variables are described below.

In particular, the present invention includes a compound of Formula I: 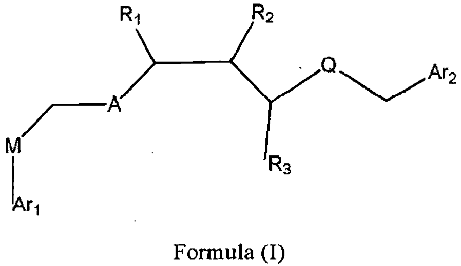 wherein
A is CH₂, O or Q;
M is (CH₂)ₙ;
n = 0-3
R₁ and R₃ are independently H, N(R₄R₅), or C₁-C₆ alkyl, wherein the C₁-C₆ alkyl is optionally substituted with one or more substituents selected from H, halogen, C₁-C₆ alkyl, N(R₄R₅) or OR₅;
R₂ is H, halogen, OR₅, N(R₄R₅), or C₁-C₆ alkyl, wherein the C₁-C₆ alkyl is optionally substituted with one or more substituents selected from H, halogen, C₁-C₆ alkyl, N(R₄R₅)or OR₅;
or
R₁ and R₂ are taken together to form a 3-7 member ring which includes one or more C, O, or N(R₄)atom;
Q is N(R₄)
R₄ are each individually H or C₁-C₆ alkyl;
R₅ are each individually H or C₁-C₆ alkyl;
Ar₁ is selected from a group having one of the following structures 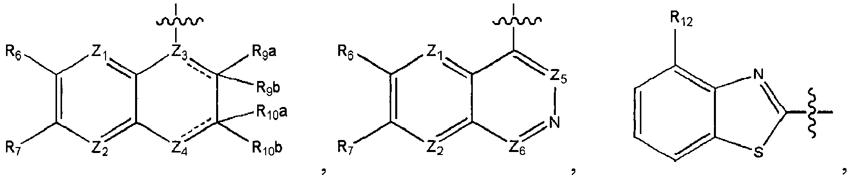 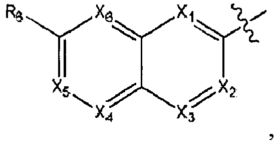 or 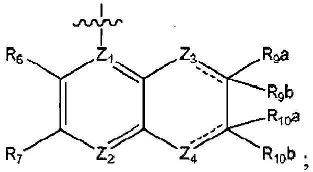 Z₁ is CR₁ₐ or N;
   Z₂, Z₅ and Z₆ are independently selected from CR_{1b}, or N;
   Z₃ is C or N if the ------ bond to which it is attached is a single bond; or
   Z₃ is C if the ------ bond to which it is attached is a double bond;
   Z₄ is CR₁₁ₐR_{11b}, NR₄, or O if the ------ bond to which it is attached is a single bond; or
   Z₄ is CR₁₁ₐ or N if the ------ bond to which it is attached is a double bond;
   X₁, X₃, X₄ and X₆ are independently selected from N or CR₁ₐ;
   wherein at least one of X₁, X₃, X₄ and X₆ is N;
   X₂ is CH, C-(C₁₋₆)alkyl, C-(C₁₋₆)alkoxy, C-halo, or C-COOH;
   X₅ is CH, C-(C₁₋₆)alkyl or C-halo;
   R₆ is H; OH; NR₁₃R₁₄; (C₁₋₆)alkyl; C(O)OR₁₃; halo; CF₃; cyano; allyloxy;
-R₁₅COOR₁₄; -OR₁₅COOR₁₄; (C₁₋₆)alkoxy, (C₃₋₆)cycloalkoxy, (C₃₋₆)heterocycleoxy, (C₃₋₆)cycloalkylalkoxy, or (C₃₋₆)heterocycloalkoxy which are optionally substituted with NR₁₃R₁₄, OH, CF₃, COOR₁₄, cyano, oxo, (C₁₋₆)alkyl or (C₁₋₆)alkoxy; S(O)₂R₁₃ optionally substituted with a (C₁₋₆)alkyl; or 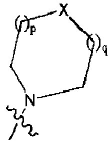 wherein X is CR_{1c}, O or S;
   each p and q is 0, 1, or 2, with the proviso that if X is O or S, both p and q cannot be 0;
   each R₇ is independently H, halo, OH, (C₁₋₆)alkoxy, NR₁₃R₁₄, CF₃, or cyano;
   R₉ₐ is H, halo, OH, (C₁₋₆)alkoxy, NH₂, or cyano; R_{9b} is absent; and the ------ bond attached to Z₃ is a double bond; or
   R₉ₐ and R_{9b} together form oxo; and the ------ bond attached to Z₃ is a single bond;
   R₁₀ₐ is H or (C₁₋₆)alkyl; R_{10b} is absent; and the ------ bond attached to Z₄ is a double bond; or
   R₁₀ₐ and R_{10b} together form oxo; and the ------ bond attached to Z₄ is a single bond;
   R₁₁ₐ is H or (C₁₋₆)alkyl; and R_{11b} is absent; and the ------ bond attached to Z₄ is a double bond; or
   R₁₁ₐ and R_{11b} together form oxo; and the ------ bond attached to Z₄ is a single bond;
   or R₁₀ₐ and R₁₁ₐ together with the atoms to which they are attached form a 5-membered saturated, unsaturated or aromatic ring having 0 to 3 N and optionally substituted with a (C₁₋₆)alkyl, wherein R_{10b} and R_{11b} are H when the ------ bond attached to Z₄ is a single bond and absent when the ------ bond attached to Z₄ is a double bond;
   each R₁₂, R₁₃ and R₁₄ is independently H or (C₁₋₆)alkyl;
   each R₁₅ is independently (C₁-C₆)alkyl or (C₂-C₆)alkenyl with the proviso that when R₆ is -OR₁₅COOR₁₄, R₁₅ is not C₂alkenyl;
   R₁ₐ is H, OH, (C₁₋₆)alkoxy, cyano, or halo;
   R_{1b} is H, (C₁₋₆)alkyl, (C₁₋₆)alkoxy, halo, cyano, or C(O)OR₁₃;
   R_{1c} is H, halo or (C₁₋₆)alkyl;
   and
Ar₂ is selected from
   (i) C₃-C₆-cycloalkyl, optionally substituted with -OH, halo, cyano, NR₁₃R₁₄ or (C₁₋₆)alkyl;
   (ii) aryl, wherein aryl is phenyl or naphthyl optionally substituted with 1 to 3 substituents selected from OH, halo, (C₁₋₆)alkoxy and (C₁₋₆)alkyl;
   (iii) a heterocyclyl, wherein the heterocyclyl is a 5- to 6-membered non-aromatic or aromatic ring having 1 or 2 heteroatoms selected from N, O or S optionally substituted with 1 to 3 substituents selected from OH, halo, cyano, (C₁₋₆)alkoxy, (C₁₋₆)alkyl, NR₁₃R₁₄ and a 5- to 6-membered aromatic or non-aromatic ring having 1 or 2 heteroatoms selected from N, O or S; wherein (C₁₋₆)alkoxy or (C₁₋₆)alkyl optionally substituted with 1 or 2 halo; or
   (iv) a group having one of the following structures: 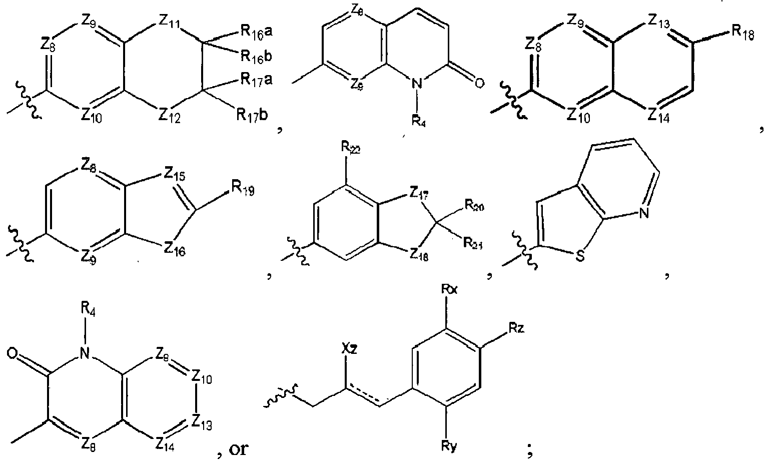
   each Z₈, Z₉ and Z₁₀ is independently CR₁ₐ or N;
   Z₁₁ and Z₁₂ are each independently selected from CR₁ₐR_{1b}, NR₄, O, and S;
   Z₁₃ and Z₁₄ are each independently selected from CR₁ₐ and N;
   Z₁₅ is CR₁ₐ or N;
   Z₁₆ is CR₁ₐR_{1b} or NH;
   each Z₁₇ and Z₁₈ is independently NR₄ or O;
   each R₁₆ₐ and R_{16b} is independently H or CH₃;
   or R₁₆ₐ and R_{16b} together form oxo;

   R₁₇ₐ and R_{17b} are H;
   or R₁₇ₐ and R_{17b} together form oxo or =NOR₃;
   R₁₈ is H or (C₁₋₆)alkoxy;
   R₁₉ is H or halo;
each R₂₀, R₂₁ and R₂₂ is independently H or halo
each Rₓ, Ry and R_{z} is independently H, halo or (C1-6)alkyl
X_{z} is H, halo or (C1-6)alkyl
or a pharmaceutically acceptable salt thereof.

In another embodiment, the present invention includes compound of Formula (II) 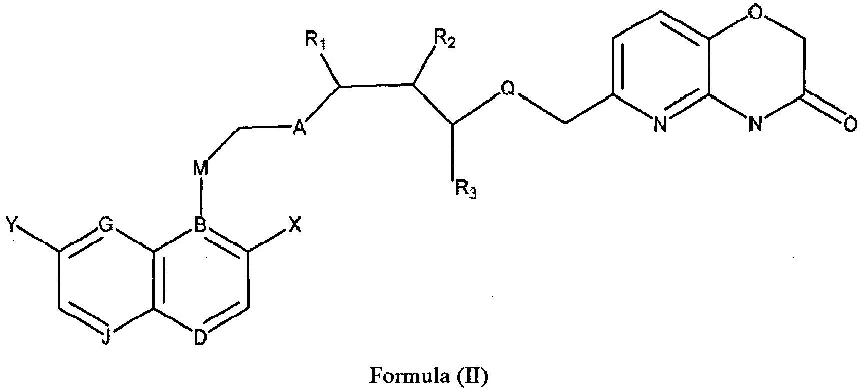 wherein,
A is CH₂, NH, or O;
B, D, G, J are each independently either C or N;
M is (CH₂)ₙ;
(n = 0-3)
X is H, F, Cl, CN, CO₂R₅ or O;
Y is OR₅, CO₂R₅ or CN;
R₁ and R₃ are independently H, N(R₄R₅), or C₁-C₆ alkyl, wherein the C₁-C₆ alkyl is optionally substituted with one or more substituents selected from H, halogen, C₁-C₆ alkyl, N(R₄R₅)or OR⁵;
R₂ is H, halogen, OR₅, N(R₄R₅), or C₁-C₆ alkyl, wherein the C₁-C₆ alkyl is optionally substituted with one or more substituents selected from H, halogen, C₁-C₆ alkyl, N(R₄R₅)or OR⁵; or
R₁ and R₂ are taken together to form a 3-7 member ring which includes one or more C, O or N atoms;
Q is NR₄;
R₄ are each individually H or C₁-C₆ alkyl; and
R₅ are each individually H or C₁-C₆ alkyl
or a pharmaceutically acceptable salt thereof.

In another embodiment, the present invention includes a compound of Formula (II) wherein
A is NH;
B and J are C;
D and G are N;
M is CH₂; X is H, F,Cl, CN, OR₅, or CO₂R₅; and
Y is H, Cl, F, CN, OR₅, or CO₂R₅
wherein R₅ is defined as above
or a pharmaceutically acceptable salt thereof.

In another embodiment, the present invention includes a compound of Formula (III) 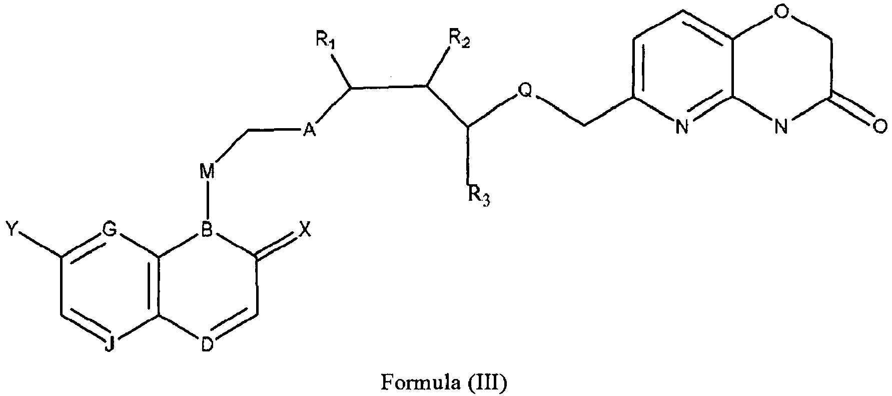 wherein,
A is CH₂, NH, or O;
B is N;
D, G, J are each independently either C or N;
M is (CH₂)ₙ; (n = 0-3)
X is O or S ;
Y is H, F, Cl, OR₅, CN, or CO₂R₅
R₁ and R₃ are H,, N(R₄R₅), or C₁-C₆ alkyl, wherein the C₁-C₆ alkyl is optionally substituted with one or more substituents selected from H, halogen, C₁-C₆ alkyl, N(R₄R₅) or OR₅;
R₂ is H, halogen, OR₅, N(R₄R₅), or C₁-C₆ alkyl, wherein the C₁-C₆ alkyl is optionally substituted with one or more substituents selected from H, halogen, C₁-C₆ alkyl, N(R₄R₅)or OR₅; or
R₁ and R₂ are taken together to form a 3-7 member ring which includes one or more C, O or N atoms;
Q is NR₄;
R₄ are each individually H or C₁-C₆ alkyl; and
R₅ are each individually H or C₁-C₆ alkyl,
or a pharmaceutically acceptable salt thereof.

In another embodiment, the present invention includes a pharmaceutically acceptable salt of the compounds of Formulas (I), (II) or (III).

In another embodiment, the present invention includes a compound selected from the following group: 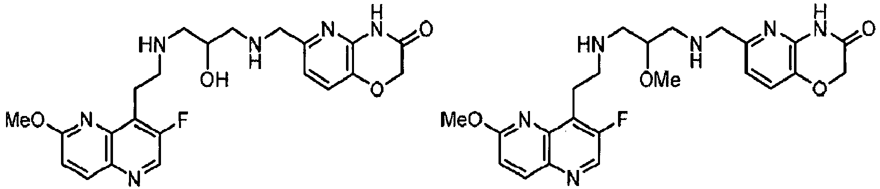 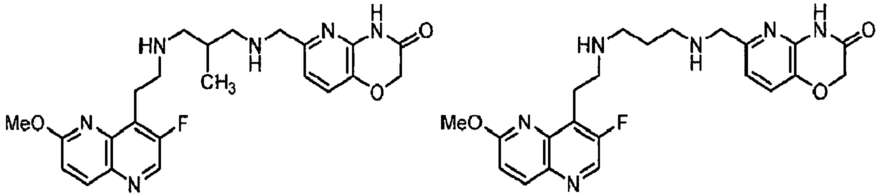 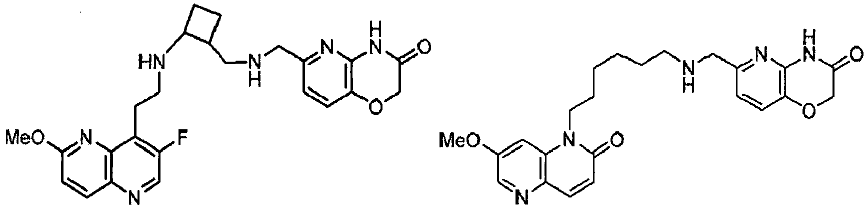 and 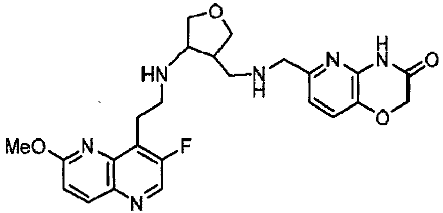 or a pharmaceutically acceptable salt thereof.

In another embodiment, the present invention includes a compound selected from the following group:
6-[[[3-[2-(3-fluoro-6-methoxy-1,5-naphthyridin-4-yl)ethylamino]-2-hydroxy-propyl]amino]methyl]-4H-pyrido[3,2-b][1,4]oxazin-3-one;
6-[[[3-[2-(3-fluoro-6-methoxy-1,5-naphthyridin-4-yl)ethylamino]-2-methoxy-propyl]amino]methyl]-4H-pyrido[3,2-b][1,4]oxazin-3-one;
6-[[[3-[2-(3-fluoro-6-methoxy-1,5-naphthyridin-4-yl)ethylamino]-2-methyl-propyl]amino]methyl]-4H-pyrido[3,2-b][1,4]oxazin-3-one;
6-[[3-[2-(3-fluoro-6-methoxy-1,5-naphthyridin-4-yl)ethylamino]propylamino]methyl]-4H-pyrido[3,2-b][1,4]oxazin-3-one;
6-[[[2-[2-(3-fluoro-6-methoxy-1,5-naphthyridin-4-yl)ethylamino]cyclobutyl]methylamino]methyl]-4H-pyrido[3,2-b][1,4]oxazin-3-one;
6-[[6-(7-methoxy-2-oxo-1,5-naphthyridin-1-yl)hexylamino]methyl]-4H-pyrido[3,2-b][1,4]oxazin-3-one; and
6-[[[4-[2-(3-fluoro-6-methoxy-1,5-naphthyridin-4-yl)ethylamino]tetrahydrofuran-3-yl]methylamino]methyl]-4H-pyrido[3,2-b][1,4]oxazin-3-one;
or a pharmaceutically acceptable salt thereof.

In another embodiment, the present invention includes a pharmaceutical composition comprising the above compounds and a pharmaceutical carrier, adjuvant or vehicle.

In another embodiment, the present invention includes a composition further comprising a second therapeutic agent selected from the group consisting of antibiotic agents, anti-inflammatory agents, anti-parasitics and antifungal agents.

In another embodiment, the present invention includes a composition, wherein the second therapeutic agent is one or more of an amphenicol, a â-lactam, a tetracycline, an aminoglycoside, a quinolone, a mutilin, a macrolide, an azole, a non-steroidal anti-inflammatory drug (NSAID), a glucocorticosteroid or their pharmaceutically acceptable salts.

In another embodiment, the present invention includes a method of treating an infectious disease in an animal comprising administration to the animal of an effective amount of the above pharmaceutical composition to the animal.

In another embodiment, the present invention includes a method, wherein the infectious disease is selected from a respiratory tract disease, a mastitis disease, a skin or soft tissue disease, a urinary tract disease or a disease caused by opportunistic pathogens.

In another embodiment, the present invention includes a method, wherein the animal is a bovine, a swine, a member of the poultry family, an ovine, a fish, a horse, a dog or a cat.

In another embodiment, the present invention includes a method, wherein the infection is *Mannheimia haemolytica, Pasteurella multocida, Histophilus somni, Mycoplasma bovis, Pseudomonas aeruginosa, Staphylococcus pseudintermedius, Staphylococcus aureus, Staphylococcus spp., Streptococcus dysgalactiae, Streptococcus uberis, Streptococcus canis, Escherichia coli, Actinobacillus pleuropneumoniae, Bordetella bronchiseptica or Rhodococcus equi* infection.

In another embodiment, the present invention includes a use of an effective amount of the compound of any one of Formulas (I), (II) or (III) for the preparation of a medicament for treating a bacterial infection.

In another embodiment of the invention, the compound of the invention is selected from the exemplary species depicted in Examples 1 through 9 shown below (including free base forms thereof and any pharmaceutically acceptable salts thereof).

Other embodiments of the present invention include the following (where reference to a compound of formula (I) encompasses the various embodiments and aspects described above, as well as their pharmaceutically acceptable salts):
(a) A composition comprising a compound of Formula (I) and a carrier, adjuvant, or vehicle;
(b) A pharmaceutical composition comprising a compound of Formula (I) and a pharmaceutically acceptable carrier, adjuvant, or vehicle;
(c) The pharmaceutical composition of (b), further comprising a second therapeutic agent;
(d) The pharmaceutical composition of (c), wherein the second therapeutic agent is an antibiotic, an anti-inflammatory; an analgesic; an anti-parasitics; or an antifungal;
(e) A pharmaceutical combination which is (1) a compound of Formula (I) and (2) a second therapeutic agent, wherein the compound of Formula (I) and the second therapeutic agent are each employed in an amount that renders the combination effective for treating bacterial infections;
(f) A method of treating a bacterial infections in a subject in need thereof comprising administering to the subject an effective amount of a compound of Formula (I);
(g) The method of (f), wherein the compound of Formula (I), is administered in combination, either sequentially or concurrently, with a second therapeutic agent effective against bacterial infections; and
(h) A method of treating bacterial infections in a subject in need thereof comprising administering to the subject a pharmaceutical composition of (b), (c), or (d), or the combination of (e).

The present invention also includes a compound of the present invention (i) for use in, (ii) for use as a medicine or medicament for, or (iii) for use in the preparation of a medicament for: treating bacterial infections. In these uses, the compounds of the present invention can optionally be employed in combination, either sequentially or concurrently, with one or more therapeutic agents effective against bacterial infections.

In the embodiments of the compound as provided above, it is to be understood that each embodiment may be combined with one or more other embodiments, to the extent that such a combination provides a stable compound and is consistent with the description of the embodiments. It is further to be understood that the embodiments of compositions and methods provided as (a) through (h) above are understood to include all embodiments of the compounds, including such embodiments as result from combinations of embodiments of the compound.

In addition, it is understood that, in the description of embodiments of the compounds as set forth above, indicated substitutions are included only to the extent that the substitutents provide stable compounds consistent with the definition.

Additional embodiments of the invention include the pharmaceutical compositions, combinations and methods set forth in (a)-(h) above and the uses set forth in the preceding paragraph, wherein the compound of the present invention employed therein is a compound of one of the embodiments or aspects of the compounds described above. In all of these embodiments or aspects as well as those described herein below, the compound may optionally be used in the form of a pharmaceutically acceptable salt or hydrate when appropriate.

In the compounds of generic Formula I, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula I. For example, different isotopic forms of hydrogen (H) include protium (¹H) and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds within generic Formula I can be prepared without undue experimentation by conventional techniques well known to those skilled in the art or by processes analogous to those described in the Schemes and Examples herein using appropriate isotopically-enriched reagents and/or intermediates.

The present compounds (including pharmaceutical acceptable salt and/or hydrate forms) have broad spectrum antimicrobial (e.g., antibacterial) activities and are useful for the treatment of bacterial infections. As used herein, unless otherwise indicated, the term "bacterial infection (s)" includes bacterial infections that occur in mammals as well as disorders related to bacterial infections that may be treated by administering antibiotics such as the compounds of the present invention. Such bacterial infections and disorders related to such infections include one or more of the following: Skin and soft tissue infections, pyodermas, abscesses, pneumonia, bronchitis, tonsillitis, otitis media, osteomyelitis and mastitis, puerperal fever, cystitis, gingivitis and periodontitis. Of specific interest are canine and feline skin, soft tissue infections and pyodermas caused by *Staphylococcus pseudintermedius, Staphylococcus epidermidis,* coagulase-negative and -positive *Staphylococci, Streptococcus spp.* or *Pasteurella multocida.* Abscesses caused by *Staphylococcus* spp., *Streptococcus spp., Streptococcus pyogenes, Pasteurella multocida* and *Nocardia spp.* Pneumonia and other respiratory tract infections in dogs and cats associated with *Bordetella bronchiseptica, Escherichia coli, Pseudomonas aeruginosa, Klebsiella pneumoniae, Pasteurella spp., Mycoplasma spp., Streptococcus spp., Staphylococcus spp.* Feline and canine urinary tract infections caused by *Staphylococcus spp., Escherichia coli, Streptococcus spp., Proteus spp., Klebsiella spp., Enterobacter spp. and Pseudomonas spp.* Bacterial cystitis, gingivitis, periodontitis, oral cavity infections, otitis media and osteomyelitis in dogs and cats caused by a variety of pathogens as: *Escherichia coli, Staphylococcus spp., Streptococcus spp., Haemophilus haemoglobinophilus, Bacteroides fragilis, Bacteroides spp., Clostridium spp., Enterobacter spp., Peptostreptococcus, Porphyromonasspp., Prevotella spp., Treponema spp, , Fusobacterium spp, Neisseria spp., Pseudomonas spp, Corynebacterium spp., Trueperella pyogenes, Proteus spp, Pasteurella spp, and Brucella canis.* Leptospirosis ehrlichiosis in dogs and cats caused by *Leptospira spp. and Ehrlichia spp.,* respectively. Pneumonia in foals and goats related to *Rhodococcus equi.* Pneumonia in horses related to *Streptococcus equi subsp. equi* or *zooepidemicus, Actinobacillus equuli.*

Further bacterial infections and associated diseases treated by the present compounds are: Bovine respiratory disease related to infection *byMannheimia haemolytica, Pasteurella multocida, Mycoplasma bovis, Mycoplasma spp., Histophilus somni, Trueperella pyogenes, Staphylococcus aureus, Chlamydia spp., Bibersteinia trehalosi.* Cow enteric disease related to infection by *Escherichia coli; Salmonella spp., Campylobcater spp.* Dairy cow mastitis related to infection by *Staphylococcus aureus, Streptococcus uberis, Streptococcus agalactiae, Streptococcus dysgalactiae, Klebsiella spp., Corynebacterium spp., or Enterococcus spp.* and *Escherichia coli.* Bovine and ovine footrot related to infection by *Fusobacterium spp.* and *Dichelobacter nodosus.* Cow metritis related to infection by *E. coli; Trueperella pyogenes, Fusobacterium spp.* Cow pink-eye disease related to infection by *Moraxella bovis.* Bovine liver abscesses related to *Fusobacterium necrophorum* and John's Disease related to *Mycobacterium avium ssp. paratuberculosis.* Swine respiratory disease related to infection by *Actinobacillus pleuropneumoniae, Pasteurella multocida, or Mycoplasma spp.* Swine Glässners Disease related to *Haemophilus parasuis.* Swine atropohic rhinitis related to *Bordetella bronchiseptica* and *Pasteurella multocida.* Swine enteric disease related to infection by *E. coli, Lawsonia intracellularis, Salmonella spp., or Brachyspira hyodyisinteriae.* Fowl cholera related to *Pasteurella multocida* and avian enteritis related to *Clostridium perfringens.* Infectious arthritis and mycoplasma diseases in birds related to *Staphylococcus spp., Enterococcus spp., Mycoplasma synovia, Mycoplasma gallisepticum and Escherichia coli.*

In another embodiment, the bacterial infections and disorders related to such infections includes one or more of the following: *Staphylococcus aureus* Smith, *Enterococcus faecium, Streptococcus pneumoniae,* and *Escherichia coli.*

In yet another embodiment, the present compounds are useful for treating an infectious disease cause by opportunistic pathogens. An opportunistic infection is an infection cause by a normally nonpathogenic organism in a host whose resistance has been decreased by such disorders as diabetes, mellitus, AIDS, or cancer or by surgical procedure (see Mosby's Pocket Dictionary of Medicine, Nursing & Allied Health, 2nd Ed, 1994, Mosby -Year Book Inc, St. Louis, MO).

Other bacterial infections and disorders related to such infections that may be treated or prevented in accord with the method of the present invention are referred to in J. P. Sanford et al., "The Sanford Guide To Antimicrobial Therapy, "26th Edition, (Antimicrobial Therapy, Inc., 1996).

It will be appreciated that other active ingredients may be combined with the compounds of the present invention. Such ingredients include, for example, anti-inflammatory agents such as corticosteroids, a non-steroidal anti-inflammatory drug (NSAID), such as flunixin, COX-inhibitors and other analgesics, antiparasitic compounds such as, for example, indoxacarb, an isoxazoline such as fluralaner or afoxolaner, an avermectin compound such as ivermectin, doramectin, milbemycin, selamectin, emamectin, eprinomectin and moxidectin and or optionally a flukicide. It may also be preferred to employ a second antibiotics. Examples are tetracycline, - β-lactams such as penicillins, cephalosporins, amoxicillin ceftiofur, and cefquinome. Additional examples are fluoroquinolones such as, for example, enrofloxacin, danofloxacin, difloxacin, orbifloxacin and marbofloxacin and a macrolide antibiotic such as tildipirosin, tilmicosin, tulathromycin, erythromycin, azithromycin and pharmaceutically acceptable salts thereof and the like. Still further examples of antibiotics are amphenicol, an aminoglycoside, a quinolone, a mutilin, an azole, a glucocorticosteroid or their pharmaceutically acceptable salts

The term "acyl", as used herein, refers to a carbonyl containing substituent represented by the formula -C(O)-R in which R is alkyl, a cycloalkyl, an aryl, a heterocycle, cycloalkyl- or aryl-substituted alkyl or heterocycle-substituted alkyl wherein the alkyl, alkoxy, cycloalkyl, aryl and heterocycle are as defined herein. Representative acyl groups include, but are not limited to, alkanoyl (e.g. acetyl), aroyl (e.g. benzoyl), and heteroaroyl.

The term "sulfonyl", as used herein, refers to a substituent represented by the formula -S(O)₂-R in which R is H, alkyl, a cycloalkyl, an aryl, a heterocycle, cycloalkyl- or aryl-substituted alkyl or heterocycle-substituted alkyl wherein the alkyl, alkoxy, cycloalkyl, aryl and heterocycle are as defined herein.

The term "alkenyl", as used herein, refers to a straight or branched-chain acyclic unsaturated hydrocarbon having a number of carbon atoms in the specified range and containing at least one double bond. Thus, for example, "C₂-C₃ alkenyl" refers to vinyl, (1Z)-1-propenyl, (1E)-1-propenyl, 2-propenyl, or isopropenyl.

The term "alkoxy", as used herein, refers to an alkyl group, as defined herein, appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

The term "alkyl", as used herein, refers to any linear or branched chain alkyl group having a number of carbon atoms in the specified range, for example 1-8, 1-6 or 1-4. Thus, for example, "C₁₋₆ alkyl" (or "C₁-C₆ alkyl") refers to all of the hexyl alkyl and pentyl alkyl isomers as well as n-, iso-, sec- and t-butyl, n- and isopropyl, ethyl and methyl. As another example, "C₁₋₄ alkyl" refers to n-, iso-, sec- and t-butyl, n- and isopropyl, ethyl and methyl. C₁₋₆ alkyl and C₁₋₄ alkyl are examples of lower alkyls.

The term "aryl", as used herein, refers to a mono-or bicyclic carbocyclic ring system having one or two aromatic rings. Exemplary aryls include, but are not limited to, phenyl, naphthyl, tetrahydronaphthyl, indanyl, indenyl and the like. Aryl groups (including bicyclic aryl groups) can be unsubstituted (unless otherwise indicated, such groups are unsubstituted) or substituted with one, two or three substituents independently selected from lower alkyl, substituted lower alkyl, haloalkyl, alkoxy, thioalkoxy, amino, alkylamino, dialkylamino, acylamino, cyano, hydroxy, halo, mercapto, nitro, carboxaldehyde, carboxy, alkoxycarbonyl and carboxamide.

The term "cycloalkylalkoxy" refers to a cycloalkyl group, as defined herein, appended to the parent molecular moiety through an alkoxy group, as defined herein. The cycloalkyl group may have one or more carbon atoms in common with the alkoxy group. A (C₃₋₆)cycloalkylalkoxy refers to a C₃₋₆ cycloalkyl group attached to an alkoxy group. Representative examples of cycloalkylalkoxy include 2-(1-ethylcyclopropyl)methoxy, 2-(1-propylcyclopropoxy), 2-(2-ethylcyclopropoxy), 2-(3-ethylcyclohexyl)methoxy, 2-(4-ethylcyclohexyl)methoxy, 2-(4-propylcyclohexyl)methoxy, 2-(2-(4-propylcyclohexyl)ethoxy), 2-(2-ethylcyclopentyl)methoxy, and 2-(2-propylcyclopentyloxy)pyridine.

The terms "cycloalkoxy" or "cycloalkyloxy" refers to a cycloalkyl group, as defined herein, appended to the parent molecular moiety through an oxygen atom. Representative examples of cycloalkyloxy include, but are not limited to, cyclopropyloxy, cyclobutyloxy, cyclopentyloxy, cyclohexyloxy, cycloheptyloxy, and cyclooctyloxy.

The term "cycloalkyl", as used herein, refers to any cyclic ring of an alkane having a number of carbon atoms in the specified range. Thus, for example, "C₃₋₆ cycloalkyl" (or "C₃-C₆ cycloalkyl") refers to cyclopropyl, cyclobutyl, cyclopentyl, and cyclohexyl.

The term "halogen" (or "halo"), as used herein, refers to fluorine, chlorine, bromine and iodine (alternatively referred to as fluoro, chloro, bromo, and iodo).

The term "heteroaryl", as used herein, refers to a cyclic aromatic radical having from five to ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms. Exemplary heteroaryls include, but are not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, and the like. Heteroaryl groups (including bicyclic heteroaryl groups) can be unsubstituted or substituted with one, two or three substituents independently selected from lower alkyl, substituted lower alkyl, haloalkyl, alkoxy, thioalkoxy, amino, alkylamino, dialkylamino, acylamino, cyano, hydroxy, halo, mercapto, nitro, carboxaldehyde, carboxy, alkoxycarbonyl and carboxamide.

The term "heterocycle" (and variations thereof such as "heterocyclic" or "heterocyclyl"), as used herein, broadly refers to (i) a stable 4- to 8-membered, saturated or unsaturated monocyclic ring, and the ring system contains one or more heteroatoms (e.g., from 1 to 6 heteroatoms, or from 1 to 4 heteroatoms) selected from N, O and S and a balance of carbon atoms (the monocyclic ring typically contains at least one carbon atom and the ring systems typically contain at least two carbon atoms); and wherein any one or more of the nitrogen and sulfur heteroatoms is optionally oxidized, and any one or more of the nitrogen heteroatoms is optionally quaternized. Unless otherwise specified, the heterocyclic ring may be attached at any heteroatom or carbon atom, provided that attachment results in the creation of a stable structure. Heterocycle groups (including bicyclic heterocycle groups) can be unsubstituted or substituted with one, two or three substituents independently selected from lower alkyl, substituted lower alkyl, haloalkyl, alkoxy, thioalkoxy, amino, alkylamino, dialkylamino, acylamino, cyano, hydroxy, halo, mercapto, nitro, carboxaldehyde, carboxy, alkoxycarbonyl and carboxamide. Unless otherwise specified, when the heterocyclic ring has substituents, it is understood that the substituents may be attached to any atom in the ring, whether a heteroatom or a carbon atom, provided that a stable chemical structure results.

The term "heterocycloalkoxy" means a heterocycle group, as defined herein, appended to the parent molecular moiety through an alkoxy group, as defined herein. The heterocycle group may have one or more carbon atoms in common with the alkoxy group. A (C₃₋₆)heterocycloalkoxy refers to a C₃₋₆ heterocycle group attached to an alkoxy group. Representative examples of heterocycloalkoxy include, but are not limited to, 2-(5-ethyltetrahydro-2H-pyran-2-yl)methoxy), 2-pyridin-3-ylethoxy, 3-quinolin-3-ylpropoxy, and 5-pyridin-4-ylpentyloxy.

The term "heterocycleoxy" means a heterocycle group, as defined herein, appended to the parent molecular moiety through an oxygen atom. Representative examples of heterocycleoxy include, but are not limited to, pyridin-3-yloxy and quinolin-3-yloxy.

The term "oxo", as used herein, means =O and as used herein, the term "imino" means =NR₀, wherein R₀ is as previously defined.

The term "or", as used herein, denotes alternatives that may, where appropriate, be combined.

Unless expressly stated to the contrary, all ranges cited herein are inclusive. For example, a heterocyclic ring described as containing from "1 to 4 heteroatoms" means the ring can contain 1, 2, 3 or 4 heteroatoms. It is also to be understood that any range cited herein includes within its scope all of the sub-ranges within that range. Thus, for example, a heterocyclic ring described as containing from "1 to 4 heteroatoms" is intended to include as aspects thereof, heterocyclic rings containing 2 to 4 heteroatoms, 3 or 4 heteroatoms, 1 to 3 heteroatoms, 2 or 3 heteroatoms, 1 or 2 heteroatoms, 1 heteroatom, 2 heteroatoms, and so forth.

Any of the various cycloalkyl and heterocyclic/heteroaryl rings and ring systems defined herein may be attached to the rest of the compound at any ring atom (i.e., any carbon atom or any heteroatom) provided that a stable compound results. Suitable 5- or 6-membered heteroaromatic rings include, but are not limited to, pyridyl, pyrrolyl, pyrazinyl, pyrimidinyl, pyridazinyl, triazinyl, thienyl, furanyl, imidazolyl, pyrazolyl, triazolyl, tetrazolyl, oxazolyl, isooxazolyl, oxadiazolyl, oxatriazolyl, thiazolyl, isothiazolyl, and thiadiazolyl. Suitable 9- or 10-membered heteroaryl rings include, but are not limited to, quinolinyl, isoquinolinyl, indolyl, indazolyl, benzimidazolyl, benztriazoyl, imidazopyridinyl, triazolopyridinyl, and imidazopyrimidinyl. Suitable 4- to 6-membered heterocyclyls include, but are not limited to, azetidinyl, piperidinyl, morpholinyl, thiomorpholinyl, thiazolidinyl, isothiazolidinyl, oxazolidinyl, isoxazolidinyl, pyrrolidinyl, imidazolidinyl, piperazinyl, tetrahydrofuranyl, tetrahydrothienyl, pyrazolidinyl, hexahydropyrimidinyl, thiazinanyl, thiadiazinanyl, tetrahydropyranyl, tetrahydrothiopyranyl, and dioxanyl.

A "stable" compound is a compound which can be prepared and isolated and whose structure and properties remain or can be caused to remain essentially unchanged for a period of time sufficient to allow use of the compound for the purposes described herein (e.g., therapeutic or prophylactic administration to a subject). Reference to a compound also includes stable complexes of the compound such as a stable hydrate.

As a result of the selection of substituents and substituent patterns, certain of the compounds of the present invention can have asymmetric centers and can occur as mixtures of stereoisomers, or as individual diastereomers, or enantiomers. Unless otherwise indicated, all isomeric forms of these compounds, whether isolated or in mixtures, are within the scope of the present invention. Also included within the scope of the present invention are tautomeric forms of the present compounds as depicted.

When any variable occurs more than one time in any constituent or in Formula (I) or in any other formula depicting and describing compounds of the invention, its definition on each occurrence is independent of its definition at every other occurrence. Also, combinations of substituents and/or variables are permissible only if such combinations result in stable compounds.

The terms "substituted" and "optionally substituted" include mono- and polysubstitution by a named substituent to the extent such single and multiple substitution (including multiple substitution at the same site) is chemically allowed. Hence, the terms specifically contemplate one or more substitutions. Unless expressly stated to the contrary, substitution by a named substituent is permitted on any atom in a ring (e.g., an aryl, a cycloalkyl, a heteroaryl, or a heterocyclyl) provided such ring substitution is chemically allowed and results in a stable compound.

Compounds of the present invention may be administered in the form of "pharmaceutically acceptable salts", hydrates, esters, etc., as appropriate. Other salts may, however, be useful in the preparation of the compounds according to the invention or of their pharmaceutically acceptable salts. For example, when the compounds of the present invention contain a basic amine group, they may be conveniently isolated as trifluoroacetic acid salts (e.g. following HPLC purification). Conversion of the trifluoroacetic acid salts to other salts, including pharmaceutically acceptable salts, may be accomplished by a number of standard methods known in the art. For example, an appropriate ion exchange resin may be employed to generate the desired salt. Alternatively, conversion of a trifluoroacetic acid salt to the parent free amine may be accomplished by standard methods known in the art (e.g. neutralization with an appropriate inorganic base such as NaHCO₃). Other desired amine salts may then be prepared in a conventional manner by reacting the free base with a suitable organic or inorganic acid. Representative pharmaceutically acceptable quaternary ammonium salts include the following: hydrochloride, sulfate, phosphate, carbonate, acetate, tartrate, citrate, malate, succinate, lactate, stearate, fumarate, hippurate, maleate, gluconate, ascorbate, adipate, gluceptate, glutamate, glucoronate, propionate, benzoate, mesylate, tosylate, oleate, lactobionate, laurylsulfate, besylate, caprylate, isetionate, gentisate, malonate, napsylate, edisylate, pamoate, xinafoate, napadisylate, hydrobromide, nitrate, oxalate, cinnamate, mandelate, undecylenate, and camsylate. When the compounds of the invention carry an acidic carboxylic acid moiety, in which case suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

The present invention includes within its scope prodrugs of the compounds of this invention. In general, such prodrugs will be functional derivatives of the compounds of this invention which are readily convertible in vivo into the required compound. Thus, in the methods of treatment of the present invention, the term "administering" shall encompass the treatment of the various conditions described with the compound specifically disclosed or with a compound which may not be specifically disclosed, but which converts to the specified compound in vivo after administration to the patient. Conventional procedures for the selection and preparation of suitable prodrug derivatives are described, for example, in "Design of Prodrugs," ed. H. Bundgaard, Elsevier, 1985, which is incorporated by reference herein in its entirety. Metabolites of these compounds include active species produced upon introduction of compounds of this invention into the biological milieu.

The term "administration" and variants thereof (e.g., "administering" a compound) in reference to a compound of the invention mean providing the compound or a prodrug of the compound to the subject in need of treatment. When a compound of the invention or a prodrug thereof is provided in combination with one or more other active agents (e.g., other antibacterial agents useful for treating bacterial infections), "administration" and its variants are each understood to include concurrent and sequential provision of the compound or prodrug and other agents.

As used herein, the term "composition" is intended to encompass a product comprising the specified ingredients, as well as any product which results, directly or indirectly, from combining the specified ingredients.

By "pharmaceutically acceptable," it is meant that the ingredients of the pharmaceutical composition must be compatible with each other and not deleterious to the recipient thereof.

The term "subject" (alternatively referred to herein as "patient") as used herein refers to an animal, who has been the object of treatment, observation or experiment.

The term "animal" means a bovine, a swine, a member of the poultry family, an ovine, a fish, a horse, a dog, a cat or a human.

The term "effective amount" as used herein means that amount of active compound or pharmaceutical agent that elicits the biological or medicinal response in a tissue, system, animal or human that is being sought by a researcher, veterinarian, medical doctor or other clinician. In one embodiment, the effective amount is a "therapeutically effective amount" for the alleviation of the symptoms of the disease or condition being treated. When the active compound (i.e., active ingredient) is administered as the salt, references to the amount of active ingredient are to the free acid or free base form of the compound.

The term pharmaceutical carrier shall include adjuvants, excipients or vehicles. Examples of such pharmaceutical carriers are provided in Remington's Pharmaceutical Sciences, 20th edition, edited by A. R. Gennaro, Mack Publishing Co., 2000.

For the purpose of treating bacterial infection, the compounds of the present invention, optionally in the form of a salt or a hydrate, can be administered by means that produces contact of the active agent with the agent's site of action. They can be administered by conventional means available for use in conjunction with pharmaceuticals, either as individual therapeutic agents or in a combination of therapeutic agents. They can be administered alone, but typically are administered with a pharmaceutical carrier selected on the basis of the chosen route of administration and standard pharmaceutical practice. The compounds of the invention can, for example, be administered by one or more of the following: orally, parenterally (including subcutaneous injections, intravenous, intramuscular, intrasternal injection or infusion techniques), by inhalation (e.g., nasal or buccal inhalation spray, aerosols from metered dose inhalator, and dry powder inhalator), by nebulizer, ocularly, topically, transdermally, or rectally, in the form of a unit dosage of a pharmaceutical composition containing an effective amount of the compound and conventional non-toxic pharmaceutically-acceptable carriers, adjuvants and vehicles. Liquid preparations suitable for oral administration (e.g., suspensions, syrups, elixirs and the like) can be prepared according to techniques known in the art and can employ the usual media such as water, glycols, oils, alcohols and the like. Solid preparations suitable for oral administration (e.g., powders, pills, capsules and tablets) can be prepared according to techniques known in the art and can employ such solid excipients as starches, sugars, kaolin, lubricants, binders, disintegrating agents and the like. Parenteral compositions can be prepared according to techniques known in the art and typically employ sterile water as a carrier and optionally other ingredients, such as a solubility aid. Injectable solutions can be prepared according to methods known in the art wherein the carrier comprises a saline solution, a glucose solution or a solution containing a mixture of saline and glucose. Further description of methods suitable for use in preparing pharmaceutical compositions of the present invention and of ingredients suitable for use in said compositions is provided in Remington's Pharmaceutical Sciences, 20th edition, edited by A. R. Gennaro, Mack Publishing Co., 2000.

The compounds of this invention can be administered, e.g., orally or intravenously, in a dosage range of, for example, 0.001 to 1000 mg/kg of mammal (e.g., human) body weight per day in a single dose or in divided doses. An example of a dosage range is 0.01 to 500 mg/kg body weight per day orally or intravenously in a single dose or in divided doses. Another example of a dosage range is 0.1 to 100 mg/kg body weight per day orally or intravenously in single or divided doses. For oral administration, the compositions can be provided in the form of tablets or capsules containing, for example, 1.0 to 500 milligrams of the active ingredient, particularly 1, 5, 10, 15, 20, 25, 50, 75, 100, 150, 200, 250, 300, 400, and 500 milligrams of the active ingredient for the symptomatic adjustment of the dosage to the patient to be treated. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention also includes processes for making compounds of Formula (I). The compounds of the present invention may be prepared according to the following reaction schemes and examples, using the appropriate intermediates and starting materials described in the Examples sections below, or modifications thereof.

### Abbreviations

- AcOH: Acetic acid
- Boc: t-Butyloxycarbonyl
- Boc₂O: di-t-Butyl dicarbonate
- BuLi: n-Butyllithium
- ButOH: Butanol
- Cat.: Catalyst
- Cbz: Benzyloxycarbonyl (also CBz)
- CH₃CN: Acetonitrile
- CH₂Cl₂: Dichloromethane
- CsOAc: Cesium carbonate
- DMA: Dimethylacetamide
- DME: Dimethoxyethane
- DCE: Dichloroethane
- DCM: Dichloromethane
- DMF: N,N-Dimethylformamide
- DMS: Dimethyl sulfide
- DMSO: Dimethyl sulfoxide
- DPPA: Diphenyl phosphoryl azide
- Et: Ethyl
- EtOAc or EA: Ethyl acetate
- EtOH: Ethanol
- Et₂O: Diethyl ether
- Et₃N: Triethylamine
- EMME: Diethyl ethoxymethylenemalonate
- H₂: Hydrogen or hydrogen atmosphere
- H₂O: Water
- H₂O₂: Hydrogen peroxide
- H₂SO₄: Sulfuric acid
- HCHO: Formaldehyde
- HCl: Hydrochloric acid
- HMPA: Hexamethylphosphoramide
- IBX: 2-(Iodoxybenzoic acid
- K₂CO₃: Potassium carbonate
- KHMDS: Potassium hexamethyldisilazide
- LAH: Lithium aluminum hydride (LiAlH₄)
- LiCl: Lithium chloride
- LiHMDS: Lithium hexamethyldisilazide
- LDA: Lithium diisopropyl amide
- MCPBA: meta-Chloroperoxybenzoic acid (m-CPBA)
- Me: Methyl
- MeOH: Methanol
- MsCl: Methanesulfonyl chloride
- NaBH₄: Sodium borohydride
- NaCl: Sodium chloride
- NaH: Sodium hydride
- NaIO₄: Sodium periodate
- NaOH: Sodium hydroxide
- NCS: N-chlorosuccinimide
- NH₄Cl: Ammonium chloride
- Na₂SO₄: Sodium sulfate
- NMM: N-Methyl morpholine
- NMO: 4-Methylmorpholine N-oxide
- NMP: N-Methyl pyrrolidinone
- NOBF₄: Nitrosyl tetrafluoroborate
- O₃: Ozone
- OsO₄: Osmium tetroxide
- Pd: Palladium
- PDC: Pyridinium dichromate
- PE: Petroleum Ether
- Ph: Phenyl
- RT or r.t.: Room temperature, approximately 25°C
- SeO₂: Selenium dioxide
- SOCl₂: Thionyl chloride
- t-BuOH: tert-Butanol
- t-BuOK: Potassium t-butoxide
- TBAB: Tetrabutylammonium bromide
- TBME: tert-Butyl methyl ether
- TsCl: Toluenesulfonyl chloride
- TsOH: Toluenesulfonic acid hydrate
- TEA: Triethanolamine
- Tf₂O: Triflic anhydride
- TFA: Trifluoroacetic acid
- THF: Tetrahydofuran
- TLC: Thin layer chromatography
- TMSCl: Trimethysilyl chloride

### EXAMPLES

### Example 1-Intermediate D1

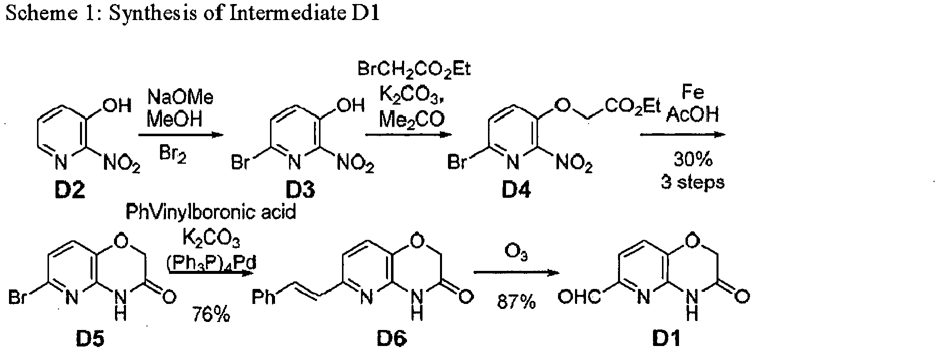

### 6-Bromo-2-nitropyridin-3-ol (D3)

To a solution of D2 (140 g, 1.00 mol) in methanol (2.5 L) was added a solution of sodium methoxide [prepared from Na (24.2 g) and methanol (215 mL)] at room temperature. The mixture was stirred at the same temperature for 30 min. Bromine (51.4 mL, 1.00 mol) was added drop wise to the mixture at 0 °C and the mixture was stirred at the 0 °C temperature for 2 hours. The reaction was quenched with acetic acid (18 mL) and the mixture was concentrated under reduced pressure to give D3, which was used for the next step without further purification.

### Ethyl 2-(6-bromo-2-nitropyridin-3-yloxy)acetate (D4)

To a suspension of the crude D3 and potassium carbonate (277 g, 2.00 mol) in acetone (1.4 L) was added ethyl bromoacetate (111 mL, 1.00 mol), the mixture was heated at reflux for 8 hours. After addition of *t*-butyl methyl ether (1.4 L) the resulting precipitates were filtered off. The filtrate was concentrated under reduced pressure to give D4, which was used for the next step without further purification.

### 6-Bromo-2H-pyrido[3,2-b][1,4]oxazin-3(4H)-one (D5)

A suspension of the crude D4 and iron powder (162 g, 2.90 mol) in acetic acid (1.2 L) was heated at 90 °C for 1.5 hours. After dilution of the mixture with EtOAc (2.4 L), the resulting precipitates were filtered off. The filtrate was concentrated under reduced pressure. Flash column chromatography (hexane/EtOAc = 2:1) of the residue gave D5 (69.0 g, 30%). ¹H NMR (400 MHz, CDCl₃) *δ*4.67 (s, 2H), 7.10 (d, *J =* 8.8 Hz, 1H), 7.14 (d, *J =* 8.8 Hz, 1H), 8.01 (brs, 1H).

### (E)-6-Styryl-2H-pyrido-[3,2-b][1,4]-oxazin-3(4H)-one (D6)

To a degassed solution of D5 (28.9 g 126 mmol) in 1,4-dioxane (630 mL) and water (100 mL) was added phenylvinylboronic acid (19.2 g, 126 mmol), potassium carbonate (35.6 g, 252 mmol) and tetrakis(triphenylphosphine)palladium (4.42 g, 3.79 mmol). The mixture was heated at reflux for 24 hours. After addition of water (720 mL) the resulting precipitates were collected by filtration and washed with water (180 mL). Flash column chromatography (NH silica-gel, hexane/1,4-dioxane = 2:1) of the crude product gave D6 (24.3 g, 76%). ¹H NMR (400 MHz, CDCl₃) *δ*4.68 (s, 2H), 7.01 (d, *J =* 7.9 Hz, 1H), 7.03 *(d, J =* 15.9 Hz, 1H), 7.23 (d, *J* = 7.9 Hz, 1H), 7.36 (t, *J* = 7.3 Hz, 2H), 7.46 (d, *J* = 15.9 Hz, 1H), 7.53 *(d, J =* 7.3 Hz, 1H), 8.09 (brs, 1H).

### 3-Oxo-3,4-dihydro-2H-pyrido[3,2-b][1,4]oxazine-6-carbaldehyde (D1)

A suspension of D6 (24.0 g, 95.2 mmol) in dichloromethane (1.2 L) and methanol (420 mL) was bubbled with ozone at -71 °C until a pale blue color appeared. The excess ozone was removed by bubbling air through the suspension for 30 min. Dimethyl sulfide (36 mL, 490 mmol) was added to the suspension. The mixture was stirred at room temperature for overnight and concentrated under reduced pressure. After addition of diethyl ether (130 mL) and 0.5 M hydrochloric acid (65 mL) the resulting precipitates were collected by filtration and washed with water (40 mL x 3) and diethyl ether (40 mL). Treatment of the crude product with acetone (80 mL) gave D1 (14.7 g, 87%)(19.83% overall yield from D2). ¹H NMR (400 MHz, CDCl₃) *δ*4.80 (s, 2H), 7.39 (d, *J* = 7.9 Hz, 1 H), 7.69 (d, *J* = 7.9 Hz, 1 H), 8.35 (brs, 1H), 9.89 (s, 1 H).

### Example 2- Intermediate L1

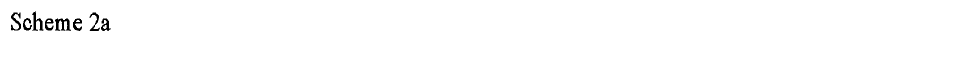

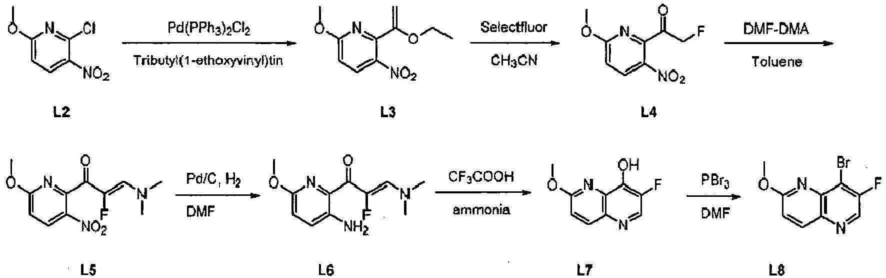

### General procedure for preparation of L3

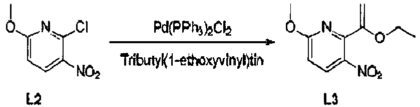

Tributyl(1-ethoxyvinyl)tin (395 mL, 1.34 mol) was added into the mixture of L2 (200 g, 1.07 mol) and Pd(PPh₃)₂Cl₂ (7.34 g, 10.67 mmol) in CH₃CN (1000 mL) at 65 °C. The resulting suspension was stirred at 65 °C overnight then cooled to room temperature. The reaction mixture was quenched with 10% KF aqueous solution (670 mL) and stirred at room temperature for 1 h. Then the mixture was filtered, and the filtrate was extracted with EtOAc. The combined organic phases were dried over Na₂SO₄, concentrated and purified by column chromatography to give 230 g of compound L3 in 96% yield. ¹H NMR (400 MHz CDCl₃) δ 7.92 (d, *J* = 8.8 Hz, 1H), 6.72 (d, *J =* 8.8 Hz, 1H), 4.96 (s, 1H), 4.48 (s, 1H), 4.00 (s, 3H), 3.90∼3.84 (m, 2H), 1.28 (d, *J* = 7.2 Hz, 3H).

### General procedure for preparation of L4

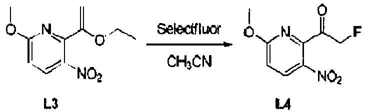

To a suspension of Selectfluor^{®} (1-Fluoro-4-methyl-1,4-diazoniabicyclo[2.2.2] octanebis(tetrafluoroborate) (545 g, 2.05 mol) in CH₃CN (1500 mL) and H₂O (750 mL) was added L3 (230 g, 1.02 mol) dropwise in CH₃CN (750 mL) over 15 min and the resulting mixture was stirred at room temperature overnight. Then water was added, and the mixture was extracted with EtOAc. The combined organic phases were dried over Na₂SO₄, and concentrated to give 200 g of compound L4 in 91% yield. ¹H NMR (400 MHz CDCl₃) δ 8.29 (d, *J* = 9.2 Hz, 1H), 6.92 (d, *J* = 9.2 Hz, 1H), 5.30 (d, *J* = 47.2 Hz, 2H), 4.03 (s, 3H).

### General procedure for preparation of L5

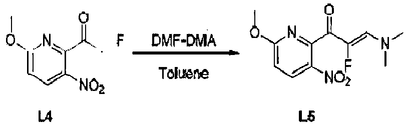

To a solution of L4 (200 g, 0.93 mol) in toluene (1200 mL) was added DMF-DMA (824 mL). The reaction mixture was heated to 50 °C and stirred overnight under N₂. Then the mixture was cooled to room temperature and filtered to give 200 g compound L5 as a yellow solid in 80% yield. ¹H NMR (400 MHz CDCl₃) δ 8.27 (d, *J* = 9.2 Hz, 1H), 7.05 (d, *J* = 28.4 Hz, 0.6H), 6.77 (t, *J =* 9.2 Hz, 1H), 6.05 (d, *J =* 26.8 Hz, 0.4H), 3.98 (s, 3H), 3.06 (s, 6H).

### General procedure for preparation of L6

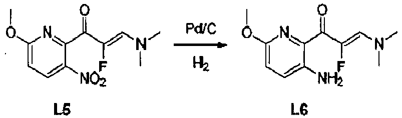

To a mixture of L5 (200 g, 0.74 mol) in DMF (1000 mL) was added Pd/C (20 g, 10% wt.). The mixture was stirred for 2 h at 50 °C under H₂ (1.0 kg/cm²). The reaction mixture was filtered and the filtrate containing L6 was used in the next step directly.

### General procedure for preparation of L7

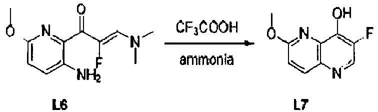

To a solution containing L6 (200 g, 0.85 mol) in DMF from above was added TFA/DCM (150 mL/150 mL) and the mixture was stirred at room temperature overnight. The residue was treated with 2M ammonia in MeOH (385 mL). Then the mixture was cooled to 0 °C and filtered to give 90 g compound L7 as a white solid in 56% yield.

### General procedure for preparation of L8

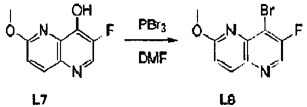

To a solution of L7 (90 g, 0.45 mol) in DMF (900 mL) was added PBr₃ (45 mL) at 0 °C. The mixture was stirred at room temperature overnight. Then the mixture was poured into ice water and filtered to give 105 g of product L8 as a white solid in 88% yield. ¹H NMR (400 MHz CDCl₃) δ 8.58 (s, 1H), 8.15 (d, *J* = 9.2 Hz, 1H), 7.08 *(d, J=* 9.2 Hz, 1H), 4.13 (s, 3H).

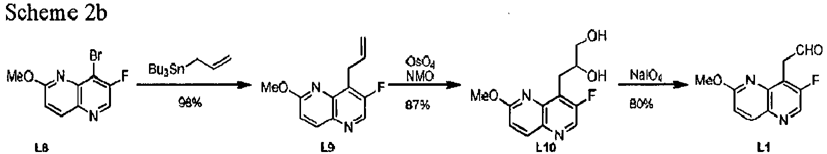

### 8-Allyl-7-fluoro-2-methoxy-1,5-naphthyridine (L9)

A mixture of L8 (5.00 g, 19.5 mmol), allyltributyltin (6.33 mL, 20.4 mmol), bis(tri-*tert-*butylphosphine)palladium(0) (Pd(*t*-Bu₃P)₂ 298 mg, 0.584 mmol), and cesium fluoride (CsF, 8.86 mg, 0.0584 mmol) in degassed 1,4-dioxane (50.0 mL) was stirred at 70 °C for 1 hour. Sufficient EtOAc was added and the mixture was washed with 10% aqueous potassium fluoride solution. The organic layers were dried over anhydrous magnesium sulfate, filtered, and concentrated in vacuo. The residue was chromatographed by flash chromatography on silica gel (hexane/EtOAc = 3:1) to furnished L9 (4.17 g, 98%). ¹H NMR (400 MHz, CDCl₃): *δ*3.96 (dd, J = 6.7, 1.2 Hz, 2H), 4.09 (s, 3H), 5.05(dd, J =9.8, 1.2 Hz, 1H), 5.17 *(d, J=* 17.1 Hz, 1H), 6.02-6.11(m, 1H), 7.07(d, *J* = 9.2 Hz, 1H),8.17 (d, *J =* 9.2 Hz, 1H), 8.63 (s, 1H).

### 3-(3-Fluoro-6-methoxy-1,5-naphthyridin-4-yl)propane-1,2-diol L10

A mixture of L9 (2.55 g, 11.7 mmol), *N*-methylmorpholine-*N-*oxide (NMO, 12.2 mL, 58.4 mmol, 50% in water), and osmium tetroxide (7.32 mL, 0.584 mmol, 2.5 wt% in *tert*-butanol) in acetone (50.0 mL) and water (10.0 mL) was stirred at room temperature for 85 minutes. Sodium sulfite (7.5 g, 59.5 mmol) was added to the reaction mixture, and the resulting mixture was stirred at room temperature for 25 minutes. The product from the reaction mixture was extracted with EtOAc after addition of water. The EtOAc extract was dried over anhydrous magnesium sulfate, filtered, and concentrated under reduced pressure to give L10 (2.56 g, 87%). The resulting diol was used for the next reaction without further purification. ¹H NMR (400 MHz, DMSO-*d*₆): *δ* 3.04-3.09 (m, 1H), 3.30-3.44 (m, 3H), 3.94-4.01 (m, 1H), 4.03 (s, 3H), 4.60 (t, *J* = 5.5 Hz, 1H), 4.67 (d, *J* = 5.5 Hz, 1H), 8.22 (d, *J* = 8.6 Hz, 1H), 8.27 (d, *J* = 8.6 Hz, 1H), 8.75 (s, 1H). MS (CI⁺) *m*/*z:* 253 (MH⁺).

### 2-(3-Fluoro-6-methoxy-1,5-naphthyridin-4-yl)acetaldehyde L1

Sodium periodate (8.48 g, 39.6 mmol) was added to a solution of L10 (2.00 g, 7.93 mmol) in tetrahydrofuran (100 mL) and water (50 mL) and the mixture was stirred at room temperature for 140 minutes. After addition of water, the mixture was extracted with EtOAc. The organic extract was dried over anhydrous magnesium sulfate, filtered, and EtOAc was removed under vacuo. Flash chromatography (hexane/EtOAc = 3:1) of the residue gave L1 (1.40 g, 80%). ¹H NMR (400 MHz, CDCl₃): *δ* 4.02 (s, 3H), 4.20-4.21 (m, 2H), 7.11 (d, *J* = 9.2 Hz, 1H), 8.22 (d, *J* = 9.2 Hz, 1H), 8.72 (s, 1H), 9.86-9.87 (m, 1H).

### Example 3 - Compound A1

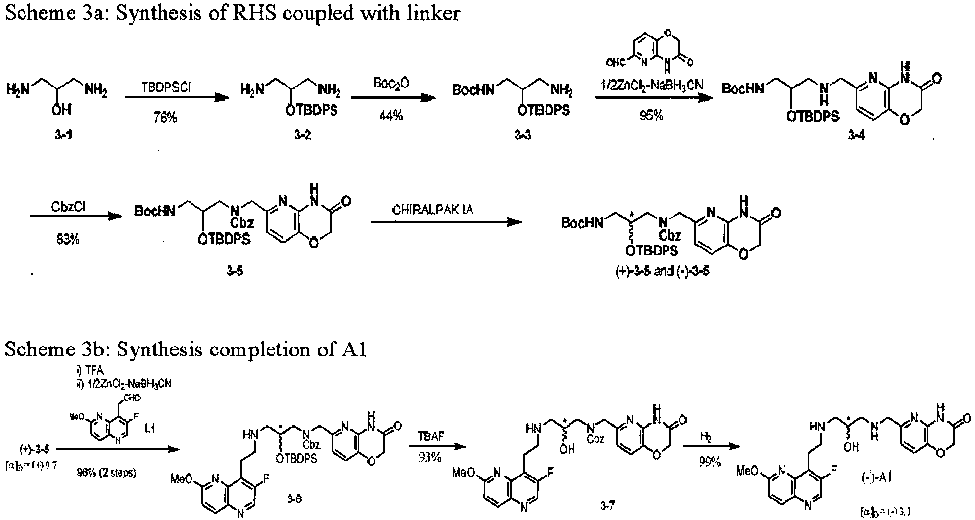

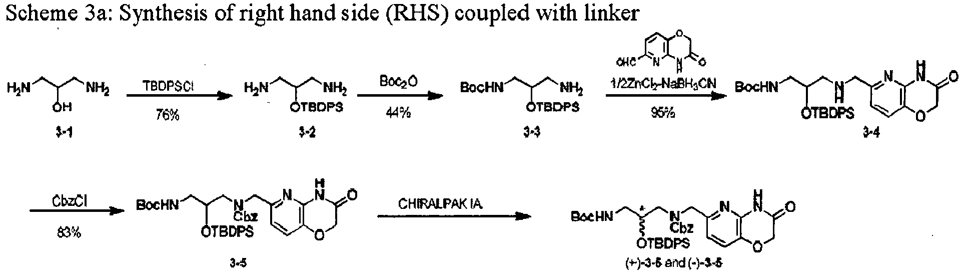

### 2-(tert-Butyldiphenylsilyloxy)propane-1,3-diamine (3-2)

To a solution of 3-1 (3.00 g, 33.3 mmol) in dichloromethane (300 mL) was added triethylamine (5.10 mL, 36.6 mmol), 4-dimethylaminopyridine (DMAP, 40.7 mg, 0.333 mmol), and *tert-*butyldiphenylsilyl chloride (TBDPSC1, 9.13 mL, 35.0 mmol), and the mixture was stirred at room temperature for 5 hours. The solvent was removed by concentration in vacuo and chromatographed by flash chromatography on silica gel (chloroform/methanol = 5:1) to give 3-2 (8.28 g, 76%). ¹H NMR (400 MHz, CDCl₃): *δ* 1.08 (s, 9H), 2.70-2.79 (m, 4H), 3.60-3.63 (m, 1H), 7.36-7.45 (m, 6H), 7.67-7.70 (m, 4H). MS (CI⁺) *m*/*z:* 329 (MH⁺).

### tert-Butyl 3-amino-2-(tert-butyldiphenylsilyloxy)propylcarbamate (3-3)

A solution of di-*tert*-butyl dicarbonate (Boc₂O, 1.91 g, 8.77 mmol) in 1,4-dioxane (20 mL) was added to a solution of 3-2 (4.32 g, 13.1 mmol) in 1,4-dioxane (50 mL) was added drop wise over 3 hours. The mixture was stirred at room temperature for 4 hours and then solvent was removed by concentration under reduced pressure. Dichloromethane (300 mL) was added to the residue and washed with water. The organic layer were dried over anhydrous magnesium sulfate, filtered, and then concentrated in vacuo. The residue was chromatographed by flash chromatography on silica gel (chloroform/methanol = 9:1) to give 3-3 (2.46 g, 44%). ¹H NMR (400 MHz, CDCl₃): *δ* 1.08 (s, 9H), 1.16 (br, 2H), 1.41 (s, 9H), 2.67 (qd, *J =* 5.5, 13.4 Hz, 2H), 3.22 (t, *J =* 5.5 Hz, 2H), 3.69 (t, J = 5.5 Hz, 1H), 4.76 (br, 1H), 7.36-7.45 (m, 6H), 7.65-7.67 (m, 4H). MS (CI⁺) *m*/*z:* 429 (MH⁺).

### tert-Butyl 2-(tert-butyldiphenylsilyloxy)-3-((3-oxo-3,4-dihydro-2H-pyrido[3,2-b][1,4]oxazin-6-yl) methylamino) propylcarbamate (3-4)

To a solution of 3-3 (265 mg, 0.617 mmol) in chloroform (6.17 mL) and methanol (2.00 mL) was added D1 (100 mg, 0.561 mmol), and the mixture was heated until dissolved completely. The reaction was allowed to cool to room temperature and zinc chloride-sodium cyanoborohydride (1/2 ZnCl₂-NaBH₃CN, 0.3 M in MeOH, 1.87 mL, 0.561 mmol,) was added and the mixture was stirred at room temperature for 1 hour. Water was added and the reaction mixture was extracted with dichloromethane. The organic extracts were dried over anhydrous magnesium sulfate, filtered, and then concentrated under reduced pressure and flash chromatographed on silica gel (chloroform/methanol = 9:1) to give 3-4 (314 mg, 95%). ¹H NMR (400 MHz, CDCl₃): *δ* 1.07 (s, 9H), 1.42 (s, 9H), 2.55-2.70 (m, 2H), 3.25-3.40 (m, 2H), 3.58 (s, 2H), 3.80-3.95 (m, 1H), 4.59-4.66 (m, 2H), 5.07 (br, 1H), 6.77 (d, *J* = 7.9 Hz, 1H), 7.13 (d, *J* = 7.9 Hz, 1H), 7.35-7.44 (m, 6H), 7.65 (d, *J* = 6.7 Hz, 4H). MS (ESI⁺) *m*/*z:* 591 (MH⁺).

### Benzyl 3-tert-butoxycarbonylamino-2-(tert-butyldiphenylsilyloxy)propyl((3-oxo-3,4-dihydro-2H- pyrido[3,2-b][1,4]oxazin-6-yl)methyl)carbamate (3-5)

To a mixture of 3-4 (725 mg, 1.23 mmol) in saturated aqueous solution of sodium hydrogen carbonate (4.92 mL) and tetrahydrofuran (12.3 mL) was added benzyl chloroformate (194 microL, 1.29 mmol) at 0 °C. The mixture was stirred at 0 °C for 3 hours. Water (50 mL) was added to the mixture and extracted with EtOAc. The organic layer were dried over anhydrous magnesium sulfate, filtered, concentrated in vacuo and flash chromatographed on silica gel (hexane/EtOAc = 2:1) to afford 3-5 (741 mg, 83%). ¹H NMR (400 MHz, CDCl₃, mixture of rotamers): *δ* 1.04,1.07(each s, total 9H), 1.45 (s, 9H), 2.99-3.01 (m, 1H), 3.10-3.14 (m, 1H), 3.47-3.70 (m, 2.5 3H), 4.07-4.15 (m, 1H), 4.19-4.35 (m, 1H), 4.58 (s, 2H), 4.93-5.05 (m, 2.5H), 5.23 (br, 0.5H), 6.44 *(d, J =* 8.0 Hz, 0.5H), 6.69 (d, *J =* 8.0 Hz, 0.5H), 6.99 (d, *J =* 8.0 Hz, 0.5H), 7.07-7.11 (m, 1.5 H), 7.24-7.41 (m, 10H), 7.60-7.66 (m, 4H), 8.99-9.05 (m, 1H). MS (ESI⁺) *m*/*z:* 725 (MH⁺).

Optical resolution of 3-5 (analytical conditions) Column: CHRALPAK IA (hexane/2-propanol = 8:2), Flow: 0.5 mL/min. (+)-3-5: t_{R} = 15.58 min, [α]_{D}²³ = +9.7 (c 0.3, MeOH), (-)-3-5: t_{R} = 19.68 min, [α]_{D}²⁴ = -5.6 (c 0.3, MeOH).

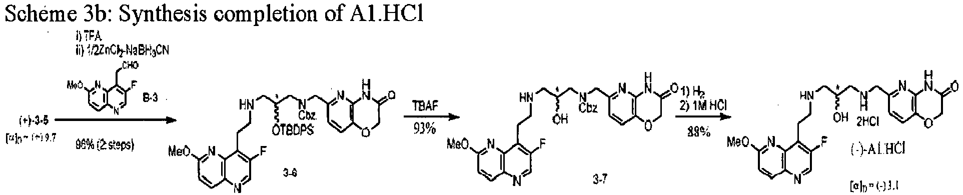

### Benzyl 2-(tert-butyldiphenylsilyloxy)-3-(2-(3-fluoro-6-methoxy-1,5-naphthyridin-4-yl) ethylamino)propyl((3-oxo-3,4-dihydro-2H-pyrido[3,2-b][1,4]oxazin-6-yl)methyl)carbamate (3-6)

A solution of (+)-3-5 (488 mg, 0.673 mmol) in trifluoroacetic acid-chloroform (1:1, 3.37 mL) was stirred at room temperature for-10 minutes and then concentrated in vacuo. Flash chromatography (NH silica, chloroform/methanol = 20:1) of the residue gave the deprotected amine (438 mg) as colorless foam which was dissolved in chloroform (6.73 mL) and was added L1 (148 mg, 0.673 mmol) and zinc chloride-sodium cyanoborohydride (1/2ZnCl₂-NaBH₃CN, 0.3 M in MeOH, 2.24 mL, 0.673 mmol) followed by stirring the mixture at room temperature for 2 hours. Water was added and the mixture was extracted with dichloromethane. The lower layer was dried over anhydrous magnesium sulfate, filtered, and concentrated in vacuo. Flash chromatography on silica gel (chloroform/methanol = 19:1) of the residue gave 3-6 (538 mg, 96%). [α]_{D}²¹ = +14.2 (c 0.3, MeOH), ¹H NMR (400 MHz, CDCl₃, mixture of rotamers): *δ* 0.97 0.99 (each s, total 9H), 2.55 (dd, *J =* 5.5, 11.6 Hz, 0.5H), 2.66-2.74 (m, 1H), 2.78-2.83 (m, 1H), 2.89-2.99 (m, 1.5H), 3.12 (ddd, *J* = 4.9, 14.1, 23.2 Hz, 1H), 3.28-3.32 (m, 0.5H), 3.36-3.42 (m, 1.5 H), 3.52 (dd, *J* = 9.2, 14.7 Hz, 0.5H), 3.67 (dd, *J* = 8.0, 14.1 Hz, 0.5H), 3.76 (dd, *J* = 8.0, 15.3 Hz, 1H), 4.00-, 4.05 (each s, total 3H), 4.23-4.38 (m, 2H), 4.60, 4.61 (each s, total 2H), 4.93-5.03 (m, 2H), 6.49, 6.67 (each d, *J =* 7.9 Hz, total 1H), 7.01-7.09 (m, 3H), 7.22-7.38 (m, 10H), 7.56-7.63 (m, 4H), 8.17 (dd, *J =* 2.4, 9.2 Hz, 1H), 8.59 *(d, J =* 3.1 Hz, 1H), MS (ESI⁺) *m*/*z:* 829 (MH⁺).

### Benzyl 3-(2-(3-fluoro-6-methoxy-1,5-naphthyridin-4-yl)ethylamino)-2-hydroxypropyl ((3-oxo-3,4-dihydro-2H-pyrido[3,2-b][1,4]oxazin-6-yl)methyl)carbamate (3-7)

To a solution of 3-6 (518 mg, 0.625 mmol) in tetrahydrofuran (6.25 mL) was added a solution of tetrabutylammonium fluoride (TBAF, 1.25 mL, 1 mol/L in THF) and the mixture was stirred at room temperature for 4 hours. After addition of 50 mL water the mixture was extracted with EtOAc. The organic extracts were dried over anhydrous magnesium sulfate, filtered, and the solvent was removed by concentration under reduced pressure. The resulting residue was flash chromatographed on silica gel (chloroform/methanol = 9:1) to afford 3-7 (343 mg, 93%). ¹H NMR (400 MHz, CDCl₃, mixture of rotamers): *δ* 2.61-2.71 (m, 1H), 2.81 (dt, *J =* 3.7, 11.6 Hz, 1H), 2.99-3.10 (m, 2H), 3.24-3.43 (m, 3H), 3.54-3.3.63 (m, 1H), 3.96-3.98 (m, 1H), 4.03, 4.07 (each s, total 3H), 4.27-4.52 (m, 2H), 4.57-4.60 (m, 2H), 5.02-5.07 (m, 2H), 6.67 (d, *J* = 8.0 Hz, 0.5H), 6.95 *(d, J=* 8.6 Hz, 0.5H), 7.04-7.09 (m, 1.5H), 7.10-7.15 (m, 1H), 7.18-7.23 (m, 1H), 7.27-7.29 (m, 3.5H), 8.15 (dd, *J* = 1.8, 9.2 Hz, 1H), 8.60 (s, 1H). MS (ESI⁺) *m*/*z:* 591 (MH⁺).

### Synthesis of A1.HCl

A suspension of 3-7 (327 mg, 0.554 mmol) and 10% Pd-C (65.4 mg) in acetic acid (5.54 mL) was stirred at room temperature for 2 hours under H₂ atmosphere (1 kg/cm²). After filtration of catalysts, the filtrate was concentrated in vacuo. Flash chromatography (NH silica, chloroform/methanol = 19:1) of the residue gave free base (-)-A1 (255 mg). [α]_{D}²⁵ = -3.1 (c 0.3, EtOH), ¹H NMR (400 MHz, CDCl₃): *δ* 2.61-2.64 (m, 2H), 2.67-2.73 (m, 2H), 3.06-3.11 (m, 1H), 3.25-3.35 (m, 2H), 3.55-3.67 (m, 3H), 3.80-3.88 (m, 1H), 4.12 (s, 3H),4.35 (d, J = 15.9Hz, 1H), 4.42 (d, *J* = 15.3 Hz, 1H), 6.69 (d, J = 7.9Hz, 1H), 6.81 (d, *J* = 9.2 Hz, 1H), 7.02 (d, *J =* 7.9 Hz, 1H), 7.91 (d, *J =* 8.6 Hz, 1H), 8.56 (s, 1H).

To a solution of free base A-1 in EtOH (10.5 mL) and THF (5.26 mL) was added 1M HCl solution (1.05 mL). The mixture was stirred at room temperature and concentrated under reduced pressure. The residue was dissolved in MeOH, filtered and concentrated under reduced pressure. Trituration of the residue gave (-)-A1.HCl (222 mg, 88%). ¹H NMR (400 MHz, DMSO-*d*₆) δ 3.04 (br, 2H), 3.18 (br, 2H), 3.32-3.37 (m, 2H, overlapped with H₂O), 3.55-3.59 8m, 2H), 4.10 (s, 3H), 4.15 (s, 2H), 4.28 (br, 1H), 4.68 (s, 2H), 6.21 (d, J = 5.5Hz, 1H), 7.20 (d, J = 7.9Hz, 1H), 7.27 (d, J = 9.2Hz, 1H), 7.43 (d, J = 9.2Hz, 1H), 8.84 (s, 1H), 9.20 (br, 2H), 9.34 (br, 2H), 11.3 (s, 1H). MS (ESI⁺) *m*/*z:* 457 (MH⁺).

(+)-A1 can be prepared following scheme 3b and starting with (-) 3-5. The racemic (±)-A1 can be prepared by following scheme 3b using 3-5 without chiral separation.

### Example 4 - Compound A2

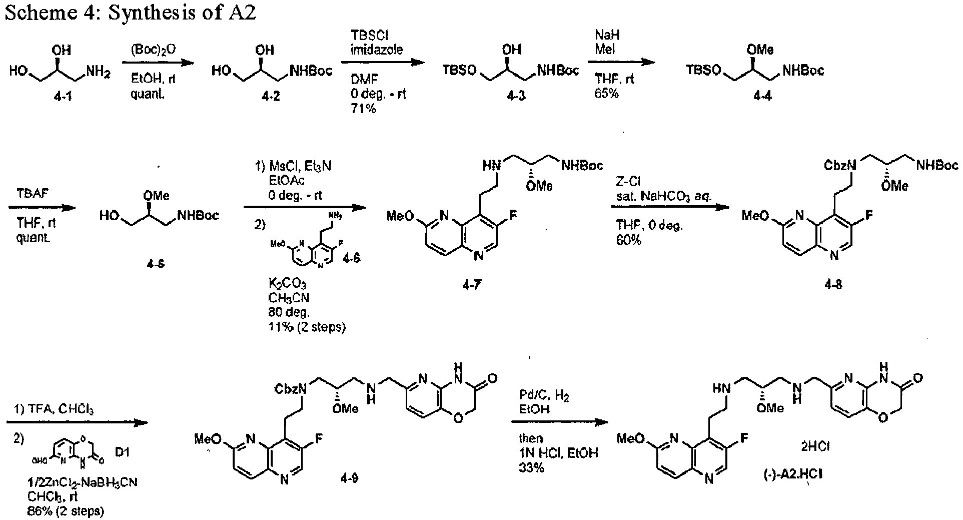

### (S)-tert-Butyl (2,3-dihydroxypropyl)carbamate (4-2)

To a solution of 4-1 (3.0 g, 32.9 mmol) in EtOH (110 mL) was added (Boc)₂O (7.91 g, 36.2 mmol), and the mixture was stirred at room temperature for 1.75 hours. The reaction mixture was concentrated and flash chromatographed on silica gel (CHCl₃/MeOH = 9:1) to give the residue of 4-2 (6.35 g, quant.). ¹H NMR (400 MHz, CDCl₃) δ 1.45 (s, 9H), 3.16-3.32 (m, 4H), 3.53-3.64 (m, 2H), 3.71-3.77 (m, 1H), 5.07 (brs, 1H).

### (S)-tert-Butyl (3-((tert-butyldimethylsilyl)oxy)-2-hydroxypropyl)carbamate (4-3)

To a solution of 4-2 (3.00 g, 15.7 mmol) and imidazole (1.28 g, 18.8 mmol) in DMF (60.4 mL) was added TBSC1 (2.62 g, 17.3 mmol) at 0 °C, and the mixture was stirred at room temperature for 3.5 hours. The reaction mixture was concentrated under reduced pressure and the residue was diluted with CH₂Cl₂ (50 mL). After the insoluble was filtered off, filtrate was concentrated under reduced pressure. Flash chromatography of the residue on silica gel (hexane/EtOAc = 2:1) gave 4-3 (3.40 g, 71%). ¹H NMR (400 MHz, CDCl₃) δ 0.07 (s, 6H), 0.90 (s, 9H), 1.45 (s, 9H), 2.83-2.84 (m, 1H), 3.09-3.15 (m, 1H), 3.32-3.38 (m, 1H), 3.53 (dd, *J* = 9.8, 6.1 Hz, 1H), 3.64 (dd, *J =* 9.8, 4.9 Hz, 1H), 3.71-3.77 (m, 1 H), 4.97 (brs, 1H).

### (S)-tert-Butyl (3-((tert-butyldimethylsilyl)oxy)-2-methoxypropyl)carbamate (4-4)

To a solution of 4-3 (1.0 g, 3.27 mmol) in THF (32.7 mL) was added NaH (144 mg, 3.60 mmol) and the mixture was stirred at room temperature for 30 minutes. MeI (407 microL, 6.55 mmol) was added and the mixture was stirred at room temperature for 3 hours. After quenching by addition of water, the mixture was extracted with EtOAc. The organic extracts were washed with brine, dried over magnesium sulfate and concentrated under reduced pressure. Flash chromatography (hexane/EtOAc = 5:1) of the residue on silica gel gave 4-4 (682 mg, 65%). ¹H NMR (400 MHz, CDCl₃) δ 0.06 (s, 6H), 0.90 (s, 9H), 1.44 (s, 9H), 3.11-3.24 (m, 1H), 3.29-3.40 (m, 2H), 3.42 (s, 3H), 3.60-3.70 (m, 2H), 4.93 (brs, 1H).

### (S)-tert-Butyl (3-hydroxy-2-methoxypropyl)carbamate (4-5)

A mixture of 4-4 (400 mg, 1.27 mmol) and TBAF (1.0 M THF solution) (1.39 mL, 1.39 mmol) in THF (6.35 mL) was stirred at room temperature for 1 hour, and then concentrated under reduced pressure. Flash chromatography (CHCl₃/MeOH = 19:1) of the residue on silica gel gave 4-5 (274 mg, quant.). ¹H NMR (400 MHz, CDCl₃) δ 1.45 (s, 9H), 2.90 (br, 0.3H), 2.98-3.01 (m, 0.7H), 3.11-3.17 (m, 0.3H), 3.24-3.46 (m, 5.7H), 3.57-3.61 (m, 1.7H), 3.84-3.88 (m, 0.3H), 4.87 (br, 0.7H), 4.96 (br, 0.3H).

### Preparation of intermediate compound 4-6'

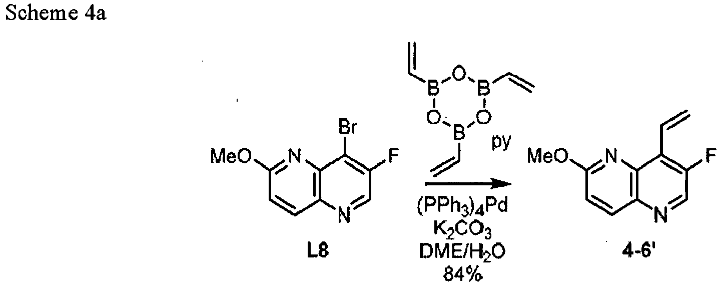

To a degassed solution of L8 (3.14 g, 12.2 mmol) in dimethoxyethane (80 mL) and water (24 mL) was added vinylboronic anhydride pyridine complex (4.41 g, 18.3 mmol), potassium carbonate (1.72 g, 12.2 mmol) and tetrakis(triphenylphosphine)palladium (0.855 g, 0.733 mmol), the mixture was stirred at 100 °C for 3.5 hours. After the mixture was diluted with water it was extracted with ether. The organic extracts were dried over anhydrous sodium sulfate, filtered, and then concentrated in vacuo. Flash chromatography (hexane/EtOAc = 10:1) of the residue gave 4-6' (2.20 g,84%) ¹H NMR (400 MHz, CDCl₃) δ 4.10 (s, 3H), 5.94 (dt, *J* = 12.2, 1.8 Hz, 1H), 6.60 (dd, *J* = 18.3, 1.8 Hz, 1H), 7.09 (d, *J* = 9.2 Hz, 1H), 7.57 (dd, *J* = 18.3, 12.2 Hz, 1H), 8.18 *(d, J =* 9.2 Hz, 1H), 8.66 (d, *J =* 2.4 Hz, 1H).

The synthetic procedure for intermediates 4-6" and 4-6

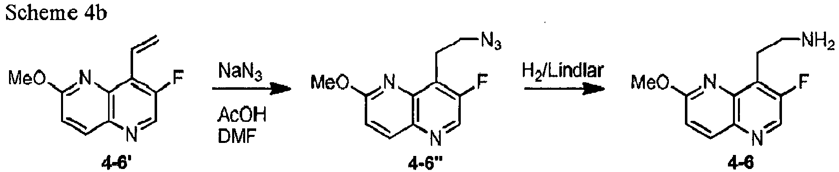

### 8-(2-Azidoethyl)-7-fluoro-2-methoxy-1,5-naphthyridine (4-6")

To a solution of 4-6' (1.00 g, 4.90 mmol) in AcOH (9.8 mL) was added a solution of NaN₃ (1.59 g, 24.5 mmol) in water (9.8 mL) and then DMF (6.0 mL) at room temperature, the mixture was stirred at the same temperature for 19 hours. The mixture was adjusted to pH 10 by adding aqueous Na₂CO₃ solution (40 mL) and extracted with EtOAc. The organic extracts were washed with brine, dried over Na₂SO₄ and concentrated under reduced pressure. Flash chromatography (hexane/acetone = 10:1) of the residue gave 4-6" (1.13 g, 93%). ¹H NMR (400 MHz, CDCl₃) δ *3.5 2 (t, J =* 7.3 Hz, 2H), 3.68 (t, *J =* 7.3 Hz, 2H), 4.10 (s, 3H), 7.10 (d, *J* = 8.6 Hz, 1H), 8.20 (d, *J* = 9.2 Hz, 1H), 8.66 (s, 1H).

### 2-(3-Fluoro-6-methoxy-1,5-naphthyridin-4-yl)ethanamine (4-6)

A suspension of 4-6" (1.00 g, 4.04 mmol) and Lindlar catalyst (300 mg, 30 wt%) in MeOH (40 mL) was stirred at room temperature under H₂ atmosphere (1 kg/cm²) for 2.5 hours. Catalyst was removed by filtration and the filtrate was concentrated under reduced pressure. Flash chromatography (CH₂Cl₂/MeOH/c.NH₄OH = 10:1:0.1) of the residue gave 4-6 (928.8 mg, quant.). ¹H NMR (400 MHz, CDCl₃) δ 3.13 (t, *J =* 6.7 Hz, 2H), 3.36 (t, *J =* 6.7 Hz, 2H), 4.09 (s, 3H), 7.08 (d, *J* = 8.6 Hz, 1H), 8.18 (d, *J* = 8.6 Hz, 1H), 8.64 (s, 1H).

### (R)-tert-Butyl (3-((2-(3-fluoro-6-methoxy-1,5-naphthyridin-4-yl)ethyl)amino)-2-methoxypropyl)carbamate (4-7)

To a solution of 4-5 (260 mg, 1.27 mmol) and Et₃N (300 micro L, 2.15 mmol) in EtOAc (6.35 mL) was added MsCl (137 microL, 1.77 mmol) at 0 °C, and the mixture was stirred at room temperature for 1 hour. After the insoluble was filtered off, filtrate was concentrated under reduced pressure. Flash chromatography (CHCl₃/MeOH = 20:1) of the residue gave mesylate (371 mg, quant.).

To a solution of mesylate in CH₃CN (6.35 mL) was added 4-6 (281 mg, 1.27 mmol) and K₂CO₃ (351 mg, 2.54 mmol), and the mixture was stirred at 80 °C for 15 hours. After addition of water, the mixture was extracted with EtOAc. The organic extracts were washed with brine, dried over magnesium sulfate and concentrated under reduced pressure. Preparative silica gel TLC (CHCl₃/MeOH = 9:1) of the residue gave 4-7 (58.4 mg, 11%). ¹H NMR (400 MHz, CDCl₃) δ 1.42 (s, 9H), 2.75-2.82 (m, 2H), 3.02-3.07 (m, 2H), 3.24-3.32 (m, 2H), 3.36 (s, 3H), 3.38-3.43 (m, 3H), 4.09 (s, 3H), 4.99 (br, 1H), 7.07 (d, *J =* 8.6 Hz, 1H), 8.18 (d, *J* = 8.6 Hz, 1H), 8.62 (s, 1H).

### (S)-Benzyl (3-((tert-butoxycarbonyl)amino)-2-methoxypropyl)(2-(3-fluoro-6-methoxy-1,5-naphthyridin-4-yl)ethyl)carbamate (4-8)

To a solution of 4-7 (55.0 mg, 0.135 mmol) in THF (2.0 mL) and saturated aqueous solution of NaHCO₃ (1.0 mL) was added Cbz-Cl (22.3 micro L, 0.148 mmol) at 0 °C, and the mixture was stirred at the same temperature for 2 hours. After addition of water, the mixture was extracted with EtOAc. The organic extracts were washed with brine, dried over magnesium sulfate and concentrated under reduced pressure. Flash chromatography (CHCl₃/MeOH = 19:1) of the residue gave 4-8 (43.7 mg, 60%). ¹H NMR (400 MHz, CDCl₃) δ 1.41-1.43 (m, 9H), 3.12-3.50 (m, 10 H), 3.68-3.88 (m, 2H), 3.99 (s, 2H), 4.12 (s, 1H), 4.93 (d, *J =* 12.2 Hz, 1H), 4.99 (d, *J =* 12.2 Hz, 1H), 5.26 (br, 1H), 7.01-7.08 (m, 1H), 7.20-7.34 (m, 5H), 8.11-8.18 (m, 1H), 8.57-8.59 (m, 1H).

### (S)-Benzyl (2-(3-fluoro-6-methoxy-1,5-naphthyridin-4-yl)ethyl)(2-methoxy-3-(((3-oxo-3,4-dihydro- 2H-pyrido[3,2-b][1,4]oxazin-6-yl)methyl)amino)propyl)carbamate (4-9)

To a solution of 4-8 (43.7 mg, 0.0805 mmol) in CHCl₃ (1 mL) was added TFA (1 mL), the mixture was stirred at room temperature for 10 min and then concentrated under reduced pressure to give TFA salt of the free amine which was passed through ion exchange resin (PoraPak Rxn CX20cc, MeOH then 5%NH₃ in MeOH) to remove TFA. The fraction of 5%NH₃ in MeOH was concentrated under reduced pressure to give free amine. To a solution of the residue in CHCl₃ (2.0 mL) was added D1 (12.8 mg, 0.0718 mmol), and the mixture was stirred at room temperature for 30 minutes. 1/2ZnCl₂-NaBH₃CN (0.3 M MeOH solution) (239 micro L, 0.0718 mmol) was added to the reaction mixture and the mixture was stirred at room temperature for 1 hour. After quenching the reaction by addition of water, the mixture was extracted with CH₂Cl₂. The organic extracts were dried over magnesium sulfate, filtered, and then concentrated under reduced pressure. Preparative TLC (CHCl₃/MeOH = 9:1) of the residue gave 4-9 (41.7 mg, 86%). ¹H NMR (400 MHz, CDCl₃, mixture of rotamers) δ 2.49-2.77 (m, 2H), 3.25-3.81 (m, H), 3.97 (s, 2H), 4.10 (s, 1H), 4.63 (s, 2H), 4.97 (s, 1.3H), 5.12 (s, 0.7H), 6.78 (d, *J =* 7.9 Hz, 0.3H), 6.88 (d, *J =* 8.6 Hz, 0.7H), 7.01-7.08 (m, 1H), 7.13-7.17 (m, 1H), 7.22-7.35 (m, 5H), 8.13-8.20 (m, 1H), 8.58, 8.60 (each s, total 1H).

### (R)-6-(((3-((2-(3-Fluoro-6-methoxy-1,5-naphthyridin-4-yl)ethyl)amino)-2-methoxypropyl)amino)methyl)-2H-pyrido[3,2-b][1,4]oxazin-3(4H)-one dihydrochloride (A2)

A suspension of 4-9 (40.0 mg, 0.0662mmol) and 10% Pd/C (12.0 mg, 30 wt%) in EtOH (1.3 mL) was stirred at room temperature under H₂ atmosphere (1 kg/cm²) for 3 hours. Catalyst was removed by filtration and the filtrate was concentrated under reduced pressure. Preparative TLC (CH₂Cl₂/MeOH = 9:1) of the residue gave free base of (-)-A2 (10.5 mg).

To a solution of free base of A2 (10.5 mg, 0.0223 mmol) in EtOH (223 micro L) was added 1N HCl solution (44.6 micro L, 0.0446 mmol), and the mixture was stirred at room temperature for 10 min and concentrated under reduced pressure. Trituration of the residue with Et₂O gave (-)- A2.HCl (11.8 mg, 33%) free base: ¹H NMR (400 MHz, CDCl₃) δ 2.67-2.77 (m, 2H), 2.88 (d, *J =* 5.5 Hz, 2H), 3.06-3.19 (m, 2H), 3.35 (s, 3H), 3.35-3.50 (m, 3H), 3.68 (s, 2H), 4.09 (s, 3H), 4.62 (s, 2H), 6.83 (d, *J =* 7.9 Hz, 1H), 7.05 (d, *J* = 9.2 Hz, 1H), 7.16 (d, *J* = 7.9 Hz, 1H), 8.16 (d, *J* = 9.2 Hz, 1H), 8.61 (s, 1H). [α]_{D}²⁵ -9.5 (c 0.2, CHCl₃)

The enantiomer (+)-A2 can be synthesized by starting with enantiomer of 4-1 following scheme 4. Likewise the racemic (+)-A2 can be prepared by starting with racemic 4-1.

### Example 5 - Compound A3

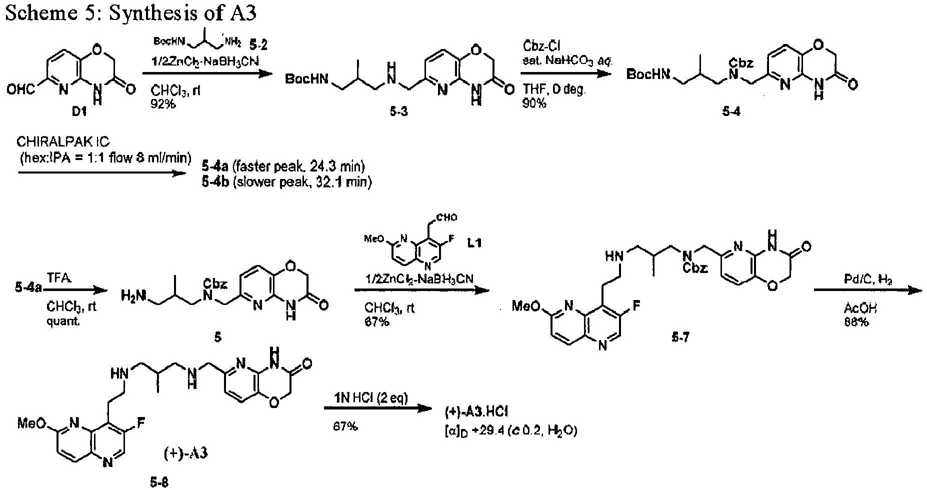

### tert-Butyl 2-methyl-3-((3-oxo-3,4-dihydro-2H-pyrido[3,2-b][1,4]oxazin-6-yl)methylamino) propylcarbamate (5-3)

A mixture of D1 (946 mg, 5.31 mmol) and 5-2 (1.00 g, 5.31 mmol) in CHCl₃ (53 mL) was stirred at room temperature for 1 hour. A solution of 1/2ZnCl₂-NaBH₃CN (0.3 M MeOH solution, 17.7 mL, 5.31 mmol) was added to the reaction mixture and the mixture was stirred at room temperature for 3.25 hours. After quenching the reaction mixture by addition of water, the mixture was extracted with CH₂Cl₂. The organic extracts were dried over NaSO₄, filtered, and then concentrated under reduced pressure. Flash chromatography (CH₂Cl₂/MeOH/NH₄OH = 10:1:0.01) of the residue on silica gel gave 5-3 (1.71 g, 92%). ¹H NMR (400 MHz, DMSO-*d*₆) δ 0.87 (d, *J =* 6.7 Hz, 3H), 1.35 (s, 9H), 1.80-1.95 (m, 1H), 2.54-2.64 (m, 1H), 2.66-2.76 (m, 1H), 2.81-2.96 (m, 2H), 3.93 (s, 2H), 4.65 (s, 2H), 6.93 (br, 1H), 7.09 (d, *J* = 7.9 Hz, 1H), 7.37 (d, *J* = 7.9 Hz, 1H), 11.3 (s, 1H).

### Benzyl (3-((tert-butoxycarbonyl)amino)-2-methylpropyl)((3-oxo-3,4-dihydro-2H-pyrido[3,2-b][1,4] oxazin-6-yl)methyl)carbamate (5-4a)

To a solution of 5-3 (1.70 g, 4.85 mmol) in THF (48.5 mL) was added saturated aqueous solution of NaHCO₃ (19.4 mL) followed by Cbz-Cl (0.73 mL, 5.09 mmol) at 0 °C. The mixture was stirred at the same temperature for 50 minutes. After addition of water, the mixture was extracted with EtOAc. The organic extracts were washed with brine, dried over NaSO₄ and concentrated under reduced pressure. Flash chromatography (hexane/EtOAc = 1:1) of the residue on silica gel gave 5-4 (2.19 g, 90%).Optical resolution of 5-4 (CHIRALPAK IC, hexane/IPA = 1:1, flow: 8 mL/min, 40 °C) gave 5-4a (1.04 g), and 5-4b (1.21 g). ¹H NMR (400 MHz, DMSO-*d*₆) δ 0.71-0.83 (m, 3H), 1.34 (s, 9H), 1.86-1.95 (m, 1H), 2.70 (br, 1H), 2.80-3.00 (br, 1H), 3.07-3.14 (br, 2H), 4.31-4.44 (m, 2H), 4.61 (s, 2H), 5.00-5.12 (m, 2H), 6.71-6.78 (m, 2H), 7.17-7.36 (m, 6H), 11.2 (s, 1H).

### Benzyl 3-amino-2-methylpropyl((3-oxo-3,4-dihydro-2H-pyrido[3,2-b][1,4]oxazin-6-yl)methyl) carbamate (5-5)

To a solution of 5-4a (200 mg, 0.413 mmol) in CH₂Cl₂ (0.41 mL) was added TFA (0.41 mL) at 0 °C. The mixture was stirred at room temperature for 20 minutes and concentrated under reduced pressure. Dichloromethane (15 mL) was added to the residue and washed with aqueous NaHCO₃. The resulting solution was dried over Na₂SO₄, filtered and concentrated under reduced pressure. Flash chromatography (CH₂Cl₂/MeOH/NH₄OH = 10:1:0.1) of the residue on silica gel gave 5-5 (165 mg, quant.). ¹H NMR (400 MHz, DMSO-*d*₆) δ 0.71-0.89 (m, 3H), 1.77-1.92 (br, 1H), 2.31-2.59 (m, 2H), 3.07-3.16 (m, 1H), 3.21-3.36 (m, 1H), 4.39 (s, 2H), 4.61 (s, 2H), 4.97-5.13 (m, 2H), 5.81 (br, 2H), 6.67-6.81 (m, 1H), 7.08-7.42 (m, 6H).

### Benzyl (3-((2-(3-fluoro-6-methoxy-1,5-naphthyridin-4-yl)ethyl)amino)propyl)((3-oxo-3,4-dihydro-2H-pyrido[3,2-b][1,4]oxazin-6-yl)methyl)carbamate (5-7)

To a solution of 5-5 (140 mg, 0.365 mmol) in CHCl₃ (3.7 mL) was added L1 (80.4 mg, 0.365 mmol), the mixture was stirred at room temperature for 5 minutes. A solution of 1/2ZnCl₂-NaBH₃CN (0.3 M MeOH solution, 1.2 mL, 0.365 mmol) was added to the mixture, and the resulting mixture was stirred at room temperature for 2.4 hours. After quenching the reaction mixture by addition of water, the mixture was extracted with CH₂Cl₂. The organic extracts were dried over NaSO₄, filtered, and then concentrated under reduced pressure. Flash chromatography (CH₂Cl₂/MeOH/NH₄OH = 10:1:0.01) of the residue on silica gel gave 5-7 (140 mg, 65%). ¹H NMR (400 MHz, CDCl₃, mixture of rotamers) δ 0.81-0.90 (m, 3H), 2.00-2.18 (m, 1H), 2.37-2.72 (m, 2H), 2.88-3.17 (m, 3H), 3.27-3.49 (m, 3H), 4.04, 4.07 (each s, total 3H), 4.24-4.36 (m, 1H), 4.39-4.52 (m, 1H), 4.62 (s, 2H), 5.00-5.17 (m, 2H), 6.64, 6.85 (each d, J = 7.9Hz, total 1H), 7.06 (d, J = 9.2Hz, 1H), 7.09-7.37 (m, 6H), 8.18 (d, *J =* 9.2 Hz, 1H), 8.61 (s, 1H).

### 6-(((3-((2-(3-Fluoro-6-methoxy-1,5-naphthyridin-4-yl)ethyl)amino)-2-methylpropyl)amino)methyl)-2H-pyrido[3,2-b][1,4]oxazin-3(4H)-one (5-8) and hydrochloride (A3)

A suspension of 5-7 (135 mg, 0.23 mmol) and 10% Pd/C (40.5 mg, 30 wt%) in AcOH (7.6 mL) was stirred at room temperature under H₂ atmosphere (1 kg/cm²) for 4.5 hours. Catalyst was removed by filtration and the filtrate was concentrated under reduced pressure. Flash chromatography (amino silica column, CH₂Cl₂/MeOH = 20:1) of the residue gave 5-8 (+)- A3 (46.5 mg, 45%). ¹H NMR (400 MHz, DMSO-*d*₆) δ0.80 (d, *J* = 6.7 Hz, 3H), 1.58-1.70 (m, 1H), 2.28 (dd, J = 11.6, 6.7Hz, 1H), 2.33-2.58 (m, 3H), 2.87 (t, J = 7.3Hz, 2H), 3.26 (t, J = 7.3Hz, 2H),-3.55 (s, 2H), 4.02 (s, 3H), 4.59 (s, 2H), 6.91 (d, *J* = 7.9 Hz, 1H), 7.21 (d, *J* = 9.2 Hz, 1H), 7.25 (d, *J =* 7.9 Hz, 1H), 8.25 (d, *J =* 9.2 Hz, 1H), 8.74(s, 1H).

To a solution of 5-8 (43.8 mg, 96.6 umol) in EtOH (0.48 mL) was added 1M HCl solution (96.6 microL, 96.6 micromol), the mixture was stirred at room temperature for 30 min and concentrated under reduced pressure. Trituration of the residue with EtOH and CH₂Cl₂ gave (+)-A3.HCl (31.6 mg, 67%). [α]_{D} +29.4 (*c* 0.2, H₂O). ¹H NMR (400 MHz, DMSO-*d*₆) δ 0.88 (brs, 3H), 2.07 (br, 1H), 2.49-2.76 (m, 2H), 2.81-3.06 (m, 2H), 3.15-3.58 (m, 4H), 3.64 (br, 2H), 4.05 (s, 3H), 4.63 (s, 2H), 6.94 (br, 1H), 7.23 (d, *J* = 9.2 Hz, 1H), 7.31 (d, *J* = 8.6 Hz, 1 H), 8.26 (d, *J =* 9.2 Hz, 1H), 8.77 (s, 1 H).

The enantiomer (-) A3 can be prepared starting from enantiomer 5-4b (slower peak) using scheme 5. The racemic A3 can be prepared likewise using 5-4 without chiral separation.

### Example 6 - Compound A4

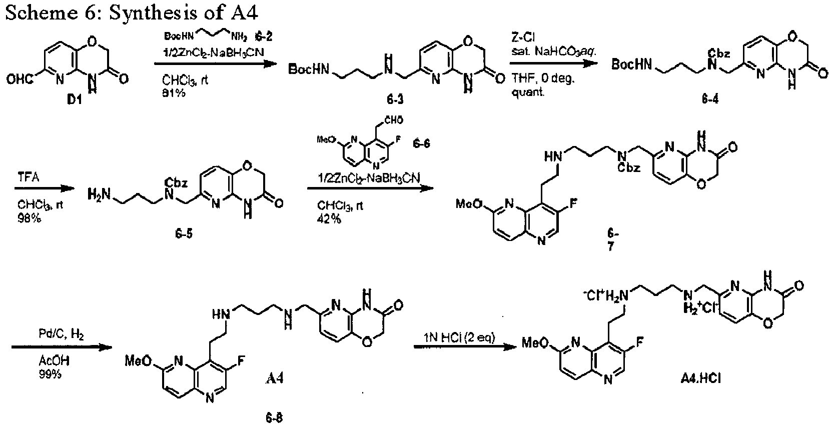

### tert-Butyl (3-(((3-oxo-3,4-dihydro-2H-pyrido[3,2-b][1,4]oxazin-6-yl)methyl)amino)propyl)carbamate (6-3)

A mixture of D1 (902 mg, 5.06 mmol) and 6-2 (926 mg, 5.31 mmol) in CHCl₃ (50.6 mL) was stirred at room temperature for 1 hour. 1/2ZnCl₂-NaBH₃CN (0.3 M MeOH solution, 16.9 mL, 5.06 mmol) was added to the reaction mixture and the mixture was stirred at room temperature for 1.5 hours. After quenching by addition of water (100 mL), the mixture was extracted with CH₂Cl₂. The organic extracts were dried over magnesium sulfate, filtered, and then concentrated under reduced pressure. Flash chromatography (amino column, CHCl₃/MeOH = 50:1) of the residue gave 6-3 (1.37 g, 81%). ¹H NMR (400 MHz, CDCl₃) δ 1.45 (s, 9H), 1.69 (quint, *J* = 6.1 Hz, 2H), 2.67-2.73 (m, 2H), 3.21-3.26 (m, 2H), 3.79 (s, 2H), 4.64 (s, 2H), 5.16 (brs, 1H), 6.92 (d, *J =* 8.0 Hz, 1H), 7.19 (d, *J =* 8.0 Hz, 1H).

### Benzyl (3-((tert-butoxycarbonyl)amino)propyl)((3-oxo-3,4-dihydro-2H-pyrido [3,2-b][1,4]oxazin-6-yl)methyl)carbamate (6-4)

To a solution of 6-3 (1.37 g, 4.07 mmol) in THF (40.7 mL) and saturated aqueous solution of NaHCO₃ (20.4 mL) was added Cbz-Cl (643 microL, 4.28 mmol) at 0 °C. The mixture was stirred at the same temperature for 2 hours. After addition of water to the mixture it was extracted with EtOAc. The organic extracts were washed with brine, dried over magnesium sulfate and concentrated under reduced pressure. Flash chromatography (CHCl₃/MeOH = 19:1) of the residue on silica gel gave 6-4 (1.94 g, quant). ¹H NMR (400 MHz, CD₃OD) δ 1.40-1.42 (m, 9H), 1.70-1.72 (m, 2H), 3.00-3.06 (m, 2H), 3.38-3.43 (m, 2H), 4.44 (s, 2H), 4.59 (s, 2H), 5.07-5.14 (m, 2H), 6.76 (d, *J =* 8.0 Hz, 0.6H), 6.88 (d, *J =* 8.0 Hz, 0.4H), 7.15-7.37 (m, 6H).

### Benzyl (3-aminopropyl)((3-oxo-3,4-dihydro-2H-pyrido[3,2-b][1,4]oxazin-6-yl)methyl)carbamate (6-5)

To a solution of 6-4 (1.94 g, 4.12 mmol) in CH₂Cl₂ (5 mL) was added TFA (5 mL) at 0 °C, and the mixture was stirred at room temperature for 1 hour. The mixture was concentrated under reduced pressure and chromatographed by flash chromatography (amino column, CHCl₃/MeOH =92/8) of the residue gave 6-5 (1.50 g, 98%). ¹H NMR (400 MHz, CDCl₃) δ1.74(br, 2H), 2.70-2.76 (m, 2H), 3.45(br, 2H), 4.42-4.46 (m, 2H), 4.63 (s, 2H), 5.11-5.18 (m, 2H), 6.72-6.74 (m, 0.6H), 6.92 (br, 0.4H), 7.11-7.38 (m, 6H).

### Benzyl (3-((2-(3-fluoro-6-methoxy-1,5-naphthyridin-4-yl)ethyl)amino)propyl) ((3-oxo-3,4-dihydro-2H-pyrido[3,2-b][1,4]oxazin-6-yl)methyl)carbamate (6-7)

To a solution of 6-5 (1.50 g, 4.05 mmol) in CHCl₃ (40 mL) was added L1 (892 mg, 4.05 mmol) and 1/2ZnCl₂-NaBH₃CN (0.3 M MeOH solution, 13.5 mL, 4.05 mmol), and the mixture was stirred at room temperature for 1 hour. After quenching the reaction mixture by addition of water (100 mL), the mixture was extracted with CH₂Cl₂. The organic extracts were dried over magnesium sulfate, filtered, and then concentrated under reduced pressure. Flash chromatography (amino column, CHCl₃/MeOH = 19:1 - 9:1) of the residue gave 6-7 (997 mg, 42%). ¹H NMR (400 MHz, CDCl₃) δ1.77 (br, 2H), 2.64-2.70 (m, 2H), 2.98-3.03 (m, 2H), 3.36-3.40 (m, 4H), 4.06 (d, *J =* 8.8 Hz, 3H), 4.38 (2H, *J* = 9.3 Hz, 2H), 4.62 (s, 2H), 5.08-5.13 (m, 2H), 6.69 (d, *J =* 8.0 Hz, 0.6H), 6.87 (d, *J* = 7.3 Hz, 0.4H), 7.06 (d, *J* = 9.2 Hz, 1H), 7.09-7.33 (m, 5H), 8.18 (d, *J =* 9.2 Hz, 1H), 8.61 (s, 1H).

### 6-(((3-((2-(3-Fluoro-6-methoxy-1,5-naphthyridin-4-yl)ethyl)amino)propyl)amino)methyl)-2H-pyrido[3,2-b][1,4]oxazin-3(4H)-one (6-8) (A4) and dihydrochloride (A4)

A suspension of 6-7 (995 mg, 1.73 mmol) and 10%Pd/C (299 mg, 30 wt%) in AcOH (17.3 mL) was stirred at room temperature under H₂ atmosphere (1 kg/cm²) for 4 hours. Catalyst was removed by filtration and the filtrate was concentrated under reduced pressure. Flash chromatography (amino column, CHCl₃/MeOH = 19:1 - 9:1) of the residue gave 6-8 (A4) (760 mg, 99%). ¹H NMR (400 MHz, CDCl₃) δ 1.70 (quint, *J* = 6.7 Hz, 2H), 2.65 (t, *J* = 6.7 Hz, 2H), 2.76 (t, *J* = 6.7 Hz, 2H), 3.05 (t, *J =* 6.7 Hz, 2H), 3.39 (t, *J* = 6.7 Hz, 2H), 3.71 (s, 2H), 4.07 (s, 3H), 4.63 (s, 2H), 6.86 (d, *J* = 7.9 Hz, 1H), 7.06 (d, *J =* 9.2 Hz, 1H), 7.16 (d, *J* = 7.9 Hz, 1H), 8.18 (d, *J* = 8.6 Hz, 1H), 8.62 (s, 1H).

To a solution of 6-8 (400 mg, 0.908 mmol) in EtOH (5.0 mL) was added 1M HCl solution (1.82 mL, 1.82 mmol), the mixture was stirred at room temperature for 10 min and concentrated under reduced pressure. Trituration of the residue with EtOH gave A4.HCl (385 mg, 83%). ¹H NMR (400 MHz, DMSO- *d*₆) δ 2.00-2.15 (m, 2H), 3.06-3.09 (m, 4H), 3.22-3.32 (m, 2H), 3.50-3.58 (m, 2H), 4.07 (s, 2H), 4.10 (s, 3H), 4.68 (s, 2H), 7.20 (d, *J* = 8.6 Hz, 1H), 7.27 (d, *J* = 9.2 Hz, 1H), 7.42 (d, *J* = 7.9 Hz, 1H), 8.30 (d, *J* = 9.2 Hz, 1H), 8.83 (s, 1H), 9.31 (br, 4H), 11.3 (br, 1H).

### Example 7 - Compound A5

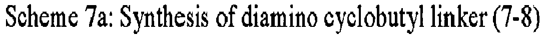

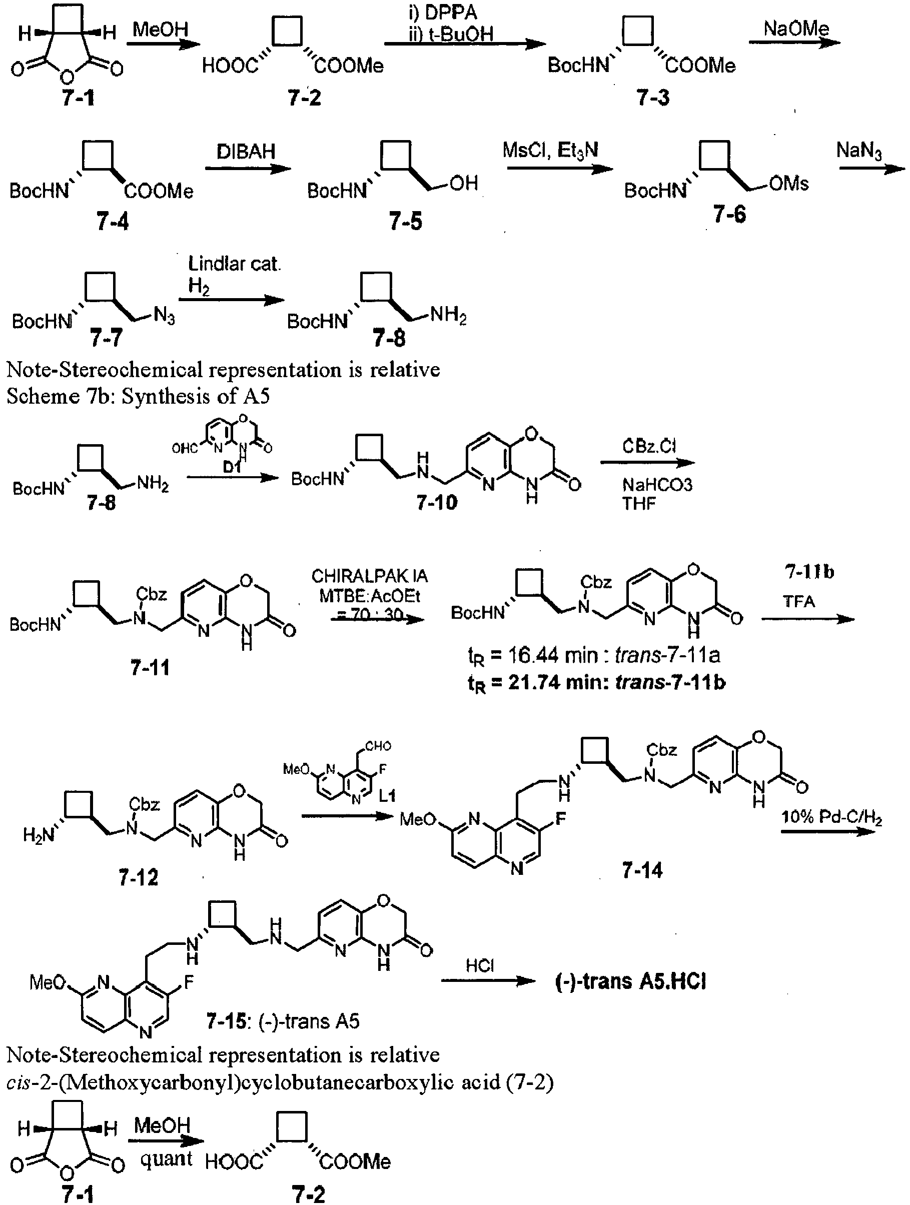

A solution of perhydrocyclobuta[c]furan-1,3-dione (7-1) (19.0 g, 146 mmol) in MeOH(95 mL) was heated under reflux for 4 hours and concentrated under reduced pressure to give 7-2 (24.1 g, quant.). This was used for the next step without further purification. (The product was solidified upon storage in the refrigerator.) ¹H NMR (CDCl₃) *δ*.21-2.26 (m, 2H), 2.36-2.45 (m, 2H), 3.42-3.46 (m, 2H), 3.69 (s, 3H), 8.44 (brs, 1H). MS (CI⁺) *m*/*z:* 159 (MH+). HRMS (CI⁺) for C₇H₁₁O₄ (MH+): calcd, 159.0657; found, 159.0636. IR (ATR) cm⁻¹: 2956, 1704, 1205, 1172.

### Methyl cis-2-((tert-butoxycarbonyl)amino)cyclobutanecarboxylate (7-3)

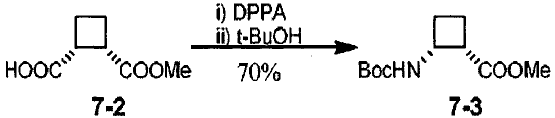

A mixture of the acid 7-2 (10.0 g, 63.2 mmol), Molecular Sieves (4A, 12.6 g) and triethylamine (9.69 mL, 69.6 mmol) in toluene (316 mL) was added DPPA (15.0 mL, 69.6 mmol), the mixture was stirred at room temperature for 3 hours. The mixture was heated under reflux for 1 hour. The insoluble materials were removed by filtration and the filtrate was concentrated under reduced pressure. The residue was diluted with *t*-BuOH (100 mL) and the mixture was heated under reflux for 6 hours. The mixture was cooled to room temperature, quenched with 10% citric acid solution (20 mL) and the mixture was concentrated under reduced pressure. The aqueous mixture was adjusted to pH 8-9 with saturated NaHCO₃ solution. The mixture was extracted with EtOAc. The organic extracts were dried over anhydrous magnesium sulfate, filtered, and concentrated under reduced pressure to dryness. Flash chromatography (hexane/EtOAc = 75:25) of the residue on silica gel gave 7-3 (10.19 g, 70% yield). ¹H NMR (CDCl₃) *δ* 1.43 (s, 9H), 1.93-2.00 (m, 2H), 2.22 (quint. J = 9.8 Hz, 1H), 2.33-2.39 (m, 1H), 3.39 (br, 1H), 3.71 (s, 3H), 4.42-4.50 (m, 1H), 5.34 (br, 1H). MS (CI⁺) *m*/*z:* 230 (MH⁺). HRMS (CI⁺) for C11H20NO4 (MH+): calcd, 230.1392; found, 230.1356. IR (ATR) cm⁻¹: 3334, 2957,1728, 1684, 1521, 1164.

### Methyl trans-2-((tert-butoxycarbonyl)amino)cyclobutanecarboxylate (7-4)

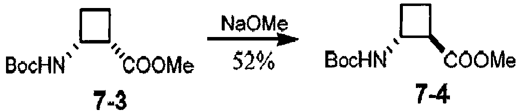

To a solution of NaOMe (3.53 g, 65.4 mmol) in MeOH (200 mL) was added a solution of 7-3 (3.00 g, 13.1 mmol) in MeOH (25 mL), the mixture was heated under reflux for 2 hours. The mixture was adjusted to pH 5 with 1 N HCl and then concentrated under reduced pressure. The aqueous mixture was extracted with EtOAc. The organic extracts were dried over anhydrous magnesium sulfate, filtered, and volatile material was removed by concentration under reduced pressure. Flash chromatography (hexane/EtOAc = 1:1) of the residue on silica gel gave 7-4 (1.59 g, 52% yield). ¹H NMR (CDCl₃) *δ* 1.43 (s, 9H),1.88-1.98 (m, 3H), 2.21-2.26 (m, 1H), 2.99 (brs, 1H), 3.69 (s, 3H), 4.22 (brs, 1H), 4.81 (brs, 1H). MS (CI+) m/z: 230 (MH+). HRMS (CI+) for C₁₁H₂₀NO₄ (MH+): calcd, 230.1392; found, 230.1408. IR (ATR) cm⁻¹: 3372, 2983, 1727, 1684, 1519, 1167.

### tert-Butyl (trans-2-(hydroxymethyl)cyclobutyl)carbamate (7-5)

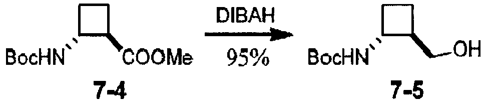

To a solution of DIBAH (1.04M in n-hexane, 22.1 mL, 23.0 mmol) was added a solution of 7-4 (1.10 g, 4.80 mmol) in toluene (6.34 mL) at -78 °C over 15 minutes. The reaction mixture was stirred at the same temperature for 30 minutes and quenched by addition of 10% citric acid solution at -78 °C. The resulting precipitates was removed by filtration using a celite pad and washed with CH₂Cl₂. The combined filtrates were dried over anhydrous magnesium sulfate, filtered, and then concentrated under reduced pressure. Flash chromatography (hexane/EtOAc = 2:1) of the residue over silica gel gave 7-5 (915 mg, 95% yield). ¹H NMR (400 MHz, CDCl₃) *δ* 1.31-1.41 (m, 1H), 1.43 (s, 9H), 1.67-1.85 (m, 2H), 2.18-2.24 (m, 1H), 2.27-2.37 (m, 1H), 3.47-3.65 (m, 3H), 4.03 (brd, J = 9.8 Hz, 1H), 4.86 (brs, 1H).

### (trans-2-((tert-Butoxycarbonyl)amino)cyclobutyl)methyl methanesulfonate (7-6)

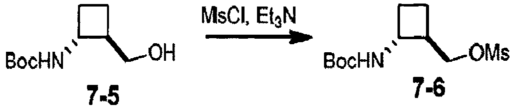

To a solution of 7-5 (600 mg, 2.98 mmol) and triethylamine (623 microL, 4.47 mmol) in THF (20 mL) was added MsCl (277 microL, 3.58 mmol) under cooling in an ice-bath. The mixture was stirred at the same temperature for 1 hours. Water was added to the mixture and the mixture was extracted with EtOAc. The organic extracts were dried over anhydrous magnesium sulfate, filtered, and then solvent was removed under reduced pressure to give 7-5. This was used for the next step without further purification. ¹H NMR (400 MHz, CDCl₃) *δ* 1.43 (s, 9H), 1.56 (quint, J = 9.8 Hz, 1H), 1.76 (quint, J = 9.8 Hz, 1H), 1.84-1.91 (m, 1H), 2.22-2.29 (m, 1H),2.45-2.54 (m, 1H), 3.04 (s, 3H),3.91 (mbrs, 1H), 4.22-4.30 (m, 2H), 4.71 (brs, 1H).

### tert-Butyl (trans-2-(azidomethyl)cyclobutyl)carbamate 7-7

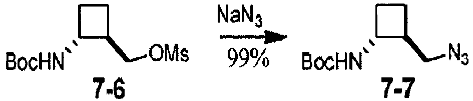

To a solution of crude 7-6 in DMF (29.8 mL) was added and NaN₃ (388 mg, 5.96 mmol) and heated while stirring at 70 °C for 45 minutes. After addition of water the mixture was extracted with EtOAc. The combined extracts were diluted with hexane and the mixture washed with water and brine. The organic extracts were dried over anhydrous magnesium sulfate, filtered, and then concentrated under reduced pressure to dryness. Flash chromatography (hexane/EtOAc = 75:25) of the residue on silica gel gave 7-7 (666 mg, 99% yield). ¹H NMR (400 MHz, CDCl₃) *δ* 1.41-1.50 (m, 1H), 1.43 (s, 9H), 1.72 (quint, J = 9.8 Hz, 1H), 1.89 (q, J = 9.2 Hz, 1H), 2.22-2.29 (m, 1H), 2.32-2.37 (m, 1H), 3.34 (dd, J = 12.2, 7.3 Hz, 1H), 3.44 (dd, J = 12.8, 5.5 Hz, 1H), 3.81-3.89 (m, 1H), 4.69 (brs, 1H). MS (CI⁺) *m*/*z:* 227 (MH⁺). HRMS (CI⁺) for C₁₀H₁₉N₄O₂ (MH⁺): calcd, 227.1508; found, 227.1525. IR (ATR) cm⁻¹: 3341, 2978, 2870, 2091, 1684, 1513, 1161

### tert-Butyl (trans-2-(aminomethyl)cyclobutyl)carbamate (7-8)

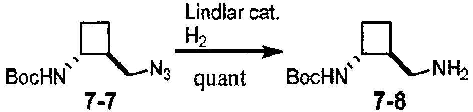

A suspension of 7-7 (666 mg, 2.94 mmol) and Lindlar cat. (233 mg, 35 wt%) in methanol (29.4 mL) was stirred at room temperature under H₂ atmosphere (1 kg/cm²) for 3.3 hours. After removal of the insoluble materials by filtration, the filtrate was concentrated under reduced pressure to give amine 7-8 (637 mg, quant.). This was used for the next step without further purification. ¹H NMR (400 MHz, CDCl₃) *δ* 1.20-1.35 (m, 3H), 1.43 (s, 9H), 1.66 (quint, J = 9.8 Hz, 1H), 1.80 (q, J = 9.8 Hz, 1H), 2.08-2.16 (m, 1H), 2.19-2.26 (m, 1H), 2.70-2.80 (m, 2H), 3.70-3.77 (m, 1H), 4.77 (brs, 1H). MS (CI⁺) m/z: 201 (MH⁺). HRMS (CI⁺) for C₁₀H₂₁N₂O₂ (MH⁺): calcd, 201.1603; found, 201.1627. IR (ATR) cm⁻¹: 3320, 2977, 2857, 1678, 1526, 1496, 1274, 1162.

### tert-Butyl trans-2-(((3-oxo-3,4-dihydro-2H-pyrido[3,2-b][1,4]oxazin-6-yl)methylamino)methyl)cyclobutylcarbamate (7-10)

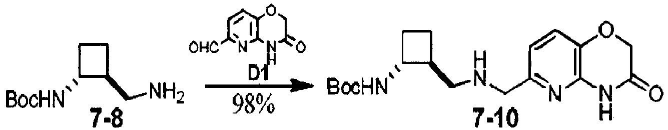

To a solution of 7-8 (450 mg, 2.25 mmol) in CHCl₃ (30.0 mL) and MeOH (5.0 mL) was added D1 (400 mg, 2.25 mmol), and the mixture was stirred at room temperature for 30 min followed by addition of solution of 1/2 ZnCl₂-NaBH₃CN (0.3M in MeOH) (7.49 mL, 2.25 mmol) and stirring the mixture for 30 min. The reaction mixture was quenched with water and extracted with CH₂Cl₂. The organic extracts were dried over magnesium sulfate, filtered, and concentrated under reduced pressure. The residue was purified by silica gel column chromatography on silica-gel (CHCl₃/MeOH=90/10) to afford 7-10 as colorless oil (796 mg, 98%). ¹H NMR (400 MHz, CD₃OD) *δ* 1.28-1.35 (m, 1H), 1.39 (s, 9H), 1.76-1.85 (m, 2H), 2.10-2.15 (m, 1H), 2.32-2.34 (m, 1H), 2.56-2.65 (m, 2H), 3.62-3.73 (m, 3H), 4.62 (s, 2H), 6.95 (d, 1H), 7.24 (d, 1H), 7.89 (s, 1H).

### tert-Butyl trans-2-(((benzyloxycarbonyl)(3-oxo-3,4-dihydro-2H-pyrido[3,2-b][1,4]oxazin-6-yl)methylamino)methyl)cyclobutylcarbamate (7-11)

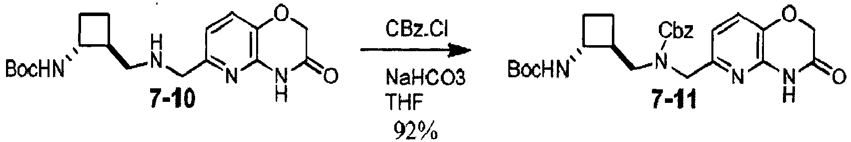

To a solution of 7-10 (796 mg, 2.20 mmol) in THF (22.0 mL) was added saturated NaHCO₃ solution (11.0 mL) followed by Cbz-Cl (363 microL, 2.42 mmol) at 0 °C, the mixture was stirred at room temperature for 3 h. The mixture was diluted with water and extracted with EtOAc. The organic extracts were washed with brine, dried over magnesium sulfate, filtered, and concentrated under vacuo. The residue was purified by flash chromatography on silica gel (hexane/EtOAc=1/2) to afford 7-11 (1.00 g, 92%). This mixture was separated by chiral high performance liquid chromatography.

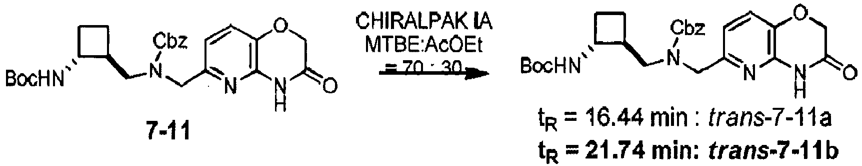

### Optical resolution of 7-11 (analytical conditions)

Column: CHRALPAK IA, Solvent: MTBE/EtOAc = 70/30, Flow: 0.5 mL/min.
trans-7-lla: t_{R} = 16.44 min.
trans-7-11b: t_{R} = 21.74 min.
Note-Stereochemical representation is relative

### Benzyl (trans-2-aminocyclobutyl)methyl((3-oxo-3,4-dihydro-2H-pyrido[3,2-b][1,4]oxazin-6-yl)methyl)carbamate (7-12)

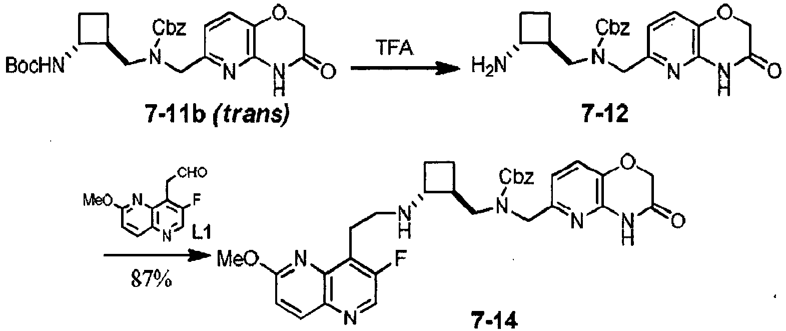

### Note-Stereochemical representation is relative

To a solution of *trans*-7-11b (slower enantiomer, 397 mg, 0.80 mmol) in CHCl₃ (2.5 mL) was added TFA (2.5 mL) at room temperature, and the mixture was stirred at room temperature for 10 min. The reaction mixture was concentrated under reduced pressure to give a residue which was purified by column chromatography (NH-silica, CHCl₃/MeOH=90/10) to afford intermediate amine 7-12 as colorless foam (319 mg). The amine was dissolved in CHCl₃ (8.0 mL), then L1 (176 mg, 0.80 mmol) and 1/2 ZnCl₂-NaBH₃CN (0.3M in MeOH) (2.67 mL, 0.80 mmol) were added, and the mixture was stirred at room temperature for 1 hour. The reaction mixture was quenched with water and extracted with CH₂Cl₂. The organic extracts were dried over magnesium sulfate, filtered and concentrated under reduced pressure to give a residue which was purified by column chromatography on silica gel (CHCl₃/MeOH=19/1) to afford 7-14 as pale yellow foam (417 mg, 87%).

### Synthesis of 7-15 and A5

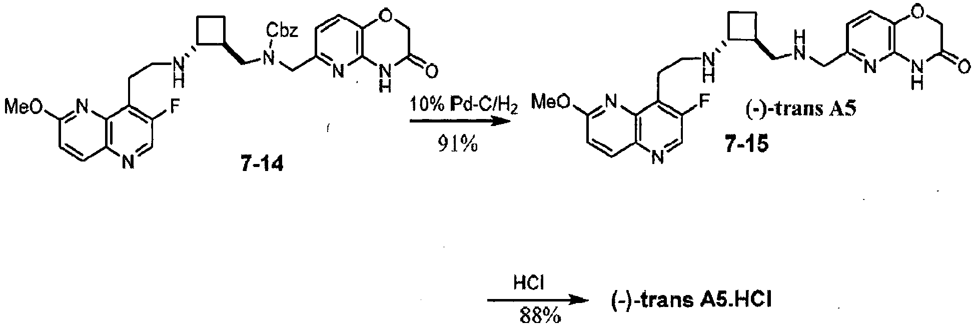

Note-Stereochemical representation is relative

A suspension of 7-14 (400 mg, 0.666 mmol) and Pd/C (80.0 mg, 20wt%) in AcOH (6.7 mL) was hydrogenated at room temperature for 4 hours. The insoluble catalyst was filtered through a pad of Celite and washed with EtOH. The filtrate was concentrated under reduced pressure. The residue was purified by column chromatography (NH-silica, CHCl₃/MeOH=30/1) to afford 7-15 (-)-trans A5 as pale yellow foam (283 mg, 91%). [α]_{D} -35.5 (c 0.83, CHCl₃)

To a solution of 7-15 (150 mg, 0.322 mmol) in EtOH (3.2 mL) was added 1N HCl solution (643 uL, 0.643 mmol), the mixture was stirred at room temperature for 10 min and concentrated in vacuo. The residue was triturated of the residue with EtOH-Et₂O (1/3) gave (-)-trans A5.HCl as light brown solid (153 mg, 88%). ¹H NMR (400 MHz, DMSO-*d*₆): *δ* 1.59-1.69 (m, 1H), 2.00-2.17 (m, 4H), 2.97-3.21 (m, 4H), 3.53 (brs, 2H), 3.65 (br, 1H), 4.08 (br, 2H), 4.10 (s, 3H), 4.68 (s, 2H), 7.23 (d, J = 7.9Hz, 1H), 7.27 (d, J = 9.2Hz, 1H), 7.43 (d, J = 8.6Hz, 1H), 8.31 (d, J = 9.2Hz, 1H), 8.83 (s, 1H), 9.27 (br, 1H), 9.46 (br, 1H), 9.97 (br, 1H), 11.3 (s, 1H). MS (ESI⁺) m/z: 467 (MH⁺). IR (ATR) cm⁻¹: 2946, 2724, 1709, 1615, 1489, 1273.

(+)-trans A5 can be similarly synthesized from the trans-7-11a following scheme 7b. Likewise both cis-enantiomers can be prepared directly using 7-3 following schemes 7a and 7b eliminating the racemization step (reaction of NaOMe) followed by separation of the cis-7-11 intermediate.

### Example 8 - Compound A7

The synthetic procedure of A7 is presented in following three schemes.

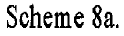

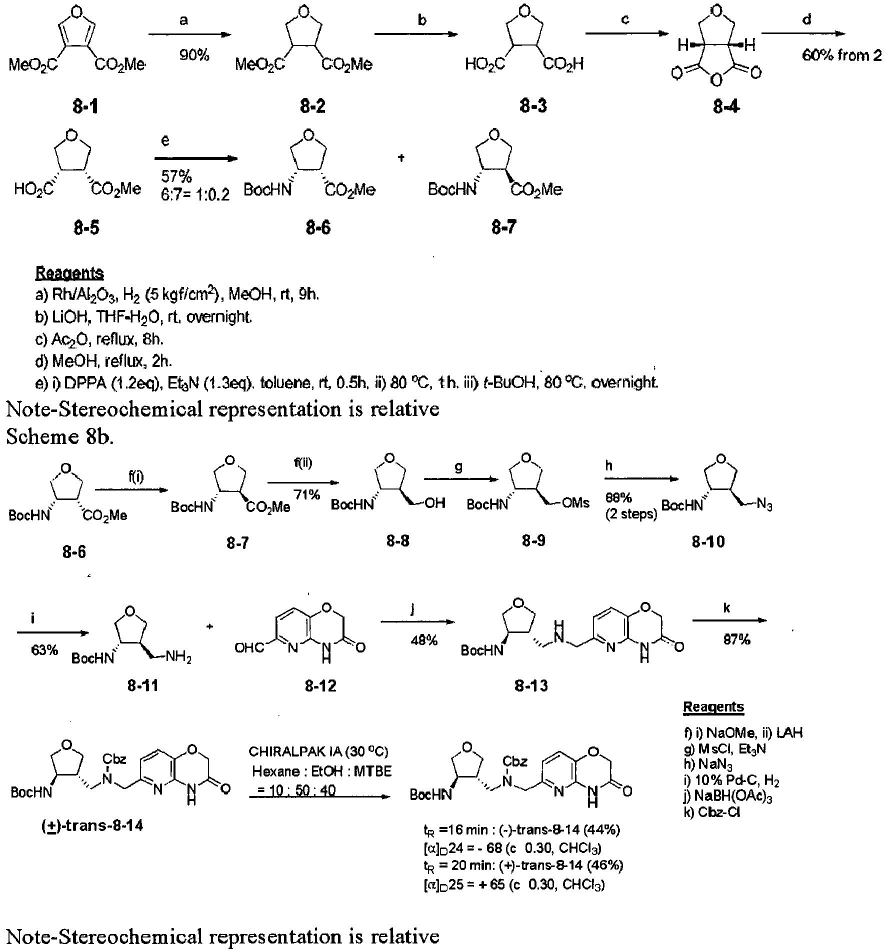

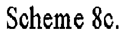

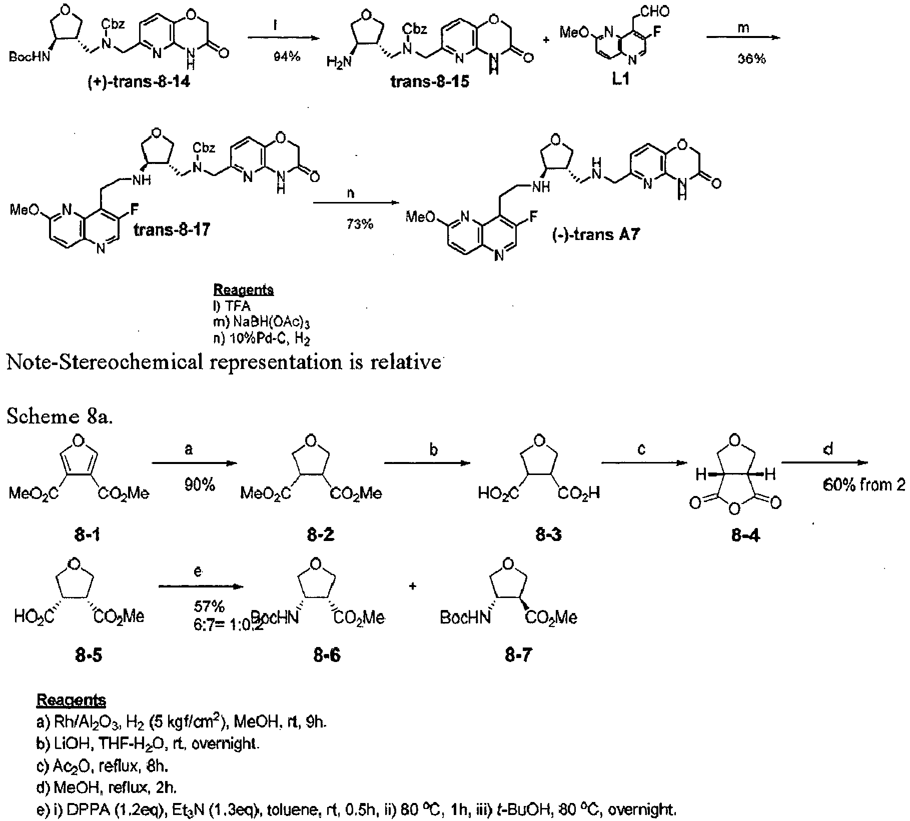

### Dimethyl tetrahydrofuran-3,4-dicarboxylate (8-2)

A suspension of 8-1 (5.00 g, 27.1 mmol) and Rh/Al₂O₃ catalyst (1.0 g) in methanol (25 mL) was stirred at room temperature for 4 hour under 4.7 kg/cm²H₂ atmosphere. After reaction was complete insoluble materials were filtered off and the filtrate was concentrated under reduced pressure. Flash chromatography (hexanes/EtOAc = 2:1) of the residue on silica gel gave cis-8-2 (more polar, 4.62 g, 90%) and trans-8-2 (less polar, 500 mg, 10%).

### Tetrahydrofuran-3,4-dicarboxylic acid (8-3)

A mixture of 8-2 (20.4 g, 108 mmol) in concentrated HCl (108 mL) and water (108 mL) was heated under reflux for 54 hours, and then concentrated under reduced pressure to give crude 8-3. This was used for the next step without further purification. ¹H NMR (400 MHz, DMSO*d*₆): *δ* 3.23-3.26 (m, 2H), 3.84-3.94 (m, 4H), 12.38 (s, 2H).

### Tetrahydrofuro-[3,4-c]furan-1,3-dione (8-4)

A mixture of the crude product 8-3 and Ac₂O (130 mL) was heated at 145 °C for 8 hours, and then concentrated under vacuo to give crude compound 8-4. This was used for next step without further purification ¹H NMR (400 MHz, DMSO*d*₆): *δ* 3.68-3.75 (m, 2H), 3.77-3.82 (m, 2H), 4.20 (d, *J* = 9.2 Hz, 2H).

### 4-(Methoxycarbonyl)tetrahydrofuran-3-carboxylic acid (8-5)

A mixture of the unpurified 8-4 in MeOH (150 mL) was heated under reflux for 2 hours, and then concentrated under reduced pressure. Flash chromatography (hexanes/EtOAc/AcOH = 1:1:0.1) of the residue gave 8-5 (11.3 g, 60% for 3 steps). ¹H NMR (400 MHz, CDCl₃): *δ* 3.35-3.42 (m, 2H), 3.71 (s, 3H), 4.09-4.17 (m, 4H), 5.85 (br, 1H).

### Methyl 4-(tert-butoxycarbonylamino)tetrahydrofuran-3-carboxylate (8-6 and 8-7)

To a solution of 8-5 (1.90 g, 10.9 mmol) in toluene (54.5 mL) was added Et₃N (1.67 mL) and DPPA (2.59 mL) at room temperature, the mixture was stirred at the same temperature for 2 hours. The resulting mixture was heated under reflux for 3 hours and then concentrated under reduced pressure. The reaction mixture was diluted with *t*-BuOH (18.1 mL) and heated under reflux for 18 hours and then concentrated under vacuo. Flash chromatography (hexanes/EtOAc = 2:1) of the residue gave isomeric compounds 8-6 and 8-7 (1.42 g, 53%). Compounds 8-6 and 8-7 were separated by careful flash chromatography but separation was not needed at this step, because 8-6 was epimerized to 8-7. In addition, cis- and trans- alcohol 8-8 of the next step were separated more easily. 8-6: ¹H NMR (400 MHz, CDCl₃): *δ* 1.43 (s, 9H), 3.31(dd, J = 14.7, 7.3 Hz, 1H), 3.67 (dd, *J =* 4.9, 9.2 Hz, 1H), 3.72 (s, 3H), 3.94 (dd, *J* = 9.2, 5.5 Hz, 1H), 4.02 (t, *J* = 8.6 Hz, 1H), 4.10 (dd, *J* = 7.3, 9.2 Hz, 1H), 4.58 (br, 1H), 5.06 (br, 1H). 8-7: ¹H NMR (400 MHz, CDCl₃): *δ* 1.44 (s, 9H), 2.99 (br, 1H), 3.67 (m, 1H), 3.73 (s, 3H), 3.93 (dd,*J=* 6.1, 9.1 Hz, 1H), 3.98 (dd, *J* = 6.1, 9.7 Hz, 1H), 4.14 (t, *J* = 9.1 Hz, 1H), 4.47 (br, 1H), 4.80 (br, 1H).

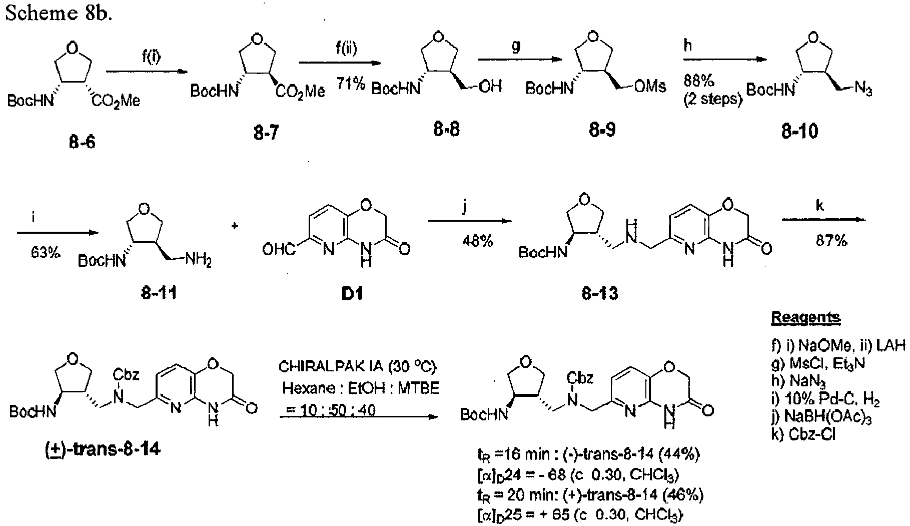

### Epimerization of 8-6 to 8-7

To a solution of NaOMe in MeOH [prepared from Na (465.0 mg) and MeOH (224 mL)] was added a solution of the *cis*-rich mixture of 8-6 and 8-7 (1.10 g, 4.48 mmol) in MeOH (10 mL) at room temperature. The mixture was stirred at the same temperature for 4 hours. The reaction mixture was cooled in ice bath was acidified (pH 3.0) by slow addition of 1M HCl and then concentrated under reduced pressure. The reaction mixture was diluted with EtOAc and washed with brine. The organic extracts were dried over anhydrous sodium sulfate, filtered, and then concentrated under reduced pressure. Flash chromatography (chloroform/methanol = 20:1) of the residue gave the *trans*-rich mixture of 8-6 and 8-7 (1.095 g, 99.5%, 8-6:8-7=0.23:1 by NMR).

### tert-Butyl trans-4-(hydroxymethyl)tetrahydrofuran-3-ylcarbamate (8-8)

To a solution of LiAlH₄ (8.15 mL, 1M solution in Et₂O) in Et₂O (6 mL) was added a solution of the *trans*-rich mixture of 8-6 and 8-7 (1.00 g, 4.08 mmol) in Et₂O (2 mL) at -78 °C. The reaction mixture was stirred at the same temperature for 4 hours. After carefully quenching the reaction mixture by addition of a solution of THF-H₂O (0.7 mL+0.7 mL), 5 N sodium hydroxide (0.3 mL) was added and stirred at room temperature for 1 hour. The resulting precipitates were filtered and thoroughly washed with CH₂Cl₂/MeOH (10:1). The combined filtrate was dried over anhydrous sodium sulfate, filtered, and then concentrated to dryness under reduced pressure. Flash chromatography (chloroform/methanol = 20:1) of the residue over silica gel gave *trans*-8-8 (633 mg, 71%) and *cis-8-8* (102 mg, 11%).- ¹H NMR (400 MHz, CDCl₃) *δ* 1.45 (s, 9H), 2.24-2.30 (m, 1H), 3.32 (br, 1H), 3.47 (t, *J* = 7.3 Hz, 1H), 3.53-3.67 (m, 3H), 3.98-4.07 (m, 3H), 4.79 (br, 1H).

### trans-4-(tert-Butoxycarbonylamino)tetrahydrofuran-3-yl)methyl methanesulfonate (8-9)

To a solution of *trans*-8-8 (600 mg, 2.76 mmol) and Et₃N (462 microL, 3.31 mmol) in THF (13.8 mL) was added MsCl (235 microL, 3.04 mmol) under ice bath cooling. The reaction mixture was stirred at the same temperature for 2 hours. After quenching the reaction by addition of ice water, the mixture was concentrated to remove organic solvent and the mixture was extracted with EtOAc. The organic extracts were dried over anhydrous sodium sulfate, filtered, and concentrated under reduced pressure to give crude 8-9. This material was used without further purification. (Mesylate 8-9 was crystallized from Et₂O.)

### tert-Butyl trans-4-(azidomethyl)tetrahydrofuran-3-ylcarbamate (8-10)

To a solution of the crude 8-9 in DMF (13.8 mL) was added NaN₃ (539 mg, 8.29 mmol) in DMF and stirred at 70 °C for 17.5 hours. Water (10 mL) was added and the reaction mixture was extracted with Et₂O. The organic extracts were dried over anhydrous sodium sulfate, filtered, and then concentrated to dryness under reduced pressure. Flash chromatography (chloroform/methanol = 20:1) of the residue on silica gel gave azide 8-10 (587 mg, 88% in 2 steps). ¹H NMR (400 MHz, CDCl₃) δ 1.45 (s, 9H), 2.28 (br, 1H), 3.38 (dd, *J* = 12.2, 7.9 Hz, 1H), 3.49-3.61 (m, 3H), 3.98-4.01 (m, 3H), 4.71 (br, 1H).

### tert-Butyl trans-4-(((3-oxo-3,4-dihydro-2H-pyrido[3,2-b][1,4]oxazin-6-yl)methylamino)methyl)tetrahydrofuran-3-ylcarbamate (8-13)

To a solution of 8-10 (570 mg, 2.35 mmol) in MeOH (12 mL) was added Lindlar cat. (171 mg, 30 wt%) and the inhomogeneous mixture was stirred at room temperature under H₂ atmosphere (1 kg/cm²). After completion of the reaction the catalyst was filtered off and the filtrate was concentrated to dryness in vacuo to give amine 8-11.

To a solution of the amine 8-11 in DMF (12 mL) was added pyridoxazine aldehyde D1 (419 mg, 2.35 mmol) and AcOH (2.7 mL, 47.1 mmol). The mixture was stirred at room temperature for 30 min and NaBH(OAc)₃ (249 mg, 1.17 mmol) was added to the mixture, and the mixture was stirred at room temperature 30 min. Then additional NaBH(OAc)₃ (249 mg, 1.17 mmol) was added and stirred for additional 30min, followed by addition of additional NaBH(OAc)₃ (199 mg, 0.939 mmol) and stirring of the mixture for 14 hours. The reaction mixture was concentrated under reduced pressure and diluted with CH₂Cl₂. The mixture was washed with water and saturated Na₂CO₃ solution. The organic extracts were dried over Na₂SO₄, filtered and concentrated in vacuo. Flash chromatography (CHCl₃/MeOH = 15:1-10:1) of the residue on silica gel gave 8-13 (550 mg, 62%). ¹H NMR (400 MHz, CDCl₃) *δ* 1.43 (s, 9H), 2.21 (br, 1H), 2.58-2.80 (m, 2H), 3.45 *(dd, J =* 9.2, 6.7 Hz, 1 H), 3.54 (*dd, J =* 9.2, 4.9 Hz, 1 H), 3.70-3.85 (m, 2H), 3.90-3.95 (m, 1H), 3.95-4.09 (m, 2H), 4.62 (s, 2H), 4.82-4.92 (m, 1H), 6.80-6.90 (m, 1H), 7.17 (d, *J* =7.9 Hz, 1H).

### tert-Butyl trans-4-((benzyloxycarbonyl)((3-oxo-3,4-dihydro-2H-pyrido[3,2-b][1,4]oxazin-6-yl)methylamino)methyl) -tetrahydrofuran-3-ylcarbamate (8-14)

To a solution of 8-13 (545 mg, 1.44 mmol) in THF (6.8 mL) and water (6.8 mL) was added NaHCO₃ (580 mg, 6.91 mmol) and Cbz-Cl (247 microL, 1.73 mmol) at room temperature. The mixture was stirred at the room temperature for 3 hours. After addition of water, the mixture was extracted with EtOAc. The organic extracts were washed with brine, dried over Na₂SO₄ and concentrated under vacuum. Flash chromatography (hexane/EtOAc = 1:2) of the residue over silica gel gave 8-14 (643 mg, 87%). ¹H NMR (400 MHz, DMSO-*d*₆) *δ* 1.36 (s, 9H), 2.40-2.49 (m, 1H), 3.31-3.44 (m, 4H), 3.70-3.90 (m, 3H), 4.38 (d, *J* = 16 5 Hz, 1H), 4.45 (d, *J* = 15.9 Hz, 1H), 4.59 (s, 2H), 5.07 (s, 2H), 6.75 (d,J= 8.6 Hz, 1H), 6.88 (br, 1H), 7.22-7.36 (m, 6H), 11.0 (s, 1H).

### Optical resolution of 8-14 (analytical conditions)

Column: CHRALPAK IA, Solvent: Hexane/EtOH/MTBE = 10/50/40, Flow: 0.4 mL/min.
(-)-*trans*-8-14: t_{R} = 16.76 min, [α]_{D}²⁴ -68.8 (c 0.30, CHCl₃).
(+)-*trans*-8-14: t_{R} = 20.87 min, [α]_{D}²⁵ 65.6 (c 0.30, CHCl₃).

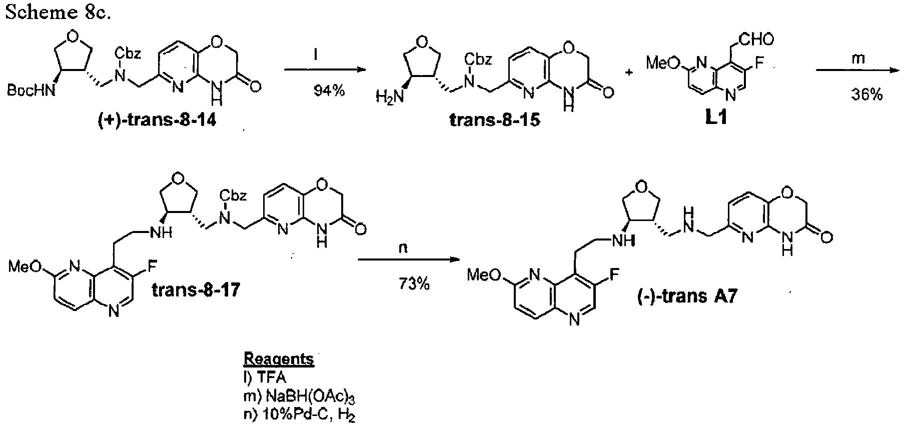

### Benzyl (trans-4-aminotetrahydrofuran-3-yl)methyl((3-oxo-3,4-dihydro-2H-pyrido[3,2-b][1,4]oxazin-6-yl) methyl)carbamate (trans-8-15).

To a solution of (+)-*trans*-8-14 (270 mg, 0.527 mmol) in CH₂Cl₂ (1 mL) was added TFA (1 mL) at 0 °C, and the mixture was stirred at room temperature for 1 hour. CH₂Cl₂ was added to the reaction mixture and 5N NaOH solution was added at 0 °C to adjust pH 11. The resulting mixture was extracted with CHCl₃-MeOH (20/1). The organic extracts were washed with brine, dried over Na₂SO₄ and concentrated to dryness under reduced pressure to afford *trans*-8-15 (220 mg, quant.). This was used for the next reaction without further purification. ¹H NMR (400 MHz, DMSO-*d*₆) *δ* 2.09-2.20 (m, 1H), 3.08-3.31 (m, 3H), 3.39-3.44 (m, 2H), 3.78-3.82 (m, 2H), 4.42 (s, 2H), 4.58 (s, 2H), 5.08 (s, 2H), 6.76 (d, *J* = 7.9 Hz, 1H), 7.24 (d, *J* = 7.9 Hz, 1H), 7.23-7.35 (m, 5H).

Benzyl ((3R,4R)-4-(2-(3-fluoro-6-methoxy-1,5-naphthyridin-4-yl)ethylamino)tetrahydrofuran-3-yl)methyl ((3-oxo-3,4-dihydro-2H-pyrido[3,2-b][1,4]oxazin-6-yl)methyl)carbamate (*trans*-8-17).

To a solution of the crude *trans*-8-15 (213 mg, 0.516 mmol) in DMF (4.1 mL) was added L1 (114 mg, 0.516 mmol) and AcOH (590 microL, 10.3 mmol), and the mixture was stirred at room temperature for 1 hour. NaBH(OAc)₃ (109 mg, 0.516 mmol) was added to the reaction mixture and the resulting mixture was stirred at room temperature, and then concentrated under reduced pressure. The reaction mixture was diluted with EtOAc and washed with saturated Na₂CO₃ solution. The organic extracts were dried over Na₂SO₄, filtered and concentrated under reduced pressure. Flash chromatography (CHCl₃/MeOH = 20:1) of the residue gave *trans*-8-17 (116 mg, 36%). ¹H NMR (400 MHz, DMSO-*d*₆) *δ* 2.22-2.30 (m, 1H), 2.75-2.84 (m, 2H), 2.89-3.06 (m, 1H), 3.20-3.37 (m, 6H), 3.66-3.84 (m, 2H), 4.01 (s, 3H), 4.33 (d, *J*= 15.9 Hz, 1H), 4.40 (d, *J=* 15.9 Hz, 1H), 4.58 (s, 2H), 5.02 (s, 2H), 6.68-6.75 (m, 1H), 7.20 (d, *J* = 9.2Hz, 1H), 7.19-7.30 (m, 6H), 8.25 (d, *J* = 9.2 Hz, 1H), 8.72 (s, 1H).

### Synthesis of A7

A suspension of *trans-8-17* (845 mg, 1.37 mmol) and 10% Pd/C (254 mg, 30 wt%) in AcOH (13.7 mL) was stirred at room temperature under H₂ atmosphere (1 kg/cm²) for 4 hours. Catalyst was removed by filtration and the filtrate was concentrated under reduced pressure. The residue was adjusted to pH 11 with 2N NaOH solution. The mixture was extracted with CHCl₃-MeOH (20/1). The organic extracts were dried over Na₂SO₄, filtered and concentrated under reduced pressure. Flash chromatography (CHCl₃/MeOH = 10:1) of the residue on silica gel gave (-)-trans A7 (538 mg, 81%). ¹H NMR (400 MHz, CDCl₃) *δ* 2.05-2.16 (m, 1H), 2.56-2.66 (m, 2H), 2.95-3.09 (m, 2H), 3.14 (dd, J = 11.0, 4.9 Hz, 1H), 3.32-3.42 (m, 2H), 3.48-3.55 (m, 2H), 3.70 (s, 2H), 3.93 (dd, *J* = 9.2, 6.1 Hz, 1 H), 3.99 (dd, J = 8.6, 7.3 Hz, 1H), 4.07 (s, 3H), 4.64 (s, 2H), 6.84 (d*, J* = 7.9 Hz, 1H), 7.08 *(d, J* = 9.2 Hz, 1H), 7.16 *(d, J =* 7.9 Hz, 1H), 8.19 *(d, J =* 9.2 Hz, 1H), 8.62 (s, 1H).[α]_{D}²⁵ -22.5 (c 0.30, CHCl₃).

(+)-trans A7 can be synthesized from (-)-trans 8-14 following the synthesis of (-)-A7. Similarly both cis-enantiomers can be prepared from 8-6 without racemization with NaOMe following rest of the reactions of schemes 8b and 8c including chiral separation.

### Example 9 - Compound A6

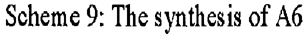

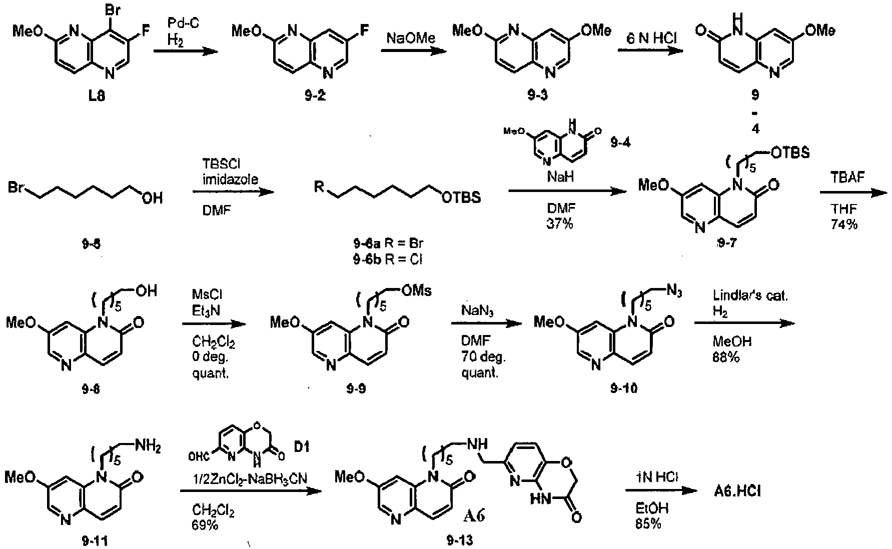

### 7-Fluoro-2-methoxy-1,5-naphthyridine (9-2).

A suspension of L8 (4.00 g, 15.6 mmol), sodium hydrogen carbonate (2.88 g) and 10% Pd-C catalyst (600 mg) in dichloromethane (31 mL) and ethanol (31 mL) was stirred at room temperature for 5 hours under 1.0 kg/cm² H₂ atmosphere. After reaction was complete insoluble materials were filtered off. The filtrate was diluted with water and extracted with dichloromethane. Organic extracts were washed with brine, dried over NaSO₄ and concentrated under reduced pressure to give 9-2 (2.54 g, 91%). ¹H NMR (400 MHz, CDCl₃) δ 4.08 (s, 3H), 7.09 (d, *J* = 9.2 Hz, 1H), 7.79 (dd, *J =* 9.5, 2.8 Hz, 1H), 8.19 (d, *J* = 9.2 Hz, 1H), 8.68 (d, *J* = 3.1 Hz, 1 H).

### 2,7-Dimethoxy-1,5-naphthyridine (9-3)

To a solution of 9-2 (1.00 g, 5.61 mmol) in methanol (28 mL) was added NaOMe (3.20 g) and methanol (15 mL), the mixture was stirred at 60 °C for 37 hours. The mixture was diluted with water and the resulting precipitates were collected by filtration to give 9-3 (829 mg, 78%). The filtrate was concentrated under reduced pressure to remove methanol. The resulting aqueous mixture was extracted with dichloromethane. Organic extracts were washed with brine, dried over NaSO₄ and concentrated under reduced pressure. Flash chromatography (hexane/EtOAc = 8:3) of the residue on silica gel gave the additional product of 9-3 (129.3 mg, total 958.3 mg, 90%). ¹H NMR (400 MHz, CDCl₃) δ 3.97 (s, 3H), 4.07 (s, 3H), 6.96 *(d, J =* 9.2 Hz, 1 H), 7.46 (d, *J=* 2.4 Hz, 1H), 8.14 *(d, J* = 9.2 Hz, 1H), 8.53 (d, *J=* 3.1 Hz, 1H).

### 7-Methoxy-1,5-naphthyridin-2(1H)-one (9-4)

A solution of 9-3 (940 mg, 4.94 mmol) in 6 N HCl (5 mL) was stirred at 110 °C for 2.5 hours. The mixture was adjusted to pH 3 by addition of aqueous NaOH while cooling in an ice bath. The resulting precipitates were collected by filtration to give 9-4 (842 mg, 97%) as a powder. ¹H NMR (400 MHz, DMSO-*d*₆) δ 3.87 (s, 3H), 6.52 (d, *J =* 9.2 Hz, 1H), 7.12 *(d, J =* 2.4 Hz, 1H), 7.85 (d, *J* = 9.8 Hz, 1H), 8.20 *(d, J* = 2.4 Hz, 1H).

### ((6-Bromohexyl)oxy)(tert-butyl)dimethylsilane (9-6a)

To a solution of 9-5 (2.00 g, 11.0 mmol) and imidazole (1.50 g, 22.1 mmol) in DMF (22.1 mL) was added TBSCl (2.00 g, 13.3 mmol) at 0 °C, and the mixture was stirred overnight at room temperature. The reaction mixture was diluted with EtOAc (150 mL) and washed with water and brine. The organic extracts were dried over magnesium sulfate, filtered, and then concentrated under reduced pressure. Flash chromatography (Hexane/EtOAc = 100:0 - 19:1) of the residue gave the mixture of 9-6a and 9-6b (2.49 g, 9-6a:9-6b = ratio 3:1). ¹H NMR (400 MHz, CDCl₃) δ 0.05 (s, 6H), 0.89 (s, 9H), 1.33-1.39 (m, 2H), 1.41-1.47 (m, 2H), 1.49-1.56 (m, 2H), 1.74-1.82 (m, 1.5H), 1.85-1.89 (m, 0.5H), 3.41 (t, *J =* 6.7 Hz, 0.5H), 3.54 (t, *J*= 6.7 Hz, 1.5H), 3.61 (t, *J* = 6.7 Hz, 2H).

### 1-(6-(tert-Butyldimethylsilyloxy)hexyl)-7-methoxy-1,5-naphthyridin-2(1H)-one (9-7)

To a solution of 9-4 (500 mg, 2.84 mmol) in DMF (15.8 mL) was added NaH (186 mg, 4.26 mmol) and 3:1 mixture of 9-6a and 9-6b (1.68 g). The mixture was stirred at room temperature for 1 hour followed by at 70 °C for 1 hour. The reaction mixture was quenched by addition of water (10 mL), extracted with EtOAc and washed with water and brine. The organic extracts were dried over magnesium sulfate, filtered, and then concentrated under reduced pressure. Flash chromatography (CH₂Cl₂/MeOH = 19:1) of the residue on silica gel gave compound 9-7 (408 mg, 37%). ¹H NMR (400 MHz, CDCl₃) δ 0.04 (s, 6H), 0.88 (s, 9H), 1.39-1.58 (m, 6H), 1.70-1.77 (m, 2H), 3.61 (t, *J* = 6.1 Hz, 2H), 3.98 (s, 3H), 4.20-4.24 (m, 2H), 6.75 (d, *J*= 9.8 Hz, 1H), 7.04 (d, *J =* 2.5 Hz, 1H), 7.84 (d, *J =* 9.8 Hz, 1H), 8.28 (d, *J* = 2.5 Hz, 1H).

### 1-(6-Hydroxyhexyl)-7-methoxy-1,5-naphthyridin-2(1H)-one (9-8)

A mixture of 9-7 (299 mg, 0.764 mmol) and TBAF (1.0 M THF solution, 917 microL, 0.917 mmol) in THF (7.64 mL) was stirred at room temperature for 2 hours and then concentrated under reduced pressure. Flash chromatography (EtOAc/MeOH = 19:1 - 9:1) of the residue on silica gel gave compound 9-8 (157 mg, 74%). ¹H NMR (400 MHz, CDCl₃) δ 1.46-1.62 (m, 6H), 1.72-1.80 (m, 2H), 3.63-3.67 (m, 2H), 3.99 (s, 3H), 4.22-4.26 (m, 2H), 6.76 (d, *J*= 9.8 Hz, 1H), 7.05 (d, *J* = 2.4 Hz, 1H), 7.84 *(d, J* = 9.8 Hz, 1H), 8.29 (d, *J* = 2.4 Hz, 1H).

### 6-(7-Methoxy-2-oxo-1,5-naphthyridin-1(2H)-yl)hexyl methanesulfonate (9-9)

To a solution of 9-8 (153 mg, 0.554 mmol) and Et₃N (154 microL, 1.11 mmol) in CH₂Cl₂ (5.5 mL) was added MsCl (55.7 microL, 0.72 mmol) under ice bath cooling. The mixture was stirred at the same temperature for 2 hours. After quenching the reaction by addition of water, the mixture was extracted with EtOAc and washed with water and brine. The organic extracts were dried over magnesium sulfate, filtered, and concentrated under reduced pressure to give crude 9-9 (212 mg). This material was used for the next step without further purification. ¹H NMR (400 MHz, CDCl₃) δ 1.49-1.53 (m, 4H), 1.72-1.82 (m, 4H), 3.01 (s, 3H), 3.99 (s, 3H), 4.22-4.25 (m, 4H), 6.75 (d, *J* = 9.2 Hz, 1H), 7.04 *(d, J =* 2.4 Hz, 1H), 7.85 (d, *J* = 9.8 Hz, 1H), 8.29 (d, *J* = 2.4 Hz, 1H).

### 1-(6-Azidohexyl)-7-methoxy-1,5-naphthyridin-2(1H)-one (9-10)

To a solution of the crude 9-9 (207 mg) in DMF (5.9 mL) was added NaN₃ (114 mg, 1.75 mmol) and the mixture was stirred at 70 °C for 2 hours. The reaction mixture was diluted with EtOAc (100 mL) and washed with water and brine. The organic extracts were dried over magnesium sulfate, filtered, and then concentrated under reduced pressure. Flash chromatography

(CH₂Cl₂/MeOH = 20:1) of the residue on silica gel gave compound 9-10 (177 mg, quant.). ¹H NMR (400 MHz, CDCl₃) δ 1.46-1.49 (m, 4H), 1.57-1.66 (m, 2H), 1.71-1.79 (m, 2H), 3.28 (t, *J* = 6.7 Hz, 2H), 3.99 (s, 3H), 4.21-4.25 (m, 2H), 6.75 *(d, J =* 9.8 Hz, 1H), 7.04 (d, *J* = 2.4 Hz, 1H), 7.84 (d, *J* = 9.8 Hz, 1H), 8.29 *(d, J =* 2.4 Hz, 1H).

### 1-(6-Aminohexyl)-7-methoxy-1,5-naphthyridin-2(1H)-one (9-11)

To a solution of 9-10 (172 mg, 0.572 mmol) in MeOH (5.7 mL) was added Lindlar catalyst (34.5 mg, 20 wt%) and the inhomogeneous mixture was stirred at room temperature for 2 hours under H₂ atmosphere (1.0 kg/cm²). The catalyst was filtered off and the filtrate was concentrated under reduced pressure. Flash chromatography on an amino silica column (CH₂Cl₂/MeOH = 19:1) of the residue gave compound 9-11 (139 mg, 88%). ¹H NMR (400 MHz, CDCl₃) δ 1.25-1.51 (m, 8H), 1.70-1.78 (m, 2H), 2.69 *(t, J=* 6.4 Hz, 2H), 3.98 (s, 3H), 4.20-4.24 (m, 2H), 6.75 (d, *J* = 9.8 Hz, 1H), 7.04 (d, *J* = 2.4 Hz, 1H), 7.84 (d, *J=* 9.2 Hz, 1H), 8.28 (d, *J* = 1.8 Hz, 1H).

### 6-(((6-(7-Methoxy-2-oxo-1,5-naphthyridin-1(2H)-yl)hexyl)amino)methyl)-2H-pyrido[3,2-b][1,4]oxazin-3(4H)-one (9-13)

To a solution of 9-11 (134 mg, 0.486 mmol) in CH₂Cl₂ (4.9 mL) was added D1 (86.6 mg, 0.486 mmol) and the mixture was stirred at room temperature for 45 minutes. Then 1/2ZnCl₂-NaBH₃CN (1.62 mL, 0.486 mmol, 0.3 M MeOH solution) was added and the reaction mixture was stirred at room temperature for 1 hour. Water (20 mL) was added to the mixture and extracted with CH₂Cl₂. The organic extracts were dried over magnesium sulfate, filtered, and then concentrated under reduced pressure. Flash chromatography on an amino silica column (CH₂Cl₂/MeOH = 40:1) of the residue gave 9-13 (A6) (138 mg, 65%). ¹H NMR (400 MHz, CDCl₃) δ 1.46-1.60 (m, 6H), 1.72-1.78 (m, 3H), 2.63 (t, *J=* 6.7 Hz, 1H), 3.76 (s, 2H), 3.98 (s, 3H), 4.22-4.26 (m, 2H), 4.64 (s, 2H), 6.82 (d, *J=* 9.8 Hz, 1H), 6.89 (d, *J* = 7.9 Hz, 1H), 7.04 (d, *J =* 2.4 Hz, 1 H), 7.19 *(d, J =* 7.9 Hz, 1 H), 7.85 (d, *J* = 9.8 Hz, 1H), 8.29 (d, *J* = 2.4 Hz, 1 H).

### Synthesis of A6

A mixture of 9-13 (133 mg, 0.304 mmol) and 1N HCl (304 mcroL, 0.304 mmol) in EtOH (3.0 mL) was stirred at room temperature for 10 min. After concentration, the residue was triturated with a mixture of EtOH and Et₂O (1:5) to give A6.HCl (123 mg, 85%). ¹H NMR (400 MHz, DMSO-*d*₆) δ 1.35-1.39 (m, 4H), 1.59-1.65 (m, 4H), 2.91-2.98 (m, 2H), 3.98 (s, 3H), 4.09-4.11 (m, 2H), 4.24 (t, *J*= 7.3 Hz, 2H), 4.68 (s, 2H), 6.66 (d, *J=* 9.8 Hz, 1H), 7.13-7.17 (m, 1H), 7.38 (d, *J* = 2.4 Hz, 1H), 7.42 (d, *J* = 8.6 Hz, 1H), 7.86 (d, *J* = 9.8 Hz, 1H), 8.29 (d, *J* = 2.4 Hz, 1H), 8.98 (br, 2H), 11.31 (s, 1H).

### Example 10 - biological testing

The antibacterial activity of the present compounds can be demonstrated by various assays known in the art, for example, by their minimum inhibitory concentration (MIC) against bacteria and minimum effective concentration (MEC). Compounds provided in the Examples were generally found to inhibit the growth of gram negative and gram positive bacteria causing various diseases in livestock and companion animals in the range of 0.03 µg/ml to 12.5 µg/mL.

The potency of antibacterial agents was measured using the Minimal Inhibitory Concentration (MIC) assay. The assay measures the ability of test agents to inhibit the growth of bacteria on agar-containing medium.

MIC values were determined using a modified agar dilution procedure described by the Clinical and Laboratory Standards Institute
1. Clinical and Laboratory Standards Institute (CLSI). *Performance standards for antimicrobial disk and dilution susceptibility tests for bacteria isolated from animals; approved standard* - *fourth edition.* CLSI document VET01-A4 (ISBN 1-56238-878-9). CLSI, Wayne, Pennsylvania 19087, USA, 2013.
2. Clinical and Laboratory Standards Institute (CLSI). *Performance standards for antimicrobial disk and dilution susceptibility tests for bacteria isolated from animals; second informational supplement.* CLSI document VET01-S2 (ISBN 1-56238-880-0). CLSI, Wayne, Pennsylvania 19087, USA, 2013.

*Mannheimia haemolytica* (one American Type Culture Collection (ATCC) 33396 serotype A2 strain from sheep and three strains from cattle) and *Pasteurella multocida* (one ATCC 43137 strain from pig and three strains from cattle) are gram negative bacteria causing bovine respiratory disease (BRD) in feedlot cattle and dairy cows. *Pasteurella multocida* is also implicated in atopic rhinitis in pigs and pyodermas in cats. *Escherichia coli* (ATCC 25922 serotype 06, biotype 1 strain and a bovine mastitis isolate) is implicated in a variety of diseases, e.g. enteritis, mastitis and metritis in bovines or urinary tract infections in dogs and cats. *Mycoplasma bovis* (eight strains from pneumonic cattle) is a further causative agent of pneumonia in cattle and is implicated in causing mastitis in dairy cows. *Staphylococcus aureus* (ATCC 29213 and one bovine mastitis isolate) and coagulase-negative Staphylococci (two bovine mastitis isolates) and *Streptococcus uberis* (two bovine mastitis isolates) and *Streptococcus dysgalactiae* (two bovine mastitis isolates) are gram positive mastitis-causing bacteria. *Staphylococcus pseudintermedius* (DSM 21284 cat isolate and a dog otitis isolate) is a gram positive bacterium and a major cause of pyodermas in dogs. *Pseudomonas aeruginosa* (ATCC 27853 and one otitis isolate from dogs) is a gram negative bacterium causing otitis, pyoderma, respiratory and urinary tract infections like cystitis in companion animals like dogs and cats.

Mueller Hinton Agar (MHA BBL; Becton Dickinson and Company, Sparks, MD) was used as the medium.

Broth-microdilution method according to CLSI document VET01-A4

### Preparation of the inoculum

A culture of the test isolate was plated on Mueller-Hinton agar with 5% sheep blood and incubated at about 35 °C with about 5% CO₂ for 18 h to 24 h. After checking for purity, sufficient well-isolated colonies from each subculture were inoculated into 4-mL to 5-mL aliquots of sterile cation-adjusted Mueller-Hinton broth (CAMHB). Broth cultures were incubated at about 35 °C for 2 h to 6 h until they achieved or exceeded the turbidity of 0.5 McFarland standards.

Incubated broth cultures were adjusted to the turbidity of 0.5 McFarland standards by adding sterile CAMHB. Turbidity adjusted suspension contained approximately 1 x 10⁸ colony-forming units (CFU)/mL.

The culture was further diluted 1:100 with sterile CAMHB to receive an inoculum with a final concentration of about 1 x 10⁶ CFU/mL.

### Inoculation of the microdilution trays

Microdilution trays containing a doubling dilution series of the compound were prepared before the study, stored below - 70 °C and were used within 6 months.

The microdilution trays were inoculated by adding 50 µL of the appropriate sterile broth and 50 µL of the inoculum into each well.

Incubation of the microdilution trays

The microdilution trays were incubated at about 35 °C for 18 h to 24 h.

### Reading and interpretation of the results

The MIC results were interpreted according to the CLSI documents VET01-A4. The MIC is the lowest concentration of antimicrobial agent that completely inhibits growth of the organisms. All wells containing test item were compared with the growth-control wells. The lowest concentration of compound at which no visible growth (i.e. no turbidity) detected by the unaided eye was recorded as the MIC. If a "trailing endpoint" occurred, the MIC was defined as the first concentration at which a markedly reduce (about 90%) of the inoculum was observed.

**Table 1 MIC values (in µM) of selected NTB Is against animal pathogens**

| Compo und ID | Mannhei mia haemolyt ica (n=4) | Pasteurel la multocid a (n=4) | Mycopla sma bovis (n=8) | Pseudom onas ieruginos a (n=2) | Staphylo coccus pseudinte rmedius (n= 2) | Staphylo coccus spp. (n=2) | Staphylo coccus aureus (n=2) | Strepto coccus dysgal actiae (n=2) | Strepto coccus uberis (n=2) | Escher ichia coli (n=2) | Actinoba cillus pleuropn eumoniae (n=2) | Bordetell a bronchise ptica (n=2) | Histophil us somni (n=2) | Rhodoco ccus equi (n=2) | Streptoco ccus canis (n= 2) |
|---|---|---|---|---|---|---|---|---|---|---|---|---|---|---|---|
| (-)-Al | 0.9 | ≤0,1 | 5.9 | 6.3 | 1.0 | 0.6 | 1.0 | 0.2 | 1.7 | 0.6 | 0.6 | 0.6 | ≤0.3 | 0.8 | 2.0 |
| (-)-A2 | 1.2 | ≤0,2 | n.d.* | 37.5 | 0.6 | 0.8 | 0.8 | 0.2 | 1.7 | 1.6 | n.d. | n.d. | n.d. | n.d. | n.d. |
| (+)-A3 | 1.0 | ≤0.1 | 2.7 | 12.5 | 0.6 | 0.6 | 1.0 | ≤0.1 | ≤0.9 | 0.8 | n.d. | n.d. | n.d. | n.d. | n.d. |
| A4 | 0.6 | ≤0.3 | n.d. | 4.7 | 1.2 | 1.2 | 0.5 | ≤0.2 | 0.9 | 0.4 | n.d. | n.d. | n.d. | n.d. | n.d |
| (-)-trans A5 | ≤0.1 | ≤0.1 | 0.4 | 2.4 | ≤0.1 | ≤0.3 | ≤0.1 | ≤0.1 | ≤0.3 | ≤0,1 | ≤0.3 | ≤0.2 | ≤0.2 | ≤0.2 | ≤0.5 |
| A6 | 1.8 | ≤0.1 | 3.5 | 25 | 0.6 | 0.4 | 0.3 | ≤0.2 | <1.6 | 3.1 | 1.6 | 2.4 | ≤0.2 | 0.6 | 1.8 |
| (-)-trans A7 | 0.5 | ≤0.1 | 0.4 | 18.8 | ≤0.2 | ≤0.2 | ≤0.2 | ≤0.1 | 0.9 | 0.8 | ≤0.3 | 0.8 | ≤0.2 | ≤0.2 | ≤0.5 |

| | | | | | | | | | | | | | | | |
|---|---|---|---|---|---|---|---|---|---|---|---|---|---|---|---|
| *n.d.: not determined | | | | | | | | | | | | | | | |

## Claims

1. The compound of Formula (I) 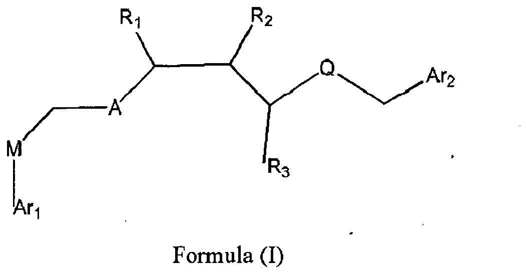 wherein
A is CH₂, O or Q;
M is (CH₂)ₙ;
n = 0-3
R₁ and R₃ are independently H, N(R₄R₅), or C₁-C₆ alkyl, wherein the C₁-C₆ alkyl is optionally substituted with one or more substituents selected from H, halogen, C₁-C₆ alkyl, N(R₄R₅) or
OR₅;
R₂ is H, halogen, OR₅, N(R₄R₅), or C₁-C₆ alkyl, wherein the C₁-C₆ alkyl is optionally substituted with one or more substituents selected from H, halogen, C₁-C₆ alkyl, N(R₄R₅)or OR₅;
or
R₁ and R₂ are taken together to form a 3-7 member ring which includes one or more C, O,or N(R₄)atom;
Q is N(R₄)
R₄ are each individually H or C₁-C₆ alkyl;
R₅ are each individually H or C₁-C₆ alkyl;
Ar₁ is selected from a group having one of the following structures 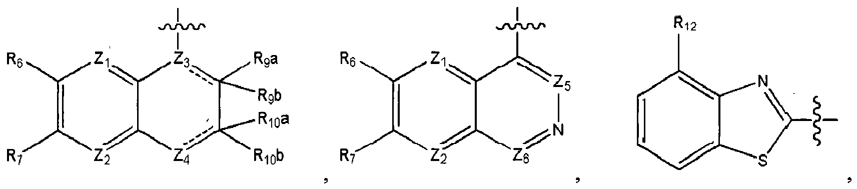 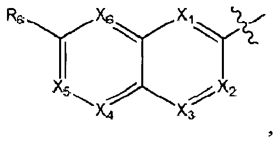 or 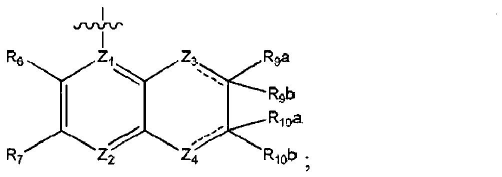 Z₁ is CR₁ₐ or N;
Z₂, Z₅ and Z₆ are independently selected from CR_{1b}, or N;
Z₃ is C or N if the ------ bond to which it is attached is a single bond; or Z₃ is C if the ------ bond to which it is attached is a double bond;
Z₄ is CR₁₁ₐR_{11b}, NR₄, or O if the ------ bond to which it is attached is a single bond;
or
Z₄ is CR₁₁ₐ or N if the ------ bond to which it is attached is a double bond; X₁, X₃, X₄ and X₆ are independently selected from N or CR₁ₐ;
wherein at least one of X₁, X₃, X₄ and X₆ is N;
X₂ is CH, C-(C₁₋₆)alkyl, C-(C₁₋₆)alkoxy, C-halo, or C-COOH;
X₅ is CH, C-(C₁₋₆)alkyl or C-halo;
R₆ is H; OH; NR₁₃R₁₄; (C₁₋₆)alkyl; C(O)OR₁₃; halo; CF₃; cyano; allyloxy;
-R₁₅COOR₁₄; -OR₁₅COOR₁₄; (C₁₋₆)alkoxy, (C₃₋₆)cycloalkoxy, (C₃₋₆)heterocycleoxy, (C₃₋₆)cycloalkylalkoxy, or (C₃₋₆)heterocycloalkoxy which are optionally substituted with NR₁₃R₁₄, OH, CF₃, COOR₁₄, cyano, oxo, (C₁₋₆)alkyl or (C₁₋₆)alkoxy; S(O)₂R₁₃ optionally substituted with a (C₁₋₆)alkyl; or 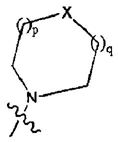
wherein X is CR_{1c}, O or S;
each p and q is 0, 1, or 2, with the proviso that if X is O or S, both p and q cannot be 0;
each R₇ is independently H, halo, OH, (C₁₋₆)alkoxy, NR₁₃R₁₄, CF₃, or cyano;
R₉ₐ is H, halo, OH, (C₁₋₆)alkoxy, NH₂, or cyano; R_{9b} is absent; and the ------ bond attached to Z₃ is a double bond; or
R₉ₐ and R_{9b} together form oxo; and the ------ bond attached to Z₃ is a single bond; R₁₀ₐ is H or (C₁₋₆)alkyl; R_{10b} is absent; and the ------ bond attached to Z₄ is a double bond; or
R₁₀ₐ and R_{10b} together form oxo; and the ------ bond attached to Z₄ is a single bond; R₁₁ₐ is H or (C₁₋₆)alkyl; and R_{11b} is absent; and the ------ bond attached to Z₄ is a double bond; or
R₁₁ₐ and R_{11b} together form oxo; and the ------ bond attached to Z₄ is a single bond; or R₁₀ₐ and R₁₁ₐ together with the atoms to which they are attached form a 5-membered saturated, unsaturated or aromatic ring having 0 to 3 N and optionally substituted with a (C₁₋₆)alkyl, wherein R_{10b} and R_{11b} are H when the ------ bond attached to Z₄ is a single bond and absent when the ------ bond attached to Z₄ is a double bond;
each R₁₂, R₁₃ and R₁₄ is independently H or (C₁₋₆)alkyl;
each R₁₅ is independently (C₁-C₆)alkyl or (C₂-C₆)alkenyl with the proviso that when R₆ is -OR₁₅COOR₁₄, R₁₅ is not C₂alkenyl;
R₁ₐ is H, OH, (C₁₋₆)alkoxy, cyano, or halo;
R_{1b} is H, (C₁₋₆)alkyl, (C₁₋₆)alkoxy, halo, cyano, or C(O)OR₁₃;
R_{1c} is H, halo or (C₁₋₆)alkyl;
and
Ar₂ is selected from
(i) C₃-C₆-cycloalkyl, optionally substituted with -OH, halo, cyano, NR₁₃R₁₄ or (C₁₋₆)alkyl;
(ii) aryl, wherein aryl is phenyl or naphthyl optionally substituted with 1 to 3 substituents selected from OH, halo, (C₁₋₆)alkoxy and (C₁₋₆)alkyl;
(iii) a heterocyclyl, wherein the heterocyclyl is a 5- to 6-membered non-aromatic or aromatic ring having 1 or 2 heteroatoms selected from N, O or S optionally substituted with 1 to 3 substituents selected from OH, halo, cyano, (C₁₋₆)alkoxy, (C₁₋₆)alkyl, NR₁₃R₁₄ and a 5- to 6-membered aromatic or non-aromatic ring having 1 or 2 heteroatoms selected from N, O or S; wherein (C₁₋₆)alkoxy or (C₁₋₆)alkyl optionally substituted with 1 or 2 halo; or
(iv) a group having one of the following structures: 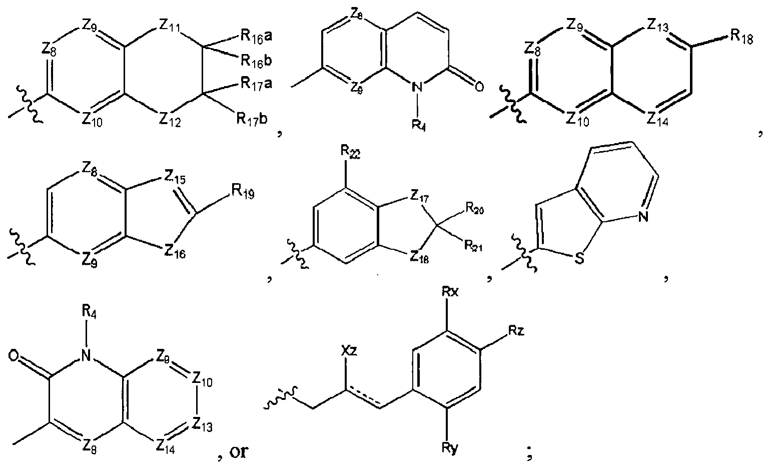 each Z₈, Z₉ and Z₁₀ is independently CR₁ₐ or N;
Z₁₁ and Z₁₂ are each independently selected from CR₁ₐR_{1b}, NR₄, O, and S; Z₁₃ and Z₁₄ are each independently selected from CR₁ₐ and N;
Z₁₅ is CR₁ₐ or N;
Z₁₆ is CR₁ₐR_{1b} or NH;
each Z₁₇ and Z₁₈ is independently NR₄ or O;
each R₁₆ₐ and R_{16b} is independently H or CH₃;
or R₁₆ₐ and R_{16b} together form oxo;
R₁₇ₐ and R_{17b} are H;
or R₁₇ₐ and R_{17b} together form oxo or =NOR₃;
R₁₈ is H or (C₁₋₆)alkoxy;
R₁₉ is H or halo;
each R₂₀, R₂₁ and R₂₂ is independently H or halo each Rₓ, Ry and R_{z} is independently H, halo or (C1-6)alkyl X_{z} is H, halo or (C1-6)alkyl
or a pharmaceutically acceptable salt thereof.

2. The compound of Formula (II) 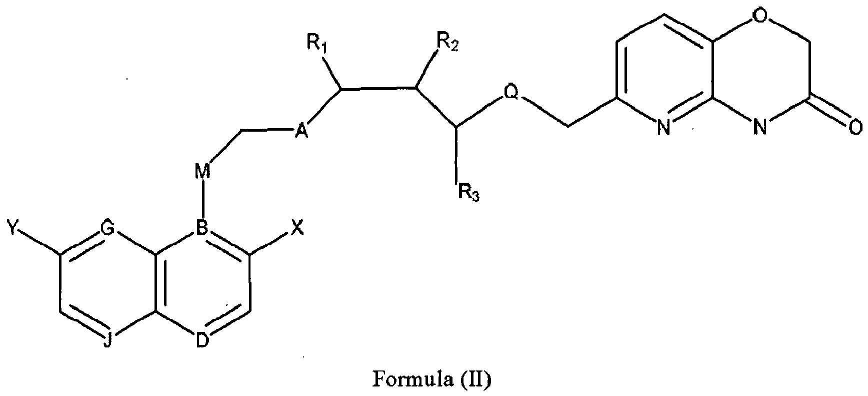 wherein,
A is CH₂, NH, or O;
B, D, G, J are each independently either C or N;
M is (CH₂)ₙ;
(n = 0-3)
X is H, F, Cl, CN, CO₂R₅ or O;
Y is OR₅, CO₂R₅ or CN;
R¹ and R₃ are independently H, N(R₄R₅), or C₁-C₆ alkyl, wherein the C₁-C₆ alkyl is optionally substituted with one or more substituents selected from H, halogen, C₁-C₆ alkyl, N(R₄R₅)or OR⁵;
R₂ is H, halogen, OR₅, N(R₄R₅), or C₁-C₆ alkyl, wherein the C₁-C₆ alkyl is optionally substituted with one or more substituents selected from H, halogen, C₁-C₆ alkyl, N(R₄R₅)or OR⁵; or
R₁ and R₂ are taken together to form a 3-7 member ring which includes one or more C, O or N atoms;
Q is NR₄;
R₄ are each individually H or C₁-C₆ alkyl; and R₅ are each individually H or C₁-C₆ alkyl or a pharmaceutically acceptable salt thereof.

3. The compound of claim 2 wherein
A is NH;
B and J are C;
D and G are N;
M is CH₂;X is H, F,Cl, CN, OR₅, or CO₂R₅; and
Y is H, Cl, F, CN, OR₅, or CO₂R₅
wherein R₅ is defined as in claim 2
or a pharmaceutically acceptable salt thereof.

4. The compound of Formula (III) 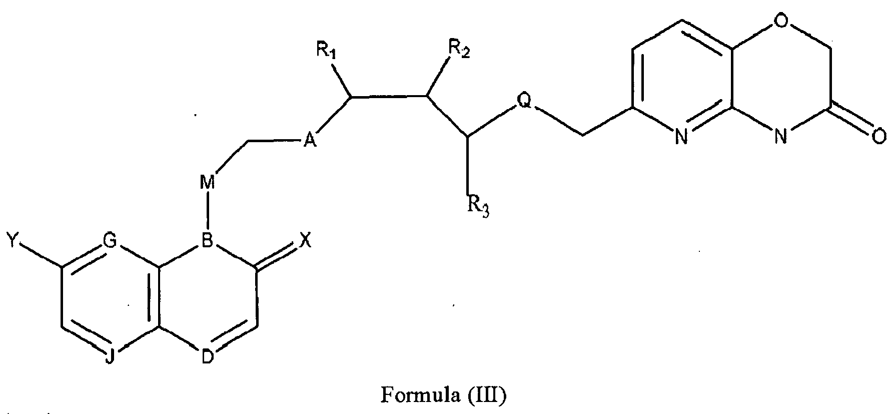 wherein,
A is CH₂, NH, or O;
BisN;
D, G, J are each independently either C or N;
M is (CH₂)ₙ; (n = 0-3)
X is O or S;
Y is H, F, Cl, OR₅, CN, or CO₂R₅
R₁ and R₃ are H,, N(R₄R₅), or C₁-C₆ alkyl, wherein the C₁-C₆ alkyl is optionally substituted with one or more substituents selected from H, halogen, C₁-C₆ alkyl, N(R₄R₅) or OR₅;
R₂ is H, halogen, OR₅, N(R₄R₅), or C₁-C₆ alkyl, wherein the C₁-C₆ alkyl is optionally substituted with one or more substituents selected from H, halogen, C₁-C₆ alkyl, N(R₄R₅)or OR₅; or
R₁ and R₂ are taken together to form a 3-7 member ring which includes one or more C, O or N atoms;
Q is NR₄;
R₄ are each individually H or C₁-C₆ alkyl; and
R₅ are each individually H or C₁-C₆ alkyl,
or a pharmaceutically acceptable salt thereof.

5. The pharmaceutically acceptable salt of the compound of any one of claims 1-4.

6. A compound selected from the following group: 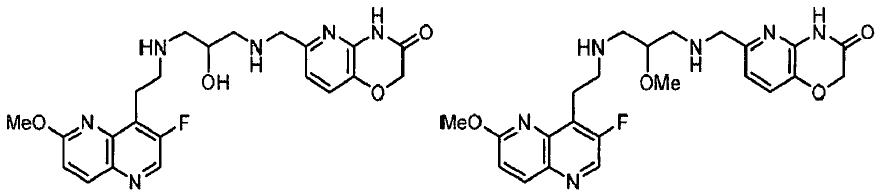 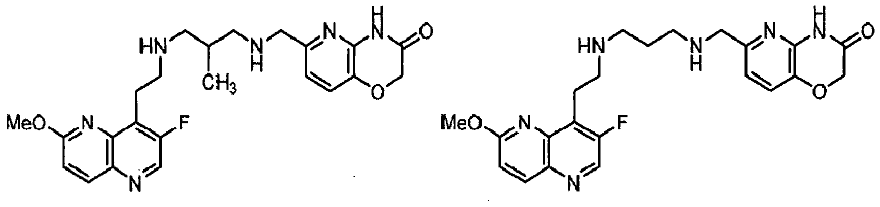 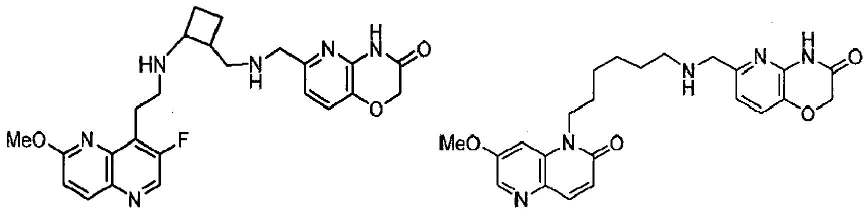 and 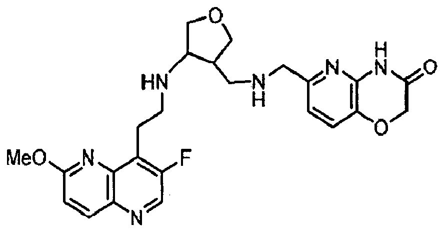 or a pharmaceutically acceptable salt thereof.

7. A compound selected from the following group:
6-[[[3-[2-(3-fluoro-6-methoxy-1,5-naphthyridin-4-yl)ethylamino]-2-hydroxy-propyl]amino]methyl]-4H-pyrido[3,2-b][1,4]oxazin-3-one;
6-[[[3-[2-(3-fluoro-6-methoxy-1,5-naphthyridin-4-yl)ethylamino]-2-methoxy-propyl]amino]methyl]-4H-pyrido[3,2-b][1,4]oxazin-3-one;
6-[[[3-[2-(3-fluoro-6-methoxy-1,5-naphthyridin-4-yl)ethylamino]-2-methyl-propyl]amino]methyl]-4H-pyrido[3,2-b][1,4]oxazin-3-one;
6-[[3-[2-(3-fluoro-6-methoxy-1,5-naphthyridin-4-yl)ethylamino]propylamino]methyl]-4H-pyrido[3,2-b][1,4]oxazin-3-one;
6-[[[2-[2-(3-fluoro-6-methoxy-1,5-naphthyridin-4-yl)ethylamino]cyclobutyl]methylamino]methyl]-4H-pyrido [3,2-b][1,4]oxazin-3-one;
6-[[6-(7-methoxy-2-oxo-1,5-naphthyridin-1-yl)hexylamino]methyl]-4H-pyrido[3,2-b][1,4]oxazin-3-one; and
6-[[[4-[2-(3-fluoro-6-methoxy-1,5-naphthyridin-4-yl)ethylamino]tetrahydrofuran-3-yl]methylamino]methyl]-4H-pyrido[3,2-b][1,4]oxazin-3-one;
6-[[[3-[2-(3-fluoro-6-methoxy-1,5-naphthyridin-4-yl)ethylamino]-2-hydroxy-propyl]amino]methyl]-4H-pyrido[3,2-b][1,4]oxazin-3-one;
6-[[[3-[2-(3-fluoro-6-methoxy-1,5-naphthyridin-4-yl)ethylamino]-2-methoxy-propyl]amino]methyl]-4H-pyrido[3,2-b][1,4]oxazin-3-one;
or a pharmaceutically acceptable salt thereof.

8. A pharmaceutical composition comprising the compound of any one of claims 1-7 and a pharmaceutical carrier.

9. The composition of claim 8, further comprising a second therapeutic agent selected from the group consisting of antibiotic agents, anti-inflammatory agents, anti-parasitics and antifungal agents.

10. The composition of claim 9, wherein the second therapeutic agent is one or more of an amphenicol, a â-lactam, a tetracycline, an aminoglycoside, a quinolone, a mutilin, a macrolide, an azole, a non-steroidal anti-inflammatory drug (NSAID), a glucocorticosteroid or their pharmaceutically acceptable salts.

11. A method of treating an infectious disease in an animal comprising administration to the animal of an effective amount of the pharmaceutical composition of claim 8 to the animal.

12. The method of claim 11, wherein the infectious disease is selected from a respiratory tract disease, a mastitis disease, a skin or soft tissue disease, a urinary tract disease or a disease caused by opportunistic pathogens.

13. The method of claim 11, wherein the animal is a bovine, a swine, a member of the poultry family, an ovine, a fish, a horse, a dog or a cat.

14. The method of claim 11, wherein the infection is a *Mannheimia haemolytica, Pasteurella multocida, Histophilus somni, Mycoplasma bovis, Pseudomonas aeruginosa, Staphylococcus pseudintermedius, Staphylococcus aureus, Staphylococcus spp., Streptococcus dysgalactiae, Streptococcus uberis, Streptococcus canis, Escherichia coli, Actinobacillus pleuropneumoniae, Bordetella bronchiseptica or Rhodococcus equi* infection.

15. Use of an effective amount of the compound of any one of claims 1-7 for the preparation of a medicament for treating a bacterial infection.
